(19) Europäisches Patentamt
European Patent Office
Office européen des brevets

(11) Veröffentlichungsnummer : **0 483 055 A1**

# (12) EUROPÄISCHE PATENTANMELDUNG

(21) Anmeldenummer : **91810757.4**

(22) Anmeldetag : **26.09.91**

(51) Int. Cl.[5] : **C07D 401/06,** C07D 417/06, A01N 51/00, // (C07D401/06, 251:00, 213:00), (C07D417/06, 277:00, 251:00)

(30) Priorität : **05.10.90 CH 3219/90**
**04.06.91 CH 1648/91**

(43) Veröffentlichungstag der Anmeldung :
**29.04.92 Patentblatt 92/18**

(84) Benannte Vertragsstaaten :
**BE CH DE DK ES FR GB GR IT LI LU NL**

(71) Anmelder : **CIBA-GEIGY AG**
**Klybeckstrasse 141**
**CH-4002 Basel (CH)**

(72) Erfinder : **Maienfisch, Peter, Dr.**
**Aegertenstrasse 21**
**CH-4118 Rodersdorf (CH)**
Erfinder : **Kristiansen, Odd, Dr.**
**Delligrabenstrasse 7**
**CH-4313 Möhlin (CH)**
Erfinder : **Gsell, Laurenz, Dr.**
**Maiengasse 56**
**CH-4056 Basel (CH)**

(54) **Triazacyclohexanderivate.**

(57) Neue Triazacyclohexanderivate der Formel I

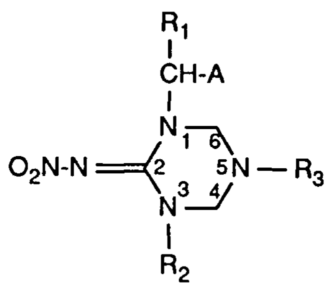

(I),

worin

$R_1$ Wasserstoff oder $C_1$-$C_4$-Alkyl,

$R_2$ Wasserstoff, $C_1$-$C_6$-Alkyl, $C_3$-$C_6$-Cycloalkyl oder einen Rest $-CH_2B$ ;

$R_3$ Wasserstoff, $C_1$-$C_{10}$-Alkyl, $C_3$-$C_6$-Cycloalkyl, mit 1-12 Resten aus der Gruppe Halogen, Hydroxy, $C_1$-$C_4$-Alkoxy, $C_1$-$C_4$-Haloalkoxy mit 1 bis 9 Halogenatomen, Di-($C_1$-$C_4$-Alkyl)-amino und $C_1$-$C_5$-Alkoxycarbonyl substituiertes $C_1$-$C_{10}$-Alkyl, mit 1 bis 4 $C_1$-$C_4$-Alkylresten oder Halogenatomen substituiertes $C_3$-$C_6$-Cycloalkyl, $C_2$-$C_8$-Alkenyl oder -Alkinyl, mit 1-6 Halogenatomen substituiertes $C_2$-$C_8$-Alkenyl oder -Alkinyl, Phenyl, Benzyl, oder mit 1-3 Ringsubstituenten aus der Gruppe Halogen, $C_1$-$C_4$-Alkyl, $C_1$-$C_4$-Haloalkyl mit 1 bis 9 Halogenatomen, $C_1$-$C_4$-Alkoxy, $C_1$-$C_4$-Haloalkoxy mit 1 bis 9 Halogenatomen, $C_1$-$C_4$-Alkylthio, Nitro und Cyano substituiertes Phenyl oder Benzyl ;

A einen unsubstituierten oder ein- bis vierfach substituierten aromatischen oder nichtaromatischen, monocyclischen oder bicyclischen heterocyclischen Rest, der ein bis zwei Substituenten aus der Gruppe $C_1$-$C_3$-Halogenalkyl mit 1 bis 7 Halogenatomen, Cyclopropyl, Halogencyclopropyl mit 1 bis 3 Halogenatomen, $C_2$-$C_3$-Alkenyl, $C_2$-$C_3$-Alkinyl, $C_2$-$C_3$-Halogenalkenyl und $C_2$-$C_3$-Halogenalkinyl mit 1 bis 4 Halogenatomen, $C_1$-$C_3$-Halogenalkoxy mit 1 bis 7 Halogenatomen, $C_1$-$C_3$-Alkylthio, $C_1$-$C_3$-Halogenalkylthio mit 1 bis 7 Halogenatomen, Allyloxy, Propargyloxy, Allylthio, Propargylthio, Halogenallyloxy, Halogenallylthio, Cyan und Nitro und ein bis vier Substituenten aus der Gruppe $C_1$-$C_3$-Alkyl, $C_1$-$C_3$-Alkoxy und Halogen aufweisen kann ; und

B Phenyl, Cyanophenyl, Nitrophenyl, Halogenphenyl mit 1 bis 3 Halogenatomen, durch $C_1$-$C_3$-Alkyl, $C_1$-$C_3$-Halogenalkyl mit 1 bis 7 Halogenatomen, $C_1$-$C_3$-Alkoxy oder $C_1$-$C_3$-Halogenalkoxy mit 1 bis 7 Halogenatomen substituiertes Phenyl, 3-Pyridyl, 5-Thiazolyl, durch ein bis zwei Substituenten

EP 0 483 055 A1

aus der Gruppe $C_1$-$C_3$-Alkyl, $C_1$-$C_3$-Halogenalkyl mit 1 bis 7 Halogenatomen, Cyclopropyl, Halogencyclopropyl, $C_2$-$C_3$-Alkenyl, $C_2$-$C_3$-Alkinyl, $C_1$-$C_3$-Alkoxy, $C_2$-$C_3$-Halogenalkenyl und $C_2$-$C_3$-Halogenalkinyl mit 1 bis 4 Halogenatomen, $C_1$-$C_3$-Halogenalkoxy mit 1 bis 7 Halogenatomen, $C_1$-$C_3$-Alkylthio, $C_1$-$C_3$-Halogenalkylthio mit 1 bis 7 Halogenatomen, Allyloxy, Propargyloxy, Allylthio, Propargylthio, Halogenallyloxy, Halogenallylthio, Halogen, Cyan und Nitro substituiertes 5-Thiazolyl ; oder durch ein bis zwei Reste aus der Gruppe $C_1$-$C_3$-Halogenalkyl mit 1 bis 7 Halogenatomen, Cyclopropyl, Halogencyclopropyl, $C_2$-$C_3$-Alkenyl, $C_2$-$C_3$-Alkinyl, $C_2$-$C_3$-Halogenalkenyl und $C_2$-$C_3$-Halogenalkinyl mit 1 bis 4 Halogenatomen, $C_1$-$C_3$-Halogenalkoxy mit 1 bis 7 Halogenatomen, $C_1$-$C_3$-Alkylthio, $C_1$-$C_3$-Halogenalkylthio mit 1 bis 7 Halogenatomen, Allyloxy, Propargyloxy, Allylthio, Propargylthio, Halogenallyloxy, Halogenallylthio, Cyan und Nitro oder durch ein bis vier Reste aus der Gruppe $C_1$-$C_3$-Alkyl, $C_1$-$C_3$-Alkoxy und Halogen substituiertes 3-Pyridyl ; bedeuten, sowie deren Salze mit anorganischen Säuren besitzen wertvolle pestizide Eigenschaften. Diese Verbindungen enthaltenden Mittel, Verfahren zu ihrer Herstellung sowie ihre Verwendung als Pestizide, insbesondere als Insektizide und Akarizide in der Landwirtschaft, werden beschrieben.

Die vorliegende Erfindung betrifft neue substituierte 2-Nitroimino-1,3,5-triazacyclohexanderivate, Verfahren zu ihrer Herstellung, Schädlingsbekämpfungsmittel, welche diese Verbindungen enthalten, sowie ihre Verwendung bei der Kontrolle von Schädlingen.

Die erfindungsgemässen Triazacyclohexanderivate entsprechen der Formel I

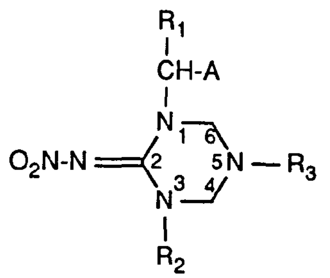

(I),

worin

$R_1$ Wasserstoff oder $C_1$-$C_4$-Alkyl;

$R_2$ Wasserstoff, $C_1$-$C_6$-Alkyl, $C_3$-$C_6$-Cycloalkyl oder einen Rest -$CH_2B$;

$R_3$ Wasserstoff, $C_1$-$C_{10}$-Alkyl, $C_3$-$C_6$-Cycloalkyl, mit 1-12 Resten aus der Gruppe Halogen, Hydroxy, $C_1$-$C_4$-Alkoxy, $C_1$-$C_4$-Haloalkoxy mit 1 bis 9 Halogenatomen, Di-($C_1$-$C_4$-Alkyl)-amino und $C_1$-$C_5$-Alkoxycarbonyl substituiertes $C_1$-$C_{10}$-Alkyl, mit 1 bis 4 $C_1$-$C_4$-Alkylresten oder Halogenatomen substituiertes $C_3$-$C_6$-Cycloalkyl, $C_2$-$C_8$-Alkenyl oder -Alkinyl, mit 1-6 Halogenatomen substituiertes $C_2$-$C_8$-Alkenyl oder -Alkinyl, Phenyl, Benzyl, oder mit 1-3 Ringsubstituenten aus der Gruppe Halogen, $C_1$-$C_4$-Alkyl, $C_1$-$C_4$-Haloalkyl mit 1 bis 9 Halogenatomen, $C_1$-$C_4$-Alkoxy, $C_1$-$C_4$-Haloalkoxy mit 1 bis 9 Halogenatomen, $C_1$-$C_4$-Alkylthio, Nitro und Cyano substituiertes Phenyl oder Benzyl;

A einen unsubstituierten oder ein- bis vierfach substituierten aromatischen oder nichtaromatischen, monocyclischen oder bicyclischen heterocyclischen Rest, der ein bis zwei Substituenten aus der Gruppe $C_1$-$C_3$-Halogenalkyl mit 1 bis 7 Halogenatomen, Cyclopropyl, Halogencyclopropyl mit 1 bis 3 Halogenatomen, $C_2$-$C_3$-Alkenyl, $C_2$-$C_3$-Alkinyl, $C_2$-$C_3$-Halogenalkenyl und $C_2$-$C_3$-Halogenalkinyl mit 1 bis 4 Halogenatomen, $C_1$-$C_3$-Halogenalkoxy mit 1 bis 7 Halogenatomen, $C_1$-$C_3$-Alkylthio, $C_1$-$C_3$-Halogenalkylthio mit 1 bis 7 Halogenatomen, Allyloxy, Propargyloxy, Allylthio, Propargylthio, Halogenallyloxy, Halogenallylthio, Cyan und Nitro und ein bis vier Substituenten aus der Gruppe $C_1$-$C_3$-Alkyl, $C_1$-$C_3$-Alkoxy und Halogen aufweisen kann; und

B Phenyl, Cyanophenyl, Nitrophenyl, Halogenphenyl mit 1 bis 3 Halogenatomen, durch $C_1$-$C_3$-Alkyl, $C_1$-$C_3$-Halogenalkyl mit 1 bis 7 Halogenatomen, $C_1$-$C_3$-Alkoxy oder $C_1$-$C_3$-Halogenalkoxy mit 1 bis 7 Halogenatomen substituiertes Phenyl, 3-Pyridyl, 5-Thiazolyl, durch ein bis zwei Substituenten aus der Gruppe $C_1$-$C_3$-Alkyl, $C_1$-$C_3$-Halogenalkyl mit 1 bis 7 Halogenatomen, Cyclopropyl, Halogencyclopropyl, $C_2$-$C_3$-Alkenyl, $C_2$-$C_3$-Alkinyl, $C_1$-$C_3$-Alkoxy, $C_2$-$C_3$-Halogenalkenyl und $C_2$-$C_3$-Halogenalkinyl mit 1 bis 4 Halogenatomen, $C_1$-$C_3$-Halogenalkoxy mit 1 bis 7 Halogenatomen, $C_1$-$C_3$-Alkylthio, $C_1$-$C_3$-Halogenalkylthio mit 1 bis 7 Halogenatomen, Allyloxy, Propargyloxy, Allylthio, Propargylthio, Halogenallyloxy, Halogenallylthio, Halogen, Cyan und Nitro substituiertes 5-Thiazolyl; oder durch ein bis zwei Reste aus der Gruppe $C_1$-$C_3$-Halogenalkyl mit 1 bis 7 Halogenatomen, Cyclopropyl, Halogencyclopropyl, $C_2$-$C_3$-Alkenyl, $C_2$-$C_3$-Alkinyl, $C_2$-$C_3$-Halogenalkenyl und $C_2$-$C_3$-Halogenalkinyl mit 1 bis 4 Halogenatomen, $C_1$-$C_3$-Halogenalkoxy mit 1 bis 7 Halogenatomen, $C_1$-$C_3$-Alkylthio, $C_1$-$C_3$-Halogenalkylthio mit 1 bis 7 Halogenatomen, Allyloxy, Propargyloxy, Allylthio, Propargylthio, Halogenallyloxy, Halogenallylthio, Cyan und Nitro oder durch ein bis vier Reste aus der Gruppe $C_1$-$C_3$-Alkyl, $C_1$-$C_3$-Alkoxy und Halogen substituiertes 3-Pyridyl; bedeuten, sowie deren Salze mit anorganischen Säuren.

Die Verbindungen der erfindungsgemässen Formel I schliessen auch die Salze mit agrochemisch verträglichen anorganischen Säuren ein. Beispiele solcher Säuren sind Salzsäure, Bromwasserstoffsäure, Schwefelsäure, Phosphorsäure und Salpetersäure sowie Säuren mit gleichem Zentralatom und höherer oder niedrigerer Oxidationsstufen wie Perchlorsäure, salpetrige Säure oder phosphorige Säure.

Die Verbindungen der Formel I können in tautomeren Formen Ia oder Ib auftreten, wenn der Rest $R_2$ für Wasserstoff steht:

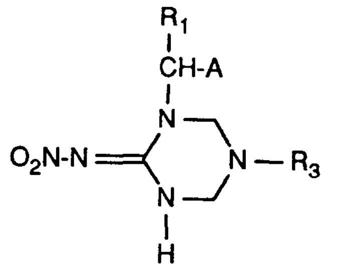

(Ia)

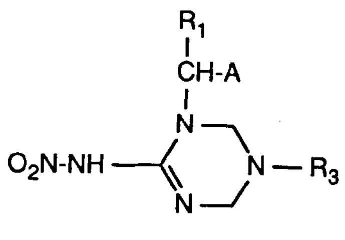

(Ib)

Die Verbindungen der Formel I können auch als Doppelbindundungsisomeren bezüglich N=C(2) auftreten.

Die erfindungsgemässe Formel I ist demnach so zu verstehen, dass auch die Formeln Ia und Ib und die Doppelbindungsisomeren in der Schreibweise der Formel I eingeschlossen sind.

In der Definition der erfindungsgemässen Formel I sollen die einzelnen generischen Begriffe wie folgt verstanden werden:

Bei den als Substituenten in Betracht kommenden Halogenatomen handelt es sich sowohl um Fluor und Chlor als auch um Brom und Jod, wobei Fluor, Chlor und Brom bevorzugt sind. Halogen ist dabei als selbständiger Substituent oder als Teil eines Substituenten zu verstehen wie im Halogenalkyl, Halogenalkylthio, Halogenalkoxy, Halogencycloalkyl, Halogenalkenyl, Halogenalkinyl, Halogenallyloxy oder Halogenallylthio. Die als Substituenten in Betracht kommenden Alkyl-, Alkylthio-, Alkenyl-, Alkinyl- und Alkoxyreste können geradkettig oder verzweigt sein. Als Beispiele solcher Alkyle seien Methyl, Aethyl, Propyl, Isopropyl, Butyl, i-Butyl, sek.-Butyl oder tert.-Butyl genannt. Als geeignete Alkoxyreste seien unter anderem genannt: Methoxy, Aethoxy, Propoxy, Isopropoxy oder Butoxy und ihre Isomeren. Alkylthio steht beispielsweise für Methylthio, Aethylthio, Isopropylthio, Propylthio oder die isomeren Butylthio. Sind die als Substituenten in Betracht kommenden Alkyl-, Alkoxy-, Alkenyl-, Alkinyl- oder Cycloalkylgruppen durch Halogen substituiert, so können sie nur teilweise oder auch perhalogeniert sein. Dabei gelten für Halogen, Alkyl und Alkoxy die oben gegebenen Definitionen. Beispiele der Alkylelemente dieser Gruppen sind das ein- bis dreifach durch Fluor, Chlor und/oder Brom substituierte Methyl wie beispielsweise $CHF_2$ oder $CF_3$; das ein- bis fünffach durch Fluor, Chlor und/oder Brom substituierte Aethyl wie zum Beispiel $CH_2CF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ oder $CClFCHClF$; das ein- bis siebenfach durch Fluor, Chlor und/oder Brom substituierte Propyl oder Isopropyl wie beispielsweise $CH_2CHBrCH_2Br$, $CF_2CHFCF_3$, $CH_2CF_2CF_3$ oder $CH(CF_3)_2$; das ein- bis neunfach durch Fluor, Chlor und/oder Brom substituierte Butyl oder eines seiner Isomeren wie zum Beispiel $CF(CF_3)CHFCF_3$ oder $CH_2(CF_2)_2CF_3$; 2-Chlorcyclopropyl oder 2,2-Difluorcyclopropyl; 2,2-Difluorvinyl, 2,2-Dichlorvinyl, 2-Chloralkyl, 2,3-Dichlorvinyl oder 2,3-Dibromvinyl.

Sind die definierten Alkyl-, Alkoxy- oder Cycloalkylgruppen durch andere Substituenten substituiert, so können sie ein- oder mehrfach durch den gleichen oder verschiedene der aufgezählten Substituenten substituiert sein. Vorzugsweise sind in den substituierten Gruppen ein oder zwei weitere Substituenten vorhanden. Bei den als Substituenten in Betracht kommenden Cycloalkylresten handelt es sich beispielsweise um Cyclopropyl, Cyclobutyl, Cyclopentyl oder Cyclohexyl. Alkenyl- und Alkinylgruppen enthalten eine ungesättigte Kohlenstoff-Kohlenstoff-Bindung. Typische Vertreter sind Allyl, Methallyl oder Propargyl, aber auch Vinyl und Äthinyl. Die Doppel- oder Dreifachbindungen in Allyloxy, Propargyloxy, Allylthio oder Propargylthio sind von der Verknüpfungsstelle zum Heteroatom (O oder S) vorzugsweise durch ein gesättigtes Kohlenstoffatom getrennt.

Unter den vorstehend definierten Verbindungen der Formel I, sind diejenigen hervorzuheben, worin der Rest

$R_3$ $C_5$-$C_{10}$-Alkyl, $C_3$-$C_6$-Cycloalkyl, mit 1-12 Resten aus der Gruppe Halogen, Hydroxy, $C_1$-$C_4$-Alkoxy, $C_1$-$C_4$-Haloalkoxy mit 1 bis 9 Halogenatomen, Di-($C_1$-$C_4$-Alkyl)-amino und $C_1$-$C_5$-Alkoxycarbonyl substituiertes $C_1$-$C_{10}$-Alkyl, mit 1-4 $C_1$-$C_4$-Alkyl-resten oder Halogenatomen substituiertes $C_3$-$C_6$-Cycloalkyl, $C_2$-$C_8$-Alkenyl oder -Alkinyl, mit 1-6 Halogenatomen substituiertes $C_2$-$C_8$-Alkenyl oder -Alkinyl, Phenyl, Benzyl, oder mit 1-3 Ringsubstituenten aus der Gruppe Halogen, $C_1$-$C_4$-Alkyl, $C_1$-$C_4$-Haloalkyl mit 1 bis 9 Halogenatomen, $C_1$-$C_4$-Alkoxy,

$C_1$-$C_4$-Haloalkoxy mit 1 bis 9 Halogenatomen, $C_1$-$C_4$-Alkylthio, Nitro und Cyano substituiertes Phenyl oder Benzyl bedeutet und $R_1$, $R_2$ und A die vorstehend angegebenen Bedeutungen haben.

Erfindungsgemäss von besonderer Bedeutung sind weiterhin solche Verbindungen der Formel I, worin der heterocyclische Rest A ungesättigt ist, über ein Kohlenstoffatom an den Rest des Moleküls der Verbindung der Formel I gebunden ist und mindestens ein Stickstoffatom enthält; solche, worin der heterocyclische Rest A ungesättigt ist, über ein Kohlenstoffatom an den Rest des Moleküls der Verbindung der Formel I gebunden ist und ein bis drei Heteroatome aus der Reihe Sauerstoff, Schwefel und Stickstoffatom enthält, wobei jeweils höchstens ein Sauerstoff- oder Schwefelatom enthalten ist; und solche, worin der heterocyclische Rest A ein bis drei Heteroatome aus der Reihe Sauerstoff, Schwefel und Stickstoff enthält, wovon stets ein Heteroatom Stickstoff ist, und höchstens ein Sauerstoffatom oder Schwefelatom enthalten ist.

Die in der Definition des heterocyclischen Restes A umfassten Ringsysteme sind bedeutsam für die biologische Wirkung der erfindungsgemässen Verbindungen der Formel 1. Diese Ringsysteme enthalten als Ringglied mindestens ein Heteroatom, das heisst mindestens eines der den zugrundeliegenden ringförmigen Grundkörper bildenden Atome ist von Kohlenstoff verschieden. Grundsätzlich sind alle Atome des periodischen Systems der Elemente befähigt, als Ringglieder zu fungieren, sofern sie mindestens zwei kovalente Bindungen bilden können. Der heterocyclische Rest ist dabei bevorzugt ungesättigt und über ein Kohlenstoffatom als Ringglied an den Grundkörper der Formel I gebunden. Ungesättigte Ringsysteme der Definition A enthalten eine oder mehrere Doppelbindungen, vorzugsweise sind derartige Ringsysteme mehrfach ungesättigt und weisen im allgemeinen einen aromatischen Charakter auf. Bevorzugt sind solche Ringsysteme, welche als Heteroatom mindestens ein Stickstoffatom enthalten. Üblicherweise enthalten solche Ringe der Definition A ein bis drei Heteroatome aus der Reihe Sauerstoff, Schwefel und Stickstoff, wobei jeweils höchstens ein Sauerstoff- oder Schwefelatom enthalten ist. Bevorzugt sind Ringsysteme unter der Definition von A, worin der heterocyclische Rest A ein bis drei Heteroatome aus der Reihe Sauerstoff, Schwefel und Stickstoff enthält, wovon stets ein Heteoatom Stickstoff ist und höchstens ein Sauerstoffatom oder Schwefelatom enthalten ist. Insbesondere finden sich Beispiele für erfindungsgemässe Heterocyclen der Definition A in der Gruppe von Grundkörpern der folgenden Strukturen:

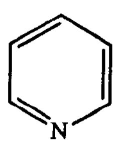 , 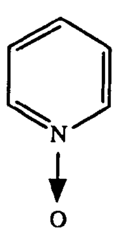 , 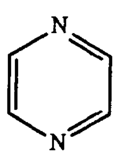 , 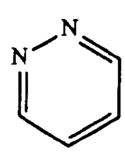 , 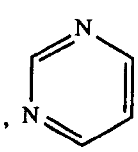 , 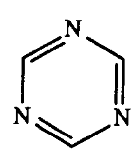 , 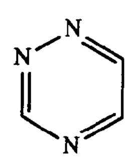 ,

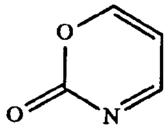 , 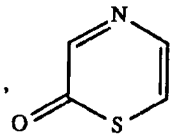 , 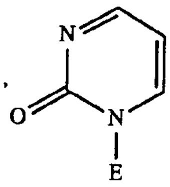 , 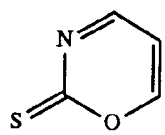 , 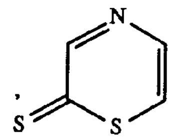 , 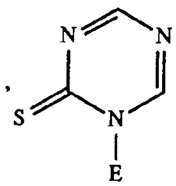 ,

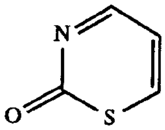 , 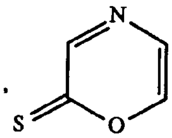 , 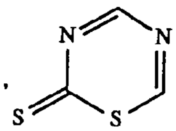 , 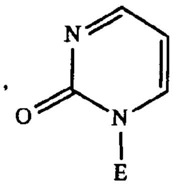 , 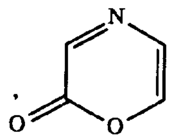 ,

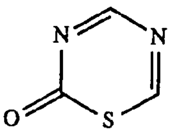 , 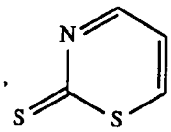 , 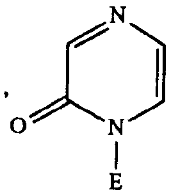 , 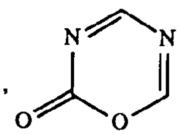 , 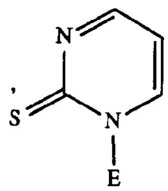 , 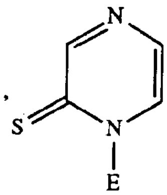 ,

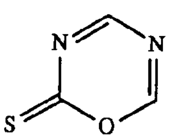 , 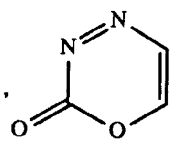 , 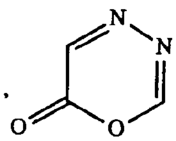 , 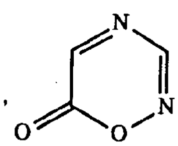 , 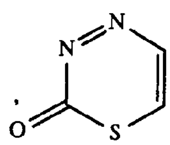 ,

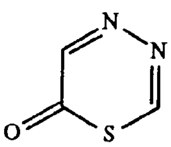 , 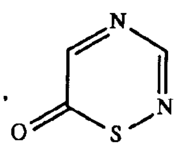 , 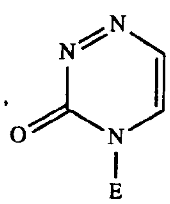 , 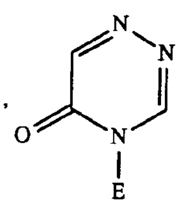 , 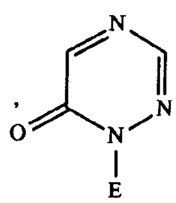 ,

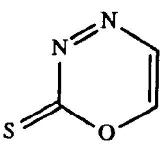 , 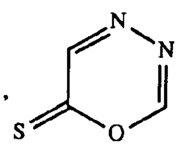 , 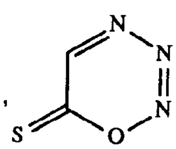 , 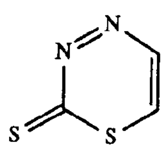 , 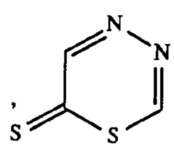 ,

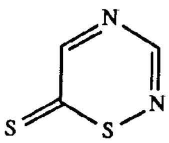 , 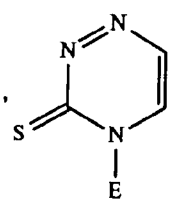 , 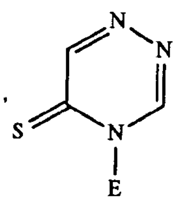 , 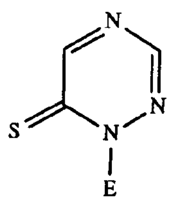 , 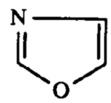 ,

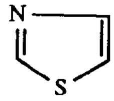 , 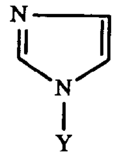 , 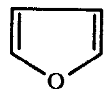 , 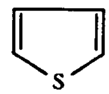 ,

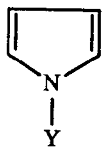 , 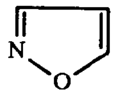 , 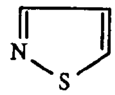 , 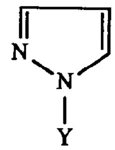 ,

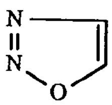 , 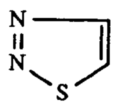 , 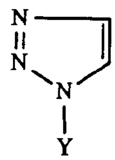 , 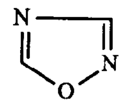 , 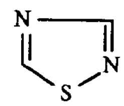 ,

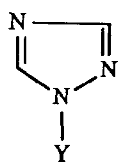 , 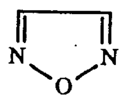 , 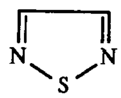 , 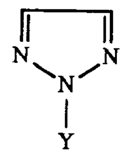 , 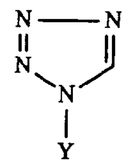 ,

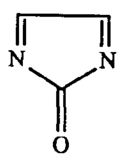 , 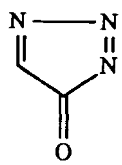 , 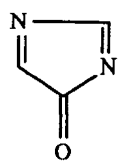 , 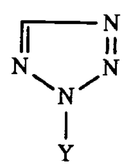 , 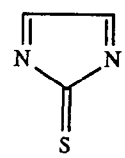

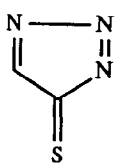 , 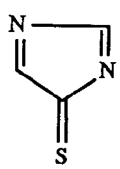 , 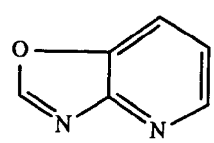 , 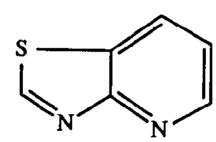 , 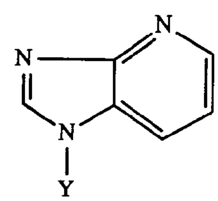 ,

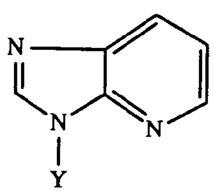 , 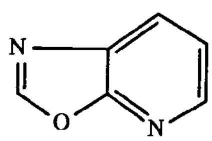 , 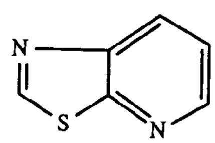 , 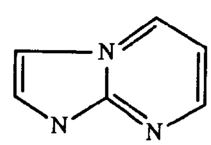 ,

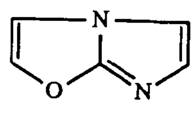 , 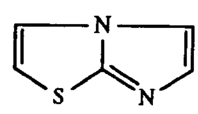 , 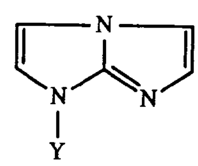 ,

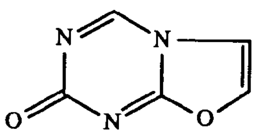 , 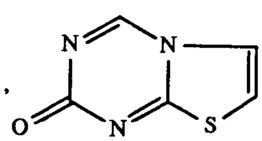 , 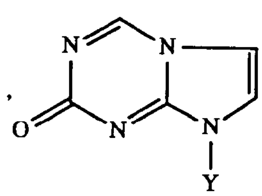 , 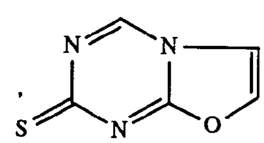 ,

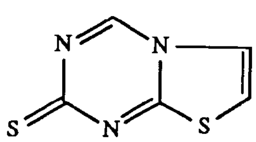 und 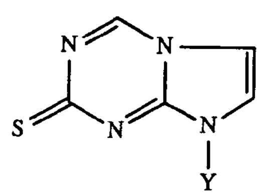 .

In den obigen Formeln steht

E für $C_1$-$C_3$-Alkyl und

Y für Wasserstoff, $C_1$-$C_3$-Alkyl oder Cyclopropyl.

Die vorstehend als Beispiele aufgezählten Heterocyclen A können unsubstituiert sein oder je nach Substitutionsmöglichkeiten des Ringsystems bis zu vier Substituenten tragen, wie sie unter Formel I angegeben sind. Bevorzugt tragen diese Heterocyclen ein bis drei Substituenten aus der Gruppe Halogen, $C_1$-$C_3$-Alkyl, $C_1$-$C_3$-Halogenalkyl und $C_1$-$C_3$-Halogenalkoxy mit je 1 bis 7 Halogenatomen und $C_1$-$C_3$-Alkoxy. Besonders bevorzugte Heterocyclen A sind Pyridylreste oder Thiazolylreste, wie zum Beispiel 3-Pyridyl, 2-Halogenpyrid-5-yl, 2,3-Dihalogenpyd-5-yl, 2-Halogenthiazol-4-yl, 1-Oxopyd-3-yl, 1-Oxo-2-halogenpyrid-5-yl und 1-Oxo-2,3-dihalogenpyrid-5-yl.

Vorzugsweise steht in der Verbindung der Formel I der Rest B für einen Phenyl-, Pyridyl-oder Thiazolylrest, welcher unsubstituiert oder durch ein bis zwei Reste aus der Gruppe Halogen, $C_1$-$C_3$-Alkyl, $C_1$-$C_3$-Halogenalkyl sowie $C_1$-$C_3$-Halogenalkoxy mit 1 bis 7 Halogenatomen und $C_1$-$C_3$-Alkoxy substituiert sein kann.

Unter den Verbindungen der Formel I sind wegen ihrer biologischen Merkmale solche herauszuheben, in denen $R_1$ Wasserstoff, $R_2$ Methyl, Äthyl oder Cyclopropyl; und A Pyridyl, 1-Oxopyridyl, Thiazolyl oder jeweils durch ein bis drei Substituenten aus der Gruppe Halogen, $C_1$-$C_3$-Alkyl, $C_1$-$C_3$-Halogenalkyl sowie $C_1$-$C_3$-Halogenalkoxy mit 1 bis 7 Halogenatomen und $C_1$-$C_3$-Alkoxy substituiertes Pyridyl, 1-Oxopyridyl oder Thiazolyl bedeuten. In diesem Sinne sind auch solche Verbindungen der Formel I interessant, worin

a) $R_1$ für Wasserstoff steht; und/oder

b) $R_2$ Methyl bedeuten; und/oder

c) $R_3$ $C_1$-$C_3$-Alkyl, Cyclopropyl, Cyclohexyl, Phenyl, Benzyl oder den Rest $-CH_2-COO-CH_3$ bedeutet.

Ferner sind erfindungsgemäss folgende Verbindungstypen der Formel I interessant, die dadurch gekennzeichnet sind, dass

$R_3$ mit 1 bis 3 Ringsubstituenten aus der Gruppe Fluor, Chlor, Brom, $C_1$-$C_2$-Alkyl, $C_1$-$C_2$-Halogenalkyl, $C_1$-$C_2$-Alkoxy, $C_1$-$C_2$-Alkylthio, Nitro und Cyano substituiertes Benzyl oder Phenyl bedeutet;

$R_3$ mit einer Hydroxy-Gruppe substituiertes $C_1$-$C_6$-Alkyl bedeutet;

$R_3$ mit einer $C_1$-$C_5$-Alkoxycarbonyl-Gruppe substituiertes $C_1$-$C_6$-Alkyl bedeutet;

$R_3$ $-CH_2CH_2F$, $-CH_2CH_2Br$, $-CH_2CH_2CH_2Cl$, $-CH_2CH_2CH_2Br$ oder $-CH_2CHClCH_2CH_2CH_2Cl$ bedeutet;

$R_3$ $-CH_2CH_2O-CH_3$, $-CH_2CH_2CH_2-O-CH_2CH_3$, $-CH(CH_3)CH_2-O-CH_3$, $-CH_2CH(OCH_3)_2$, $-CH_2CH_2-N(CH_2CH_3)_2$, $-CH_2-CH_2CH_2-N(CH_3)_2$ oder $-CH_2CH_2CH_2-N(CH_2CH_3)_2$ bedeutet;

$R_3$ unsubstituiertes oder mit ein oder zwei $C_1$-$C_4$-Alkylresten substituiertes $C_4$-$C_6$-Cycloalkyl bedeutet;

$R_3$ Cyclopentyl oder Cyclohexyl bedeutet;

$R_3$ mit einer oder zwei Methylgruppen substituiertes $C_3$-$C_6$-Cycloalkylbedeutet;

A 2-Chlorthiazol-4-yl, 2,3-Dichlorpriyd-5-yl, 1-Oxopyrid-3-yl oder 1-Oxo-2-chlorpyrid-5-yl; $R_2$ Methyl und $R_3$ Cyclopropyl, $-CH_2CH_2Cl$, $-CH_2CH(OCH_3)_2$ oder $-CH_2CH_2N(CH_3)_2$ bedeuten;

A 2-Chlorthiazol-4-yl bedeutet;

A 2-Chlorpyrid-5-yl bedeutet; oder

A 2-Chlorpyrid-5-yl, 2,3-Dichlorpyrid-5-yl, 2-Chlorthiazol-4-yl, 1-Oxopyrid-3-yl oder 1 -Oxo-2-chlorpyrid-5-yl; $R_1$ Wasserstoff, $R_2$ Methyl; und $R_3$ n-Propyl bedeuten.

Die erfindungsgemässen Verbindungen der Formel I können hergestellt werden indem man beispielsweise entweder

a) eine Verbindung der Formel II

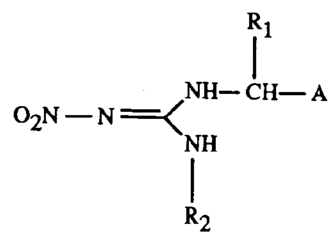

(II)

mit Formaldehyd, bzw. Paraformaldehyd und einer Verbindung der Formel III

$H_2N\text{-}R_3$ (III)

umsetzt; oder

b) eine Verbindung der Formel IV

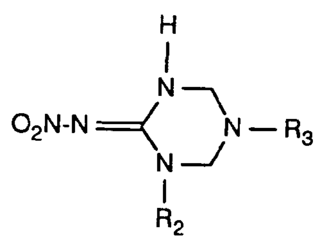

(IV)

mit einer Verbindung der Formel V

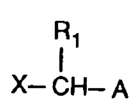

(V)

umsetzt; oder

c) zur Herstellung einer Verbindung der Formel I, worin $R_2$ eine von Wasserstoff abweichende Bedeutung hat, eine erhaltene Verbindung der Formel I, worin $R_2$ Wasserstoff bedeutet, mit einer Verbindung der Formel VI

$Y\text{-}R_2$ (VI)

umsetzt;

und dass man erwünschtenfalls eine erhaltene Verbindung der Formel I in an sich bekannter Weise in ein Salz derselben überführt; wobei in den Formeln II bis VI $R_1$, $R_2$, $R_3$ und A die oben angegebenen Bedeutungen haben, und X für Halogen und Y für eine Abgangsgruppe steht. Als Abgangsgruppe X und Y können z.B. in Betracht kommen: Halogen, vorzugsweise Chlor, Brom oder Jod, oder Sulfonsäurereste, wie Alkylsulfonsäurereste, Mesilat oder Tosylat.

Die Durchführung der Variante a) des obigen erfindungsgemässen Verfahrens erfolgt mit Vorteil unter normalem Druck, gegebenenfalls auch unter erhöhtem Druck in einem inerten Lösungsmittel und bei Temperaturen zwischen 0°C und +140°C, insbesondere zwischen +20°C und +120°C. Als Lösungsmittel eignen sich in besonderer Weise Alkohole, wie Methanol, Äthanol und Propanol, sowie Wasser. Weitere geeignete Lösungsmittel sind z.B. aromatische Kohlenwasserstoffe, wie Benzol, Toluol und Xylol; Aether, wie Tetrahydrofuran, Dioxan und Diäthyläther; halogenierte Kohlenwasserstoffe, wie Methylenchlorid, Chloroform, Tetrachlorkohlenstoff und Chlorbenzol sowie andere Lösungsmittel, die die Reaktion nicht beeinträchtigen. Die Lösungsmittel

können auch als Gemische verwendet werden. Gegebenenfalls wird unter Zugabe eines Säurekatalysators, wie HCl, $H_2SO_4$, oder einer Sulfonsäure, wie p-Toluolsulfonsäure, gearbeitet. Das enstehende Reaktionswasser kann gegebenenfalls mittels eines Wasserabscheiders oder durch Molekularsieb-Zusatz entfernt werden

Die vorerwähnten Verfahrensvarianten b) und c) können vorzugsweise unter normalem oder leicht erhöhtem Druck und in Gegenwart von vorzugsweise aprotischen Lösungs- oder Verdünnungsmitteln durchgeführt werden. Als Lösungs- oder Verdünnungsmittel eignen sich z.B. Aether und ätherartige Verbindungen, wie Diäthyläther Dipropyläther, Dibutyläther, Dioxan, Dimethoxyäthen und Tetrahydrofuran; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Dimethylformamid. Die Verfahren werden im allgemeinen bei einer Temperatur von -20 bis + 140°C, vorzugsweise zwischen 0 und +120°C, vorzugsweise in Gegenwart einer Base, durchgeführt. Als Basen eignen sich z.B. Carbonate, wie Natrium- und Kaliumcarbonat. Auch Hydride, wie Natriumhydrid, Kaliumhydrid und Calciumhydrid, können als Basen eingesetzt werden.

Die Ausgangsprodukte der Formeln II, III, V und VI sind bekannt oder können in Analogie zu bekannten Verfahren hergestellt werden.

Die als Ausgangsverbindungen der Formel II verwendeten 2-Nitroguanidinderivate und deren Herstellung sind aus den EP Patentanmeldungen 375.907 und 376.279 bekannt. Die primären Amine der Formel III sind handelsübliche und leicht zugängliche Produkte.

Die 2-Nitroimino-1,3,5-triazole der Formel IV, die ebenfalls einen Gegenstand der vorliegenden Erfindung darstellen, sind erhältlich, indem man ein 2-Nitroguanidin der Formel VII

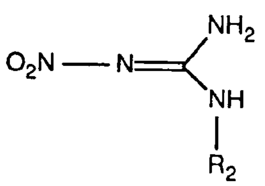

(VII)

mit Formaldehyd, bzw. Paraformaldehyd, und einer Verbindung der Formel III

$H_2N\text{-}R_3$ (III)

umsetzt, wobei in der Formeln VII und III $R_2$ und $R_3$ die oben angegebenen Bedeutungen haben. Bei diesem Verfahren sind die Reaktionsbedingungen die gleichen wie bei der obigen Verfahrensvariante a) zur Herstellung der Verbindungen der Formel I. Die Verbindungen der Formel IV sind neu, bis auf 2-Nitroimino-5-methyl-1,3,5-triazacyclohexan (EP-Patentanmeldung 0.386.565) und 2-Nitroimino-1,3,5-triazacyclohexan (US-PS 4,937,340). Die Nitroguanidine der Formel VII sind bekannt (vgl. US-PS 4,804,780 und 4,221,802) oder können in analoger Weise hergestellt werden.

Verbindungen der Formel V sind in grosser Zahl bekannt (vgl. z.B. EP-Patentanmeldung 375,907 und 376,279). Bevorzugt werden als Ausgangsprodukte diejenigen Verbindungen der Formel V verwendet, worin X Chlor bedeutet.

Verbindungen der Formel VI sind ebenfalls in grosser Zahl bekannt sie sind handelsübliche oder -analog bekannter Verfahren - leicht zugängliche Produkte. Die Abgangsgruppe Y in diesen Verbindungen ist vorzugsweise ein Halogenatom, speziell Chlor.

Es ist bereits bekannt, dass bestimmte offenkettige 2-Nitroguanidinderivate pestizide Eigenschaften besitzen (vgl. z.B. EP-Patentanmeldungen 0 375 907 und 0 376 279). Es sind jedoch auch pestizide heterozyklische Verbindungen, welche auf einer Nitroguanidinstruktur basieren bekannt. So werden in den EP-Patentanmeldungen 0192 060 und 0 259 738 2-Nitroiminopyrimidin-Derivate mit insektizider Wirkung beschrieben. Ferner werden in der US-PS 4.937.340 das 2-Nitroimino-1,3,5-triazacyclohexan und weitere Nitrogruppen enthaltende entsprechende Derivate als Sprengstoff-Zusätze vorgeschlagen. Insektizide Verbindungen vom erfindungsgemässen Typ werden in der EP-Patentanmeldung 0 386 565 vorgeschlagen, wobei die erfindungsgemässen Verbindungen der Formel I fachweise vom Anspruchs-Umfang dieser EP-Patentanmeldung umfasst werden.

Es wurde überraschenderweise gefunden, dass die erfindungsgemässen Verbindungen der Formel I bei günstiger Warmblüter-, Fisch- und Pflanzenverträglichkeit wertvolle Wirkstoffe in der Schädlingsbekämpfung sind. Insbesondere betrifft die Anwendung der erfindungsgemässen Wirkstoffe Insekten, die in Nutz- und Zierpflanzen in der Landwirtschaft, insbesondere in Baumwoll-, Gemüse- und Obstpflanzungen, im Forst, im Vorrats- und Materialschutz sowie im Hygienesektor insbesondere an Haus- und Nutztieren vorkommen. Die Verbindungen sind vor allem wirksam gegen saugende pflanzenschädigende Insekten, insbesondere gegen Aphiden und Zikaden. Sie sind gegen alle oder einzelne Entwicklungsstadien von normal sensiblen aber auch resistenten Arten wirksam. Dabei kann sich ihre Wirkung in unmittelbarer Abtötung der Schädlinge oder erst

nach einiger Zeit, beispielsweise bei einer Häutung, oder in einer verminderten Eiablage und/oder Schlupfrate zeigen. Zu den oben erwähnten Schädlingen gehören:
aus der Ordnung Lepidoptera zum Beispiel
Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Yponomeuta spp.;
aus der Ordnung Coleoptera zum Beispiel
Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp. Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.;
aus der Ordnung der Orthoptera zum Beispiel Blatta spp., Blattella spp., Gryllotalpa spp., Leucophaea maderae, Locusta spp., Periplaneta spp. und Schistocerca spp.;
aus der Ordnung der Isoptera zum Beispiel
Reticulitermes spp.; aus der Ordnung der Psocoptera zum Beispiel Liposcelis spp.; aus der Ordnung der Anoplura zum Beispiel Haematopinus spp., Linognathus spp. Pediculus spp., Pemphigus spp. und Phylloxera spp.;
aus der Ordnung der Mallophaga zum Beispiel Damalinea spp. und Trichodectes spp.;
aus der Ordnung der Thysanoptera zum Beispiel
Frankliniella spp., Hercinothrips spp., Taeniothrips spp., Thrips palmi, Thrips tabaci und Scirtothrips aurantii;
aus der Ordnung der Heteroptera zum Beispiel
Cimex spp., Distantiella theobroma, Dysdercus spp., Euchistus spp. Erygaster spp. Leptocorisa spp., Nezara spp., Piesma spp., Rhodnius spp., Sahlbergella singularis, Scotinophara spp. und Triatoma spp.;
aus der Ordnung der Homoptera zum Beispiel
Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;
aus der Ordnung der Hymenoptera zum Beispiel
Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;
aus der Ordnung der Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp. Oscinella frit, Pegomyia hyoscyami, Phorbia spp., Rhagoletis pomonella, Sciara spp., Stomoxys spp., Tabanus spp., Tannia spp. und Tipula spp.;
aus der Ordnung der Siphonaptera z.B.
Ceratophyllus spp., Xenopsylla cheopis,
aus der Ordnung der Akarina zum Beispiel
Acarus siro, Aceria sheldoni, Aculus schlechtendali, Amblyomma spp., Argas spp., Boophilus spp., Brevipalpus spp., Bryobia praetiosa, Calipitrimerus spp., Chorioptes spp., Dermanyssus gallinae, Eotetranychus carpini, Eriophyes spp., Hyalomma spp., Ixodes spp., Olygonychus pratensis, Ornithodoros spp., Panonychus spp., Phyllocoptruta oleivora, Polyphagotarsonemus latus, Psoroptes spp., Rhipicephalus spp., Rhizoglyphus spp., Sarcoptes spp., Tarsonemus spp. und Tetranychus spp.; und
aus der Ordnung der Thysanura zum Beispiel
Lepisma saccharina.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen und der sie enthaltenden Mittel lässt sich durch Zusatz von anderen Insektiziden und/oder Akariziden wesentlich verbreitern und an gegebene Umstände anpassen. Als Zusätze kommen zum Beispiel Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher beispielsweise zu emulgierbaren Konzentraten, direkt versprüh- oder verdünnbaren Lösungen, verdünnten Emulsionen, Spritzpulvern, löslichen Pulvern, Stäubemitteln, Granulaten, auch Verkapselungen in polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt. Ferner eignen sich die Verbindungen der Formel I auch für den Einsatz bei der Behandlung von Saatgut. Dabei kann sowohl das Saatgut vor dem Säen mit dem Wirkstoff oder einer den Wirkstoff enthaltenden Formulierung behandelt oder gebeizt werden, als auch der Wirkstoff beim Säen in die Saatfurche appliziert werden.

Die Formulierung, das heisst die den Wirkstoff der Formel I, beziehungsweise Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, zum Beispiel durch inniges Vermischen und/oder Vermahlen der Wirkstoffe mit Streckmitteln, wie beispielsweise mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$ von Alkylbenzolen wie Xylolgemische oder alkylierte Naphthaline, aliphatische oder cycloaliphatische Kohlenwasserstoffe, wie Cyclohexan, Paraffine oder Tetrahydronaphthalin, Alkohole wie Aethanol, Propanol oder Butanol, und Glykole sowie deren Aether und Ester, wie Propylenglykol, Dipropylenglykoläther, Aethylenglykol, Aethylenglykolmonomethyl- oder -äthyläther, Ketone, wie Cyclohexanon, Isophoron oder Diacetonolalkohol, starke polare Lösungsmittel, wie N-Methyl-2-pyrrolidon, Dimethylsulfoxid oder Dimethylformamid oder Wasser, Pflanzenöle, wie Raps-, Rizinus-, Kokosnuss- oder Sojaöl; gegebenenfalls auch Silikonöle.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und-/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie die Natrium- oder Kalium-Salze der Oel- oder Stearinsäure, oder von natürlichen Fettsäuregemischen, die beispielsweise aus Kokosnuss- oder Tallöl gewonnen werden können. Ferner sind als Tenside auch die Fettsäuremethyl-taurin-salze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, beispielsweise das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind zum Beispiel die Natrium-, Calcium- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie zum Beispiel Salze des Phosphorsäureesters eines p-Nonylphenol-(4-14)-Aethylenoxid-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quartemäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, beispielsweise das Stearyltrimethylammonium-chlorid oder das Benzyl-di-(2-chloräthyl)-äthylammoniumbromid.

Die in der Formulierungstechnik gebräuchlichen Tenside sind beispielsweise in folgenden Publikationen beschrieben:

"Mc Cutcheon's Detergents and Emulsifiers Annual", Mc Publishing Corp., Glen Rock, NJ, USA, 1988",

H. Stache, "Tensid-Taschenbuch", 2. Aufl., C. Hanser Verlag München, Wien 1981.

M. and J. Ash. "Encyclopedia of Surfactants", Vol. I-III, Chemical Publishing Co., New York, 1980-1981.

Die pestiziden Zubereitungen enthalten in der Regel 0,1 bis 99 %, insbesondere 0,1 bis 95 % Wirkstoff der Formel I oder Kombinationen dieses Wirkstoffs mit andern Insektiziden oder Akariziden, 1 bis 99,9 % eines festen oder flüssigen Zusatzstoffes und 0 bis 25 %, insbesondere 0,1 bis 25 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 1 bis 1000 g Wirkstoff pro Hektar, vorzugsweise 25 bis 500 g/ha.

Insbesondere setzen sich bevorzugte Formulierungen folgendermassen zusammen: (% = Gewichtsprozent), wobei als aktiver Wirkstoff ein solcher der Formel I zu verstehen ist:

Emulgierbare Konzentrate:

| | |
|---|---|
| Aktiver Wirkstoff: | 1 bis 90 %, bevorzugt 5 bis 20 % |
| oberflächenaktives Mittel: | 1 bis 30 %, vorzugsweise 10 bis 20 % |
| flüssiges Trägermittel: | 5 bis 94 %, vorzugsweise 70 bis 85 % |

Stäube:

| | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

Suspension-Konzentrate:

| | |
|---|---|
| Aktiver Wirkstoff: | 5 bis 75 %, vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30 % |

Benetzbare Pulver:

| | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermaterial: | 5 bis 95 %, vorzugsweise 15 bis 90 % |

Granulate:

| | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 % |

Die Mittel können auch weitere Zusätze wie Stabilisatoren, z.B. gegebenenfalls epoxidierte Pflanzenöle (epoxidiertes Kokosnussöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe zur Erzielung spezieller Effekte enthalten.

Die folgenden Beispiele dienen der Erläuterung der Erfindung. Sie schränken die Erfindung nicht ein.

Beispiel 1 (Herstellung von Auspangsprodukten der Formel IV):

a) Herstellung von 2-Nitroimino-5-methyl-1,3,5-triazacyclohexan

Ein Gemisch aus 26.0 g 2-Nitroguanidin, 31.1 ml einer 8M Lösung von Methylamin in Aethanol; 38 ml einer 37%igen Lösung von Formaldehyd in Wasser und 100 ml Aethanol wird für 2 Std. auf 50°C erhitzt und anschliessend filtriert. Die abfiltrierten Kristalle werden 3-mal mit je 20 ml Aethanol gewaschen und anschliessend getrocknet. Man erhält die Titelverbindung, Smp. 173-175°C, der Formel

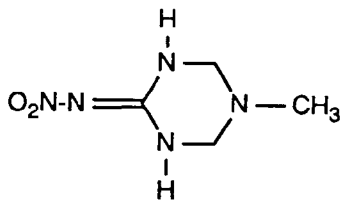

(Verbindung Nr. 2.001)

b) Herstellung von 1-Methyl-2-nitroimino-5-n-propyl-1,3,5-triazacyclohexan:

Ein Gemisch aus 17.1 g 1-Methyl-2-nitroguanidin, 12,0 ml n-Propylamin, 22,0 ml einer 37%igen Lösung von Formaldehyd in Wasser und 40 ml Aethanol wird während 4 Std. auf 50°C erhitzt. Dann werden weitere 7,0 ml n-Propylamin und 13,0 ml einer 37%igen Lösung von Formaldehyd in $H_2O$ zugegeben. Nach zweistündigem Rühren bei 50°C wird das Reaktionsgemisch im Vakuum eingedampft und die abgeschiedenen Kristalle werden mit Aether verrührt. Man erhält 26.9 g der Titelverbindung Smp. 84-86°C, der Formel

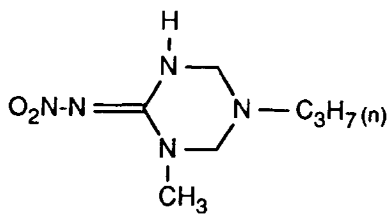

(Verbindung Nr. 2.011)

c) Herstellung von 1-Methyl-2-nitroimino-5-phenyl-1,3,5-triazacyclohexan:

Ein Gemisch aus 2.36 g 1-Methyl-2-nitroguanidin, 2.11 ml Anilin und 1.80 g Paraformaldehyd in 30 ml Toluol wird mit 3 Tropfen konzentrierter HCl-Lösung versetzt und anschliessend während 6 Std. am Wasserabscheider gekocht. Dann wird das Reaktionsgemisch im Vakuum eingedampft und das erhaltene Rohprodukt aus Methanol umkristallisiert. Man erhält die Titelverbindung, Smp.: 169-172°C, der Formel

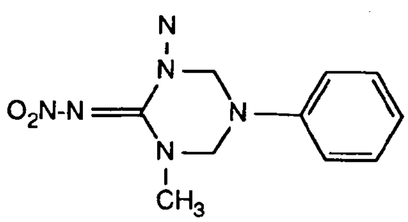

(Verbindung Nr. 2.044)

Wie vorstehend angegeben können die folgenden Verbindungen der Formel IV hergestellt werden:

| Verb. Nr. | $R_2$ | $R_3$ | phys. Daten |
|---|---|---|---|
| 2.001 | H | $CH_3$ | Smp. 173-175°C |
| 2.002 | H | $-C_2H_5$ | Smp. 181-182°C |
| 2.003 | H | $-C_3H_7(n)$ | |
| 2.004 | H | $CH(CH_3)_2$ | |
| 2.005 | H | 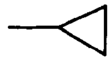 | Smp. 225-227°C |
| 2.006 | H | 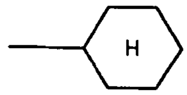 | |
| 2.007 | H | 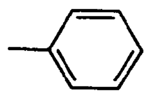 | |
| 2.008 | H | 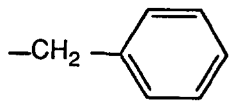 | |
| 2.009 | $CH_3$ | $-CH_3$ | Smp. 134-135°C |
| 2.010 | $CH_3$ | $-C_2H_5$ | Smp. 112°C |
| 2.011 | $CH_3$ | $-C_3H_7(n)$ | Smp. 84-86°C |
| 2.012 | $CH_3$ | $-CH_2(CH_3)_2$ | Smp. 154°C |
| 2.013 | $CH_3$ | 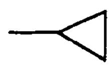 | Smp. 177°C |
| 2.014 | $CH_3$ | 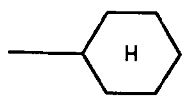 | Smp. 103-104°C |
| 2.015 | $CH_3$ | $-C_6H_5$ | Smp. 169-172°C |
| 2.016 | $CH_3$ | 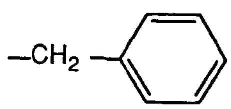 | Smp. 161-163°C |
| 2.017 | $-C_2H_5$ | $-CH_3$ | |
| 2.018 | $-C_2H_5$ | $-C_2H_5$ | Smp. 95-96°C |
| 2.019 | $-C_2H_5$ | $-C_3H_7(n)$ | |
| 2.020 | $-C_2H_5$ | $-CH(CH_3)_2$ | |

| Verb. Nr. | $R_2$ | $R_3$ | phys. Daten |
|---|---|---|---|
| 2.021 | $-C_2H_5$ | 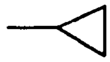 | |
| 2.022 | $-C_2H_5$ | 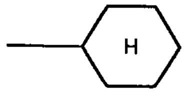 | |
| 2.023 | $-C_2H_5$ | 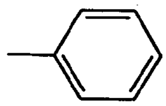 | |
| 2.024 | $-C_2H_5$ | 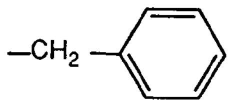 | |
| 2.025 | 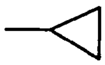 | $-CH_3$ | |
| 2.026 | 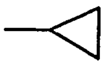 | $-C_2H_5$ | Smp. 138-139°C |
| 2.027 | 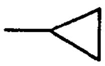 | $-C_3H_7$(n) | |
| 2.028 | 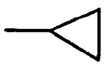 | $-CH(CH_3)_2$ | |
| 2.029 | 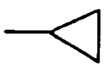 | 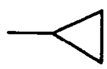 | |
| 2.030 | 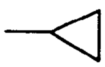 | 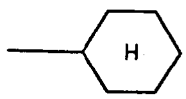 | |
| 2.031 | 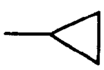 | 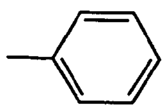 | |
| 2.032 | 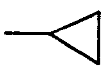 | 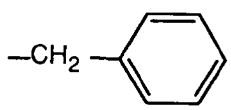 | |
| 2.033 | 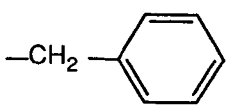 | $-CH_3$ | Smp. 109-111°C |
| 2.034 | 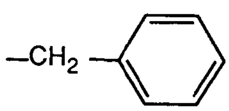 | $-C_2H_5$ | |

| Verb. Nr. | $R_2$ | $R_3$ | phys. Daten |
|---|---|---|---|
| 2.035 | 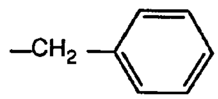 | $-C_3H_7(n)$ | |
| 2.036 | 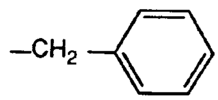 | $CH(CH_3)_2$ | |
| 2.037 | 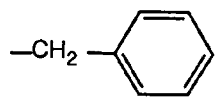 | 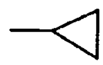 | |
| 2.038 | 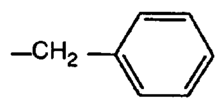 | 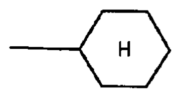 | |
| 2.039 | 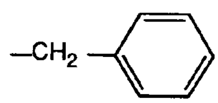 | 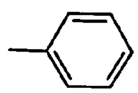 | |
| 2.040 | 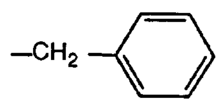 | 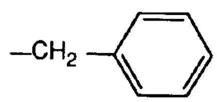 | |
| 2.041 | H | $-CH_2-COOCH_3$ | |
| 2.042 | $-CH_3$ | $-CH_2-COOCH_3$ | |
| 2.043 | 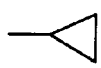 | $-CH_2-COOCH_3$ | |
| 2.044 | $-CH_3$ | 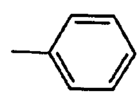 | Smp. 169-172°C |
| 2.045 | $-CH_3$ | $-CH_2CF_3$ | |
| 2.046 | $-CH_3$ | $-CH_2CH_2F$ | |
| 2.047 | $-CH_3$ | $-CH_2CH_2Br$ | |
| 2.048 | $-CH_3$ | $-CH_2CH_2CH_2Cl$ | |
| 2.049 | $-CH_3$ | $-CH_2CH_2CH_2Br$ | |
| 2.050 | $-CH_3$ | $-CH_2CH_2Cl$ | |
| 2.051 | $-CH_3$ | $-CH_2CH(Cl)CH_2CH_2CH_2Cl$ | |
| 2.052 | $-CH_3$ | $-CH_2CH_2OH$ | Smp. 121-123°C |
| 2.053 | $-CH_3$ | $-CH_2CH_2CH_2OH$ | |
| 2.054 | $-CH_3$ | $-CH_2CH_2CH_2CH_2OH$ | Smp. 81-83°C |

| Verb. Nr. | $R_2$ | $R_3$ | phys. Daten |
|---|---|---|---|
| 2.055 | $-CH_3$ | $-CH_2CH_2CH_2CH_2CH_2OH$ | |
| 2.056 | $-CH_3$ | $-CH(CH_3)CH_3OH$ | |
| 2.057 | $-CH_3$ | $-CH(C_2H_5)CH_2OH$ | |
| 2.058 | $-CH_3$ | $-CH_2CH(CH_3)OH$ | |
| 2.059 | $-CH_3$ | $-CH_2CH(OH)CH_2OH$ | |
| 2.060 | $-CH_3$ | $-CH(CH_2OH)_2$ | |
| 2.061 | $-CH_3$ | $-CH_2CH_2OCH_3$ | |
| 2.062 | $-CH_3$ | $-CH_2CH_2CH_2OC_2H_5$ | |
| 2.063 | $-CH_3$ | $-CH(CH_3)CH_2OCH_3$ | |
| 2.064 | $-CH_3$ | $-CH_2CH(OCH_3)_2$ | |
| 2.065 | $-CH_3$ | $-CH_2CH(OC_2H_5)_2$ | |
| 2.066 | $-CH_3$ | $-CH_2CH_2N(CH_3)_2$ | |
| 2.067 | $-CH_3$ | $-CH_2CH_2N(C_2H_5)_2$ | |
| 2.068 | $-CH_3$ | $-CH_2CH_2CH_2N(CH_3)_2$ | |
| 2.069 | $-CH_3$ | $-CH_2CH_2CH_2N(C_2H_5)_2$ | |
| 2.070 | $-CH_3$ | $-CH_2COOC_2H_5$ | |
| 2.071 | $-CH_3$ | $CH_2CH_2COOC_2H_5$ | Smp. 110-112°C |
| 2.072 | $-CH_3$ | $-CH(CH_3)CH_2COOC_2H_5$ | |
| 2.073 | $-CH_3$ | $-CH(CH_2OH)COOCH_3$ | |
| 2.074 | $-CH_3$ | 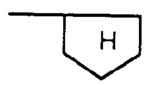 | |
| 2.075 | $-CH_3$ | 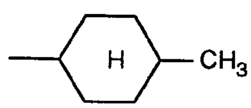 | Smp. 151-153°C (cis-Isomer)<br>Smp. 138-140°C (trans-Isomer) |
| 2.076 | $-CH_3$ | 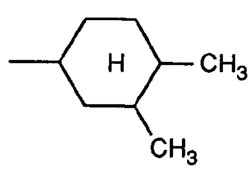 | |
| 2.077 | $-CH_3$ | 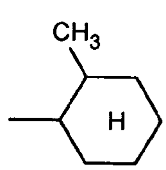 | |

| Verb. Nr. | $R_2$ | $R_3$ | phys. Daten |
|---|---|---|---|
| 2.078 | $-CH_3$ | $-CH_2-CH=CH_2$ | Smp. 53-55°C |
| 2.079 | $-CH_3$ | 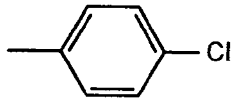 | |
| 2.080 | $-CH_3$ | 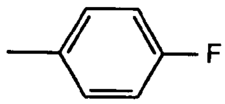 | Smp. 170-173°C |
| 2.081 | $-CH_3$ | 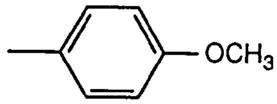 | Smp. 174-176°C |
| 2.082 | $-CH_3$ | 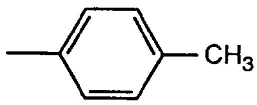 | Smp. 195-197°C |
| 2.083 | $-CH_3$ | 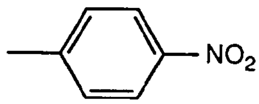 | Smp. 230°C |
| 2.084 | $-CH_3$ | 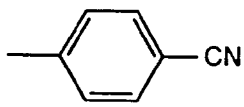 | Smp. 222-226°C |
| 2.085 | $-CH_3$ | 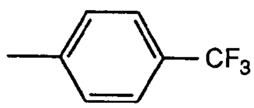 | Smp. 163-166°C |
| 2.086 | $-CH_3$ | 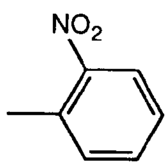 | |
| 2.087 | $-CH_3$ | 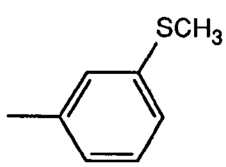 | |
| 2.088 | $-CH_3$ | 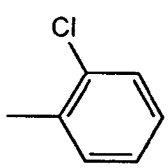 | |

| Verb. Nr. | $R_2$ | $R_3$ | phys. Daten |
|---|---|---|---|
| 2.089 | $-CH_3$ | 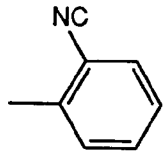 | |
| 2.090 | $-CH_3$ | 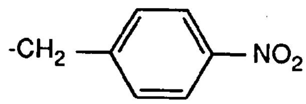 | Smp. 235-238°C |
| 2.091 | $-CH_3$ | 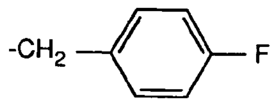 | Smp. 143-145°C |
| 2.092 | $-CH_3$ | 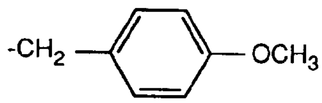 | Smp. 132-134°C |
| 2.093 | $-CH_3$ | 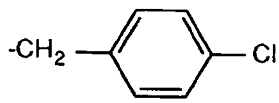 | Smp. 160-162°C |
| 2.094 | $-CH_3$ | 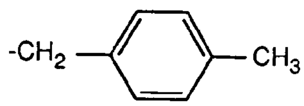 | Smp. 161-163°C |
| 2.095 | $-CH_3$ | 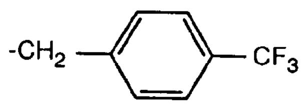 | Smp. 160-162°C |
| 2.096 | $-CH_3$ | 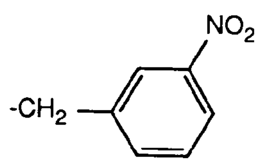 | |
| 2.097 | $-CH_3$ | 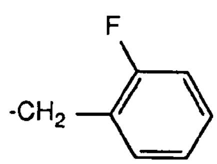 | |
| 2.098 | $-CH_3$ | 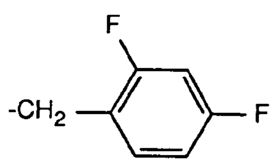 | |

| Verb. Nr. | $R_2$ | $R_3$ | phys. Daten |
|---|---|---|---|
| 2.099 | $-CH_3$ | 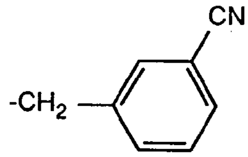 | |

Beispiel 2:

a) Herstellung von 1-(2-Chlorpyrid-5-ylmethyl)-2-nitroimino-5-äthyl-1,3,5-triazacyclohexan:

Ein Gemisch aus 1. 15 g 1-(2-Chlorpyrid-5-ylmethyl)-2-nitroguanidin, 0,75 ml einer 37%igen Lösung von Formaldehyd in Wasser, 0,32 ml einer 70%igen Lösung von Aethylamin in Wasser und 5 ml Aethanol werden für 4 Std. auf 50°C erhitzt. Dann wird das Reaktionsgemisch im Vakuum eingedampft, der Rückstand in 20 ml Aethanol aufgeschlemmt und die ausgefallenen Kristalle abfiltriert. Man erhält die Titelverbindung, Smp. 125-126°C, der Formel

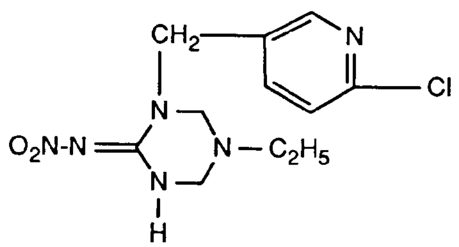

(Verbindung Nr. 1.001)

b) Herstellung von 1-(2-Chlorpyrid-5-ylmethyl)-2-nitroimino-5-cyclopropyl-1,3,5-triazacyclohexan:

Ein Gemisch aus 2,96 g 2-Nitroimino-5-cyclopropyl-1,3,5-triazacyclohexan, 2,59 2-Chlor-5-chlormethylpyridin und 2,43 g Kaliumcarbonat in 60 ml Acetonitril wird 16 Std. unter Rückfluss erhitzt. Das erhaltene Reaktionsgemisch wird filtriert, das Filtrat im Vakuum eingedampft und der gebildete Rückstand an Kieselgel mit Dichlormethan/Essigsäureäthylester (1:1) chromatographiert. Man erhält die Titelverbindung, Smp. 125-127°C, der Formel

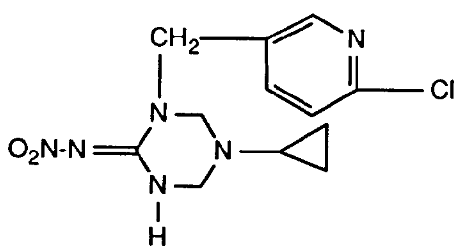

(Verbindung Nr. 1.003).

c) Herstellung von 1-(2-Chlorpyrid-5-ylmethyl)-2-nitroimino-3-methyl-5-n-propyl-1,3,5-triazacyclohexan:

Ein Gemisch aus 20,1 g 1-Methyl-2-nitroimino-5-n-propyl-1,3,5-triazacyclohexan, 16,2 g 2-Chlor-5-chlormethylpyridin, 0,17 g Cäsiumchlorid und 27,7 g Kaliumcarbonat in 150 ml DMF*) wird für 9 Std. auf 110°C erhitzt und dann über Celite filtriert. Das Filtrat wird im Vakuum eingedampft. Das erhaltene Rohprodukt wird in 200 ml Dichlormethan gelöst und mit 100 ml Wasser und 100 ml gesättigter NaCl-Lösung gewaschen, über $MgSO_4$ getrocknet und dann eingedampft. Der Rückstand wird aus Essigsäureäthylester umkristallisiert. Man erhält so die Titelverbindung, Smp.: 137-138°C, der Formel

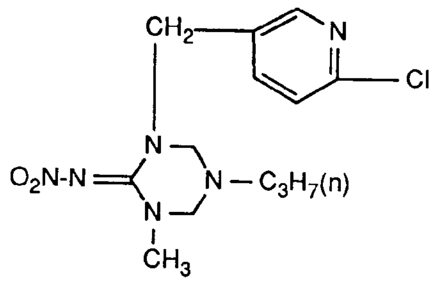

(Verbindung Nr. 1.009)

* Dimethylformamid

d) Herstellung von 1-(2-Chlorpyrid-5-ylmethyl)-2-nitroimino-3,5-di-(n-propyl)-1,3,5-triazacyclohexan:

Zu einer Lösung aus 3.12 g 1-(2-Chlorpyrid-5-ylmethyl)-2-nitroimino-5-n-propyl-1,3,5-triazacyclohexan in 50 ml Acetonitril werden 0,30 g Natriumhydrid (80 % in Weissöl) gegeben. Nach 3 Std. Rühren bei Raumtemperatum wird das Reaktionsgemisch mit 1.8 ml n-Propyljodid versetzt, dann 16 Std. bei Raumtemperatur und während 2 Std. bei 80°C gerührt. Nach dem Eindampfen im Vakuum wird der erhaltene Rückstand in 100 ml Essigsäureäthylester aufgenommen, mit 50 ml gesättigter NaCl-Lösung gewaschen, über $MgSO_4$ getrockent und wieder eingedampft. Die als Rückstand erhaltenen Kristalle werden bei 0°C aus Essigsäureäthylester umkristallisiert. Man erhält so die Titelverbindung, Smp. 112-113°C, der Formel

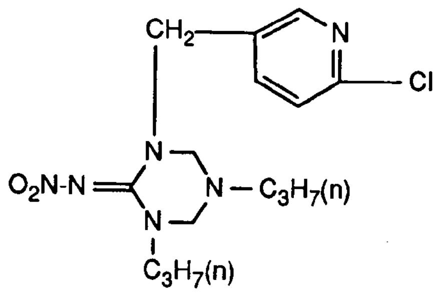

(Verbindung Nr. 1.025).

Wie vorstehend angegeben können die folgenden Verbindungen der Formel I hergestellt werden:

| Verb. Nr. | A | $R_1$ | $R_2$ | $R_3$ | phys. Daten |
|---|---|---|---|---|---|
| 1.001 | 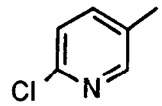 | H | H | $-C_2H_5$ | Smp. 125-126°C |
| 1.002 | 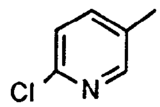 | H | H | $-C_3H_7$(n) | Smp. 115-117°C |
| 1.003 | 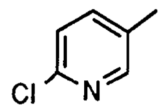 | H | H | 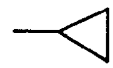 | Smp. 125-127°C |
| 1.004 | 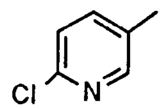 | H | H | 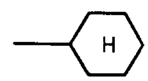 | Smp. 150-151°C |
| 1.005 | 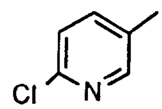 | H | H | 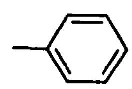 | Smp. 143-145°C |
| 1.006 | 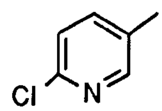 | H | H | 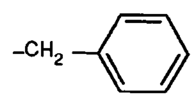 | Smp. 108-110°C |
| 1.007 | 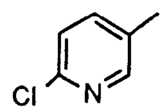 | H | $CH_3$ | $-CH_3$ | amorphe Masse |
| 1.008 | 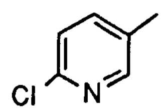 | H | $CH_3$ | $-C_2H_5$ | Smp. 124-125°C |
| 1.009 | 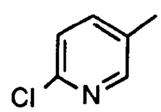 | H | $CH_3$ | $-C_3H_7$(n) | Smp. 137-138°C |
| 1.010 | 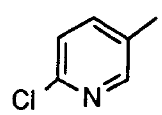 | H | $CH_3$ | $-CH(CH_3)_2$ | |
| 1.011 | 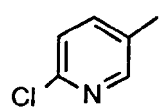 | H | $-CH_3$ | 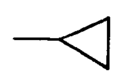 | Smp. 104-106°C |
| 1.012 | 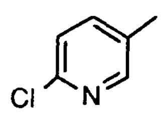 | H | $-CH_3$ | 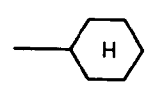 | Smp. 146-147°C |

| Verb. Nr. | A | $R_1$ | $R_2$ | $R_3$ | phys. Daten |
|---|---|---|---|---|---|
| 1.013 | 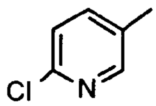 | H | $-CH_3$ | 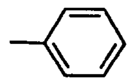 | Smp. 146-149°C |
| 1.014 | 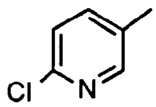 | H | $-CH_3$ | 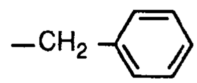 | Smp. 116-118°C |
| 1.015 | 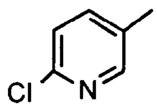 | H | $-C_2H_5$ | $-CH_3$ | |
| 1.016 | 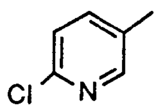 | H | $-C_2H_5$ | $-CH_2CH_3$ | Smp. 113-114°C |
| 1.017 | 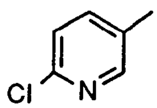 | H | $-C_2H_5$ | $-C_3H_7(n)$ | |
| 1.018 | 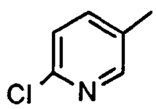 | H | $-C_2H_5$ | $-CH(CH_3)_2$ | |
| 1.019 | 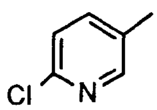 | H | $-C_2H_5$ | 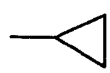 | |
| 1.020 | 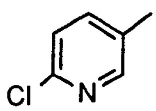 | H | $-C_2H_5$ | 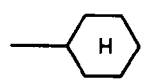 | |
| 1.021 | 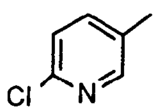 | H | $-C_2H_5$ | 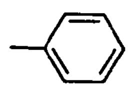 | |
| 1.022 | 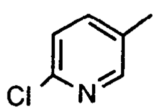 | H | $-C_2H_5$ | 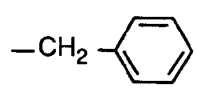 | |
| 1.023 | 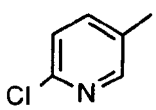 | H | $-C_3H_7(n)$ | $-CH_3$ | |
| 1.024 | 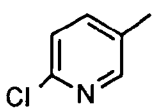 | H | $-C_3H_7(n)$ | $-C_2H_5$ | |

| Verb. Nr. | A | $R_1$ | $R_2$ | $R_3$ | phys. Daten |
|---|---|---|---|---|---|
| 1.025 | 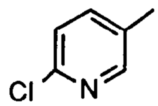 | H | $-C_3H_7(n)$ | $-C_3H_7(n)$ | Smp. 112-113°C |
| 1.026 | 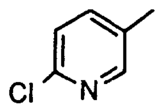 | H | $-C_3H_7(n)$ | $-CH(CH_3)_2$ | |
| 1.027 | 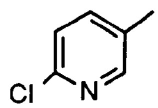 | H | $-C_3H_7(n)$ | 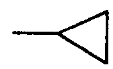 | |
| 1.028 | 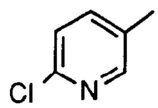 | H | $-C_3H_7(n)$ | 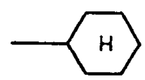 | |
| 1.029 | 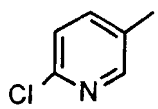 | H | $-C_3H_7(n)$ | 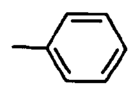 | |
| 1.030 | 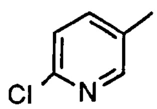 | H | $-C_3H_7(n)$ | 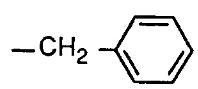 | |
| 1.031 | 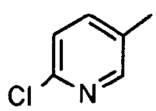 | H | $-CH(CH_3)_2$ | $-CH_3$ | |
| 1.032 | 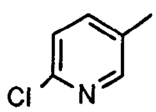 | H | $-CH(CH_3)_2$ | $-C_2H_5$ | |
| 1.033 | 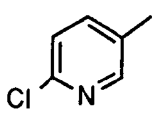 | H | $-CH(CH_3)_2$ | $-C_3H_7(n)$ | |
| 1.034 | 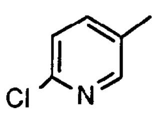 | H | $-CH(CH_3)_2$ | $-CH(CH_3)_2$ | |
| 1.035 | 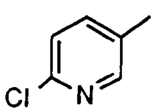 | H | $-CH(CH_3)_2$ | 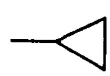 | |
| 1.036 | 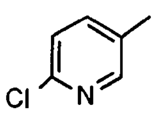 | H | $-CH(CH_3)_2$ | 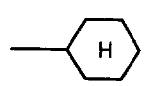 | |

| Verb. Nr. | A | $R_1$ | $R_2$ | $R_3$ | phys. Daten |
|---|---|---|---|---|---|
| 1.037 | 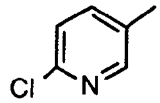 | H | $-CH(CH_3)_2$ | 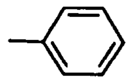 | |
| 1.038 | 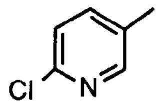 | H | $-CH(CH_3)_2$ | 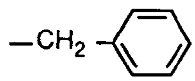 | |
| 1.039 | 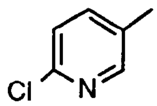 | H | 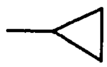 | $CH_3$ | |
| 1.040 | 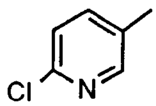 | H | 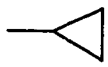 | $-C_2H_5$ | Smp. 115-116°C |
| 1.041 | 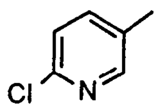 | H | 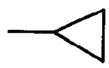 | $-C_3H_7(n)$ | |
| 1.042 | 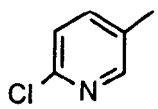 | H | 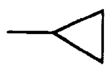 | $-CH(CH_3)_2$ | |
| 1.043 | 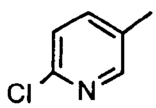 | H | 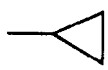 | 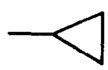 | |
| 1.044 | 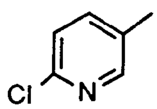 | H | 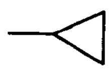 | 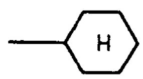 | |
| 1.045 | 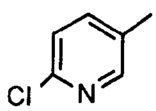 | H | 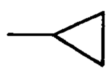 | 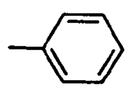 | |
| 1.046 | 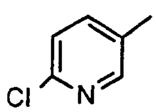 | H | 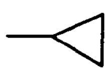 | 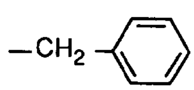 | |
| 1.047 | 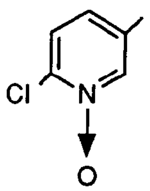 | H | H | $-CH_3$ | |

| Verb. Nr. | A | $R_1$ | $R_2$ | $R_3$ | phys. Daten |
|---|---|---|---|---|---|
| 1.048 | 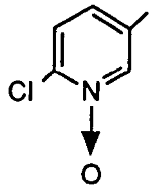 | H | H | $-C_2H_5$ | |
| 1.049 | 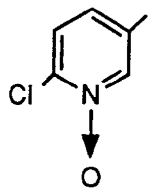 | H | H | $-C_3H_7(n)$ | |
| 1.050 | 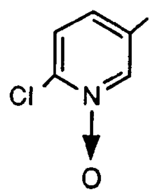 | H | H | $-CH(CH_3)_2$ | |
| 1.051 | 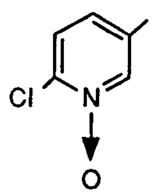 | H | H | 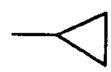 | |
| 1.052 | 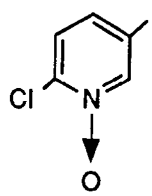 | H | H | 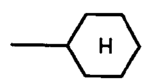 | |
| 1.053 | 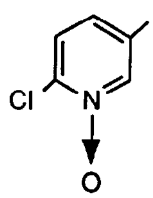 | H | H | 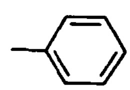 | |
| 1.054 | 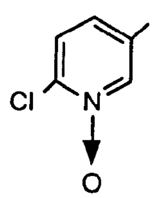 | H | H | 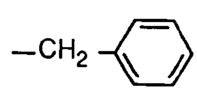 | |

| Verb. Nr. | A | $R_1$ | $R_2$ | $R_3$ | phys. Daten |
|---|---|---|---|---|---|
| 1.055 | 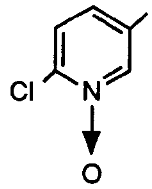 | H | $CH_3$ | $-CH_3$ | |
| 1.056 | 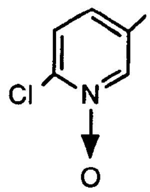 | H | $CH_3$ | $-C_2H_5$ | Smp. 152-155°C |
| 1.057 | 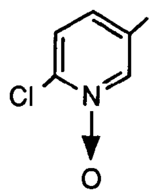 | H | $CH_3$ | $-C_3H_7(n)$ | Smp. 117-121°C |
| 1.058 | 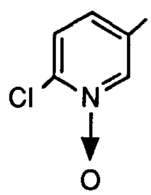 | H | $CH_3$ | $-CH(CH_3)_2$ | Smp. 138°C |
| 1.059 | 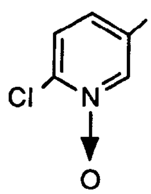 | H | $CH_3$ | 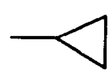 | |
| 1.060 | 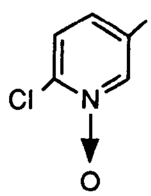 | H | $CH_3$ | 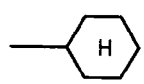 | Smp. 153°C |
| 1.061 | 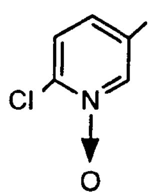 | H | $CH_3$ | 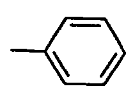 | |

| Verb. Nr. | A | R<sub>1</sub> | R<sub>2</sub> | R<sub>3</sub> | phys. Daten |
|---|---|---|---|---|---|
| 1.062 | 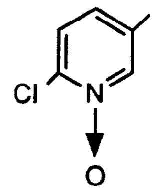 | H | $CH_3$ | 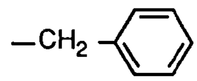 | |
| 1.063 | 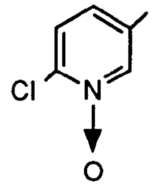 | H | 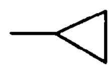 | $-CH_3$ | |
| 1.064 | 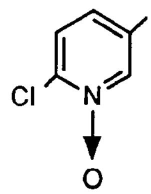 | H | 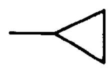 | $-C_2H_5$ | |
| 1.065 | 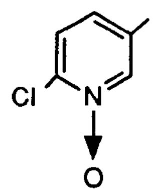 | H | 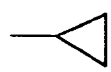 | $-C_3H_7$(n) | |
| 1.066 | 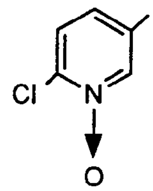 | H | 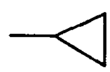 | $-CH(CH_3)_2$ | |
| 1.067 | 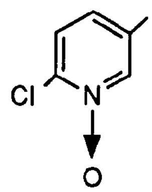 | H | 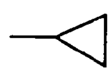 | 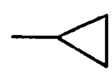 | |
| 1.068 | 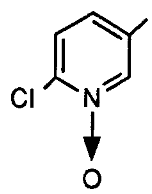 | H | 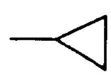 | 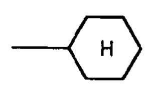 | |

| Verb. Nr. | A | $R_1$ | $R_2$ | $R_3$ | phys. Daten |
|---|---|---|---|---|---|
| 1.069 | 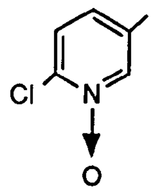 | H | 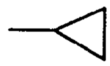 | 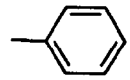 | |
| 1.070 | 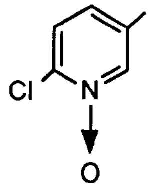 | H | 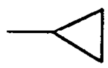 | 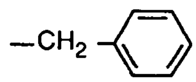 | |
| 1.071 | 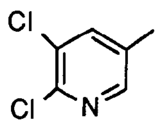 | H | H | $-CH_3$ | |
| 1.072 | 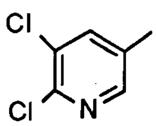 | H | H | $-C_2H_5$ | |
| 1.073 | 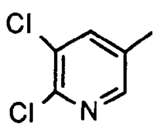 | H | H | $-C_3H_7(n)$ | |
| 1.074 | 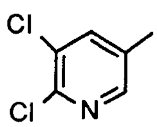 | H | H | $-CH(CH_3)_2$ | |
| 1.075 | 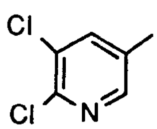 | H | H | 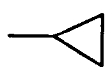 | |
| 1.076 | 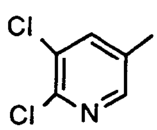 | H | H | 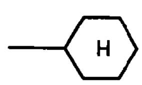 | |
| 1.077 | 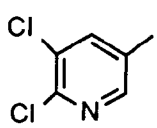 | H | H | 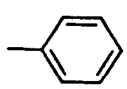 | |

| Verb. Nr. | A | $R_1$ | $R_2$ | $R_3$ | phys. Daten |
|---|---|---|---|---|---|
| 1.078 | 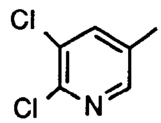 | H | H | 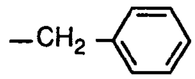 | |
| 1.079 | 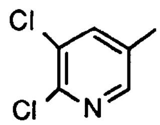 | H | $CH_3$ | $-CH_3$ | |
| 1.080 | 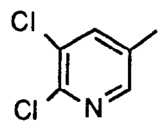 | H | $CH_3$ | $-C_2H_5$ | |
| 1.081 | 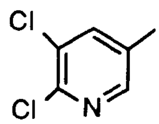 | H | $CH_3$ | $-C_3H_7(n)$ | |
| 1.082 | 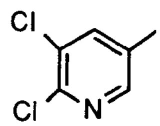 | H | $CH_3$ | $-CH(CH_3)_2$ | |
| 1.083 | 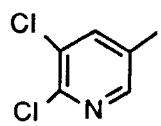 | H | $CH_3$ | 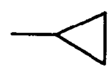 | |
| 1.084 | 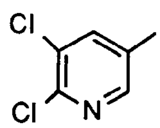 | H | $CH_3$ | 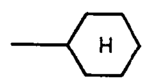 | |
| 1.085 | 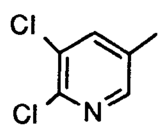 | H | $CH_3$ | 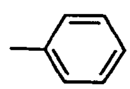 | |
| 1.086 | 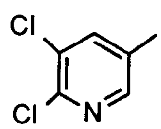 | H | $CH_3$ | 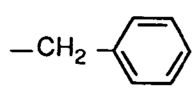 | |
| 1.087 | 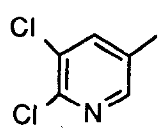 | H | 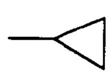 | $-CH_3$ | |

| Verb. Nr. | A | $R_1$ | $R_2$ | $R_3$ | phys. Daten |
|---|---|---|---|---|---|
| 1.088 | 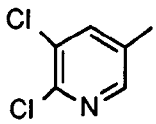 | H | 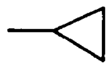 | $-C_2H_5$ | |
| 1.089 | 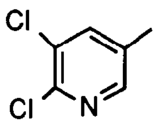 | H | 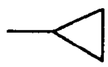 | $-C_3H_7$(n) | |
| 1.090 | 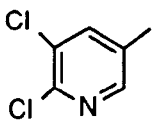 | H | 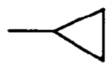 | $-CH(CH_3)_2$ | |
| 1.091 | 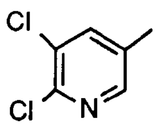 | H | 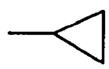 | 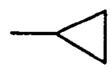 | |
| 1.092 | 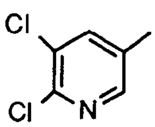 | H | 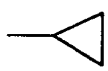 | 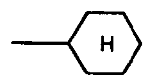 | |
| 1.093 | 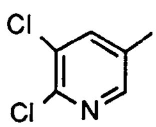 | H | 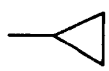 | 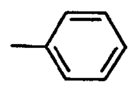 | |
| 1.094 | 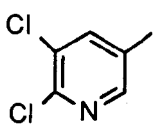 | H | 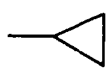 | 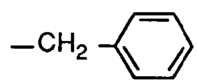 | |
| 1.095 | 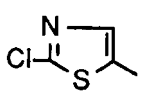 | H | H | $-C_2H_5$ | |
| 1.096 | 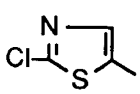 | H | H | $-C_3H_7$(n) | |
| 1.097 | 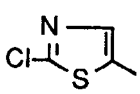 | H | H | $-CH(CH_3)_2$ | |
| 1.098 | 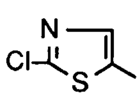 | H | H | 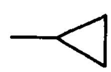 | |
| 1.099 | 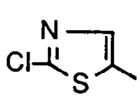 | H | H | 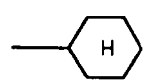 | |

| Verb. Nr. | A | $R_1$ | $R_2$ | $R_3$ | phys. Daten |
|---|---|---|---|---|---|
| 1.100 | 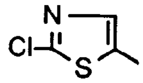 | H | H | 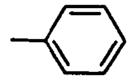 | |
| 1.101 | 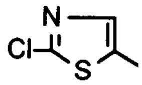 | H | H | $-CH_2-$ 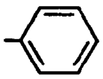 | |
| 1.102 | 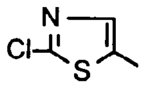 | H | $CH_3$ | $-CH_3$ | |
| 1.103 | 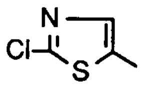 | H | $CH_3$ | $-C_2H_5$ | |
| 1.104 | 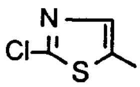 | H | $CH_3$ | $-C_3H_7(n)$ | amorph |
| 1.105 | 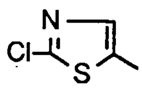 | H | $CH_3$ | $-CH(CH_3)_2$ | |
| 1.106 | 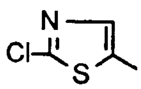 | H | $CH_3$ | 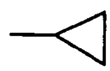 | |
| 1.107 | 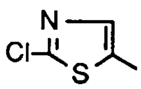 | H | $CH_3$ | 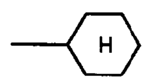 | |
| 1.108 | 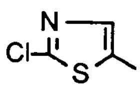 | H | $CH_3$ | 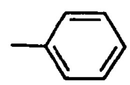 | |
| 1.109 | 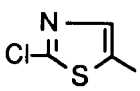 | H | $CH_3$ | $-CH_2-$ 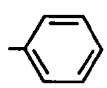 | |
| 1.110 | 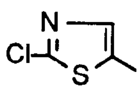 | H | 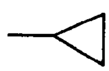 | $-CH_3$ | |
| 1.111 | 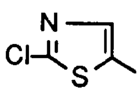 | H | 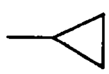 | $-C_2H_5$ | |
| 1.112 | 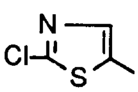 | H | 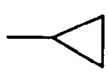 | $-C_3H_7(n)$ | |
| 1.113 | 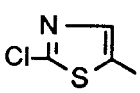 | H | 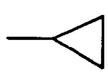 | $-CH(CH_3)_2$ | |
| 1.114 | 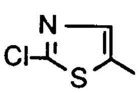 | H | 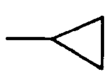 | 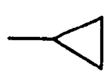 | |
| 1.115 | 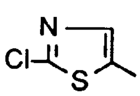 | H | 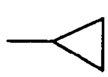 | 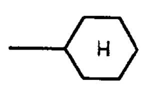 | |

| Verb. Nr. | A | $R_1$ | $R_2$ | $R_3$ | phys. Daten |
|---|---|---|---|---|---|
| 1.116 | 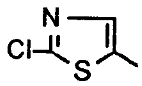 | H | 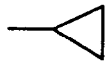 | 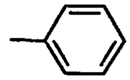 | |
| 1.117 | 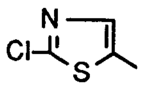 | H | 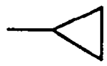 | $-CH_2$ 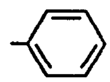 | |
| 1.118 | 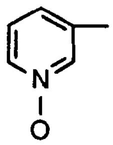 | H | H | $-CH_3$ | |
| 1.119 | 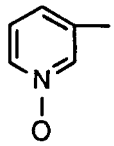 | H | H | $-C_2H_5$ | |
| 1.120 | 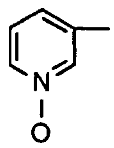 | H | H | $-C_3H_7$(n) | |
| 1.121 | 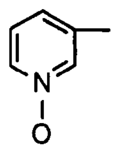 | H | H | $-CH(CH_3)_2$ | |
| 1.122 | 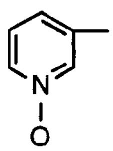 | H | H | 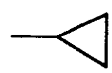 | |
| 1.123 | 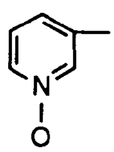 | H | H | 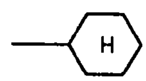 | |
| 1.124 | 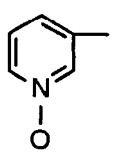 | H | H | 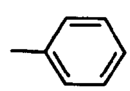 | |
| 1.125 | 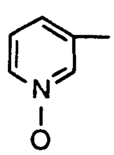 | H | H | $-CH_2$ 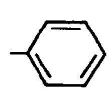 | |

| Verb. Nr. | A | $R_1$ | $R_2$ | $R_3$ | phys. Daten |
|---|---|---|---|---|---|
| 1.126 | 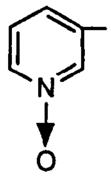 | H | H | $-CH_3$ | |
| 1.127 | 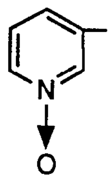 | H | $CH_3$ | $-C_2H_5$ | |
| 1.128 | 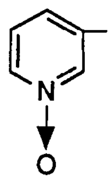 | H | $CH_3$ | $-C_3H_7(n)$ | amorphe Masse |
| 1.129 | 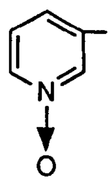 | H | $CH_3$ | $-CH(CH_3)_2$ | |
| 1.130 | 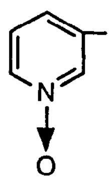 | H | $CH_3$ | 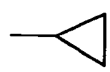 | Smp. 185°C |
| 1.131 | 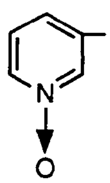 | H | $CH_3$ | 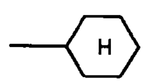 | |
| 1.132 | 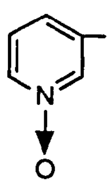 | H | $CH_3$ | 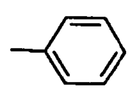 | |
| 1.133 | 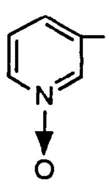 | H | $CH_3$ | 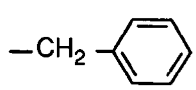 | |

| Verb. Nr. | A | $R_1$ | $R_2$ | $R_3$ | phys. Daten |
|---|---|---|---|---|---|
| 1.134 | 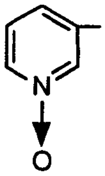 | H | 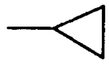 | $-CH_3$ | |
| 1.135 | 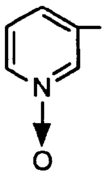 | H | 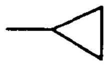 | $-C_2H_5$ | |
| 1.136 | 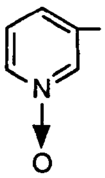 | H | 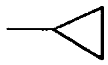 | $-C_3H_7$(n) | |
| 1.137 | 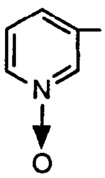 | H | 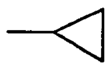 | $-CH(CH_3)_2$ | |
| 1.138 | 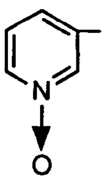 | H | 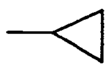 | 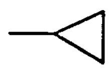 | |
| 1.139 | 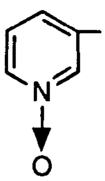 | H | 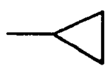 | 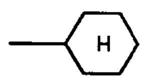 | |
| 1.140 | 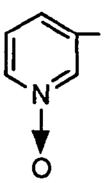 | H | 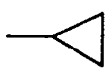 | 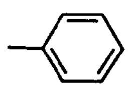 | |
| 1.141 | 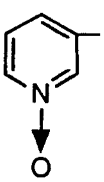 | H | 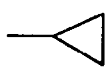 | 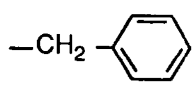 | |

| Verb. Nr. | A | $R_1$ | $R_2$ | $R_3$ | phys. Daten |
|---|---|---|---|---|---|
| 1.142 | 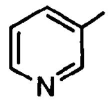 | H | H | $-CH_3$ | Smp. 142-144°C |
| 1.143 | 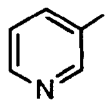 | H | $-CH_3$ | $-CH_3$ | |
| 1.144 | 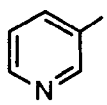 | H | 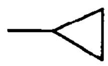 | $-CH_3$ | |
| 1.145 | 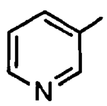 | H | H | $-C_2H_5$ | |
| 1.146 | 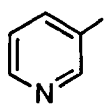 | H | $-CH_3$ | $-C_2H_5$ | |
| 1.147 | 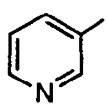 | H | 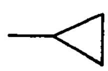 | $-C_2H_5$ | |
| 1.148 | 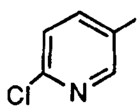 | H | 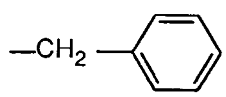 | $-C_3H_7(n)$ | Smp. 127-129°C |
| 1.149 | 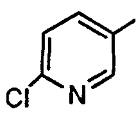 | H | 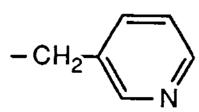 | $-C_3H_7(n)$ | |
| 1.150 | 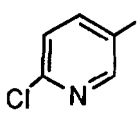 | H | 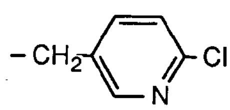 | $-C_3H_7(n)$ | |
| 1.151 | 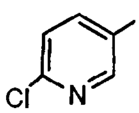 | H | 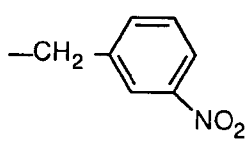 | $-C_3H_7(n)$ | |
| 1.152 | 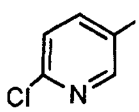 | H | 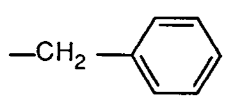 | 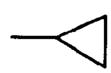 | |

| Verb. Nr. | A | $R_1$ | $R_2$ | $R_3$ | phys. Daten |
|---|---|---|---|---|---|
| 1.153 | 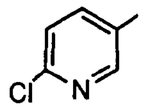 | H | 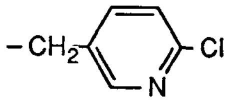 | 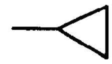 | Smp. 190-192°C |
| 1.154 | 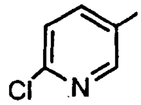 | H | 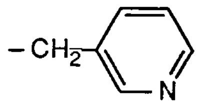 | 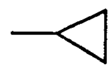 | |
| 1.155 | 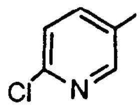 | H | H | $-CH_2COOCH_3$ | Smp. 184-186°C |
| 1.156 | 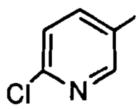 | H | $CH_3$ | $-CH_2COOCH_3$ | Smp. 185°C |
| 1.157 | 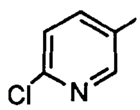 | H | 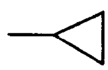 | $-CH_2COOCH_3$ | |
| 1.158 | 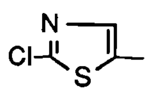 | H | H | $-CH_2COOCH_3$ | |
| 1.159 | 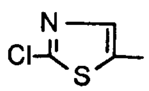 | H | $CH_3$ | $-CH_2COOCH_3$ | |
| 1.160 | 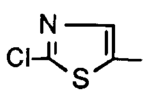 | H | 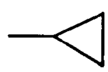 | $-CH_2COOCH_3$ | |
| 1.161 | 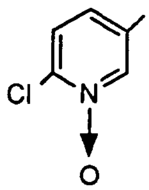 | H | H | $-CH_2COOCH_3$ | |
| 1.162 | 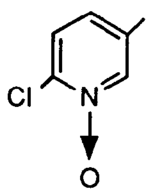 | H | $CH_3$ | $-CH_2COOCH_3$ | |

| Verb. Nr. | A | $R_1$ | $R_2$ | $R_3$ | phys. Daten |
|---|---|---|---|---|---|
| 1.163 | 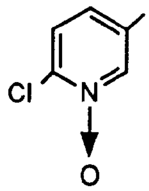 | H | 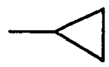 | $-CH_2COOCH_3$ | |
| 1.164 | 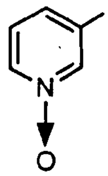 | H | H | $-CH_2COOCH_3$ | |
| 1.165 | 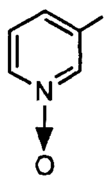 | H | $CH_3$ | $-CH_2COOCH_3$ | |
| 1.166 | 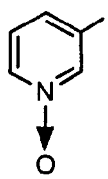 | H | 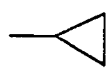 | $-CH_2COOCH_3$ | |
| 1.167 | 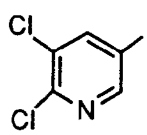 | H | H | $-CH_2COOCH_3$ | |
| 1.168 | 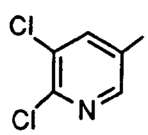 | H | $CH_3$ | $-CH_2COOCH_3$ | |
| 1.169 | 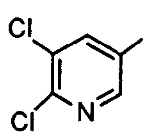 | H | 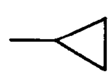 | $-CH_2COOCH_3$ | |
| 1.170 | 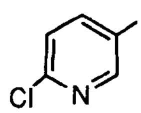 | H | $CH_3$ | $-CH_2CF_3$ | |

| Verb. Nr. | A | $R_1$ | $R_2$ | $R_3$ | phys. Daten |
|---|---|---|---|---|---|
| 1.171 | 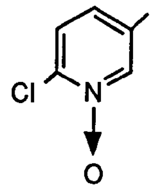 | H | $CH_3$ | $-CH_2CF_3$ | |
| 1.172 | 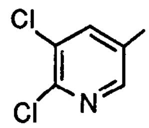 | H | $CH_3$ | $-CH_2CF_3$ | |
| 1.173 | 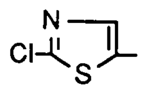 | H | $CH_3$ | $-CH_2CF_3$ | |
| 1.174 | 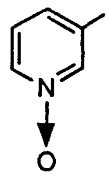 | H | $CH_3$ | $-CH_2CF_3$ | |
| 1.175 | 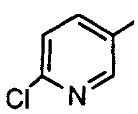 | H | $CH_3$ | $-CH_2CH_2F$ | |
| 1.176 | 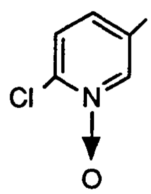 | H | $CH_3$ | $-CH_2CH_2F$ | |
| 1.177 | 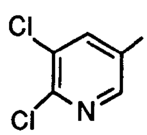 | H | $CH_3$ | $-CH_2CH_2F$ | |
| 1.178 | 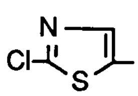 | H | $CH_3$ | $-CH_2CH_2F$ | |
| 1.179 | 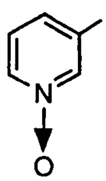 | H | $CH_3$ | $-CH_2CH_2F$ | |

| Verb. Nr. | A | $R_1$ | $R_2$ | $R_3$ | phys. Daten |
|---|---|---|---|---|---|
| 1.180 | 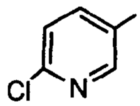 | H | $CH_3$ | $-CH_2CH_2Br$ | |
| 1.181 | 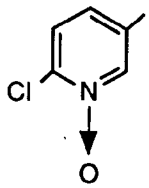 | H | $CH_3$ | $-CH_2CH_2Br$ | |
| 1.182 | 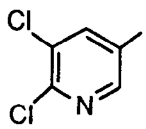 | H | $CH_3$ | $-CH_2CH_2Br$ | |
| 1.183 | 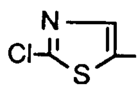 | H | $CH_3$ | $-CH_2CH_2Br$ | |
| 1.184 | 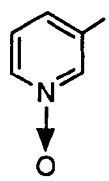 | H | $CH_3$ | $-CH_2CH_2Br$ | |
| 1.185 | 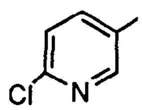 | H | $CH_3$ | $-CH_2CH_2CH_2Cl$ | |
| 1.186 | 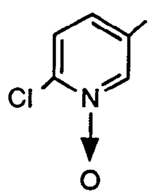 | H | $CH_3$ | $-CH_2CH_2CH_2Cl$ | |
| 1.187 | 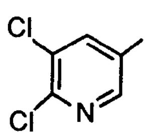 | H | $CH_3$ | $-CH_2CH_2CH_2Cl$ | |
| 1.188 | 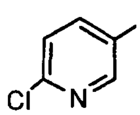 | H | $CH_3$ | $-CH_2CH_2CH_2Cl$ | |

| Verb. Nr. | A | $R_1$ | $R_2$ | $R_3$ | phys. Daten |
|---|---|---|---|---|---|
| 1.189 | 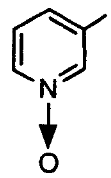 | H | $CH_3$ | $-CH_2CH_2CH_2Cl$ | |
| 1.190 | 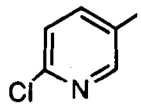 | H | $CH_3$ | $-CH_2CH_2CH_2Br$ | |
| 1.191 | 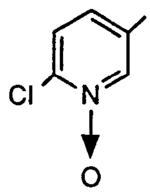 | H | $CH_3$ | $-CH_2CH_2CH_2Br$ | |
| 1.192 | 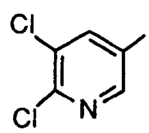 | H | $CH_3$ | $-CH_2CH_2CH_2Br$ | |
| 1.193 | 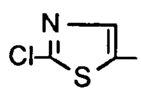 | H | $CH_3$ | $-CH_2CH_2CH_2Br$ | |
| 1.194 | 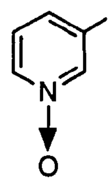 | H | $CH_3$ | $-CH_2CH_2CH_2Br$ | |
| 1.195 | 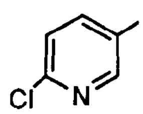 | H | $CH_3$ | $-CH_2CH_2Cl$ | |
| 1.196 | 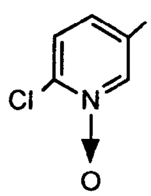 | H | $CH_3$ | $-CH_2CH_2Cl$ | |
| 1.197 | 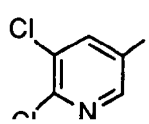 | H | $CH_3$ | $-CH_2CH_2Cl$ | |

| Verb. Nr. | A | $R_1$ | $R_2$ | $R_3$ | phys. Daten |
|---|---|---|---|---|---|
| 1.198 | 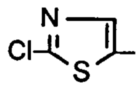 | H | $CH_3$ | $-CH_2CH_2Cl$ | |
| 1.199 | 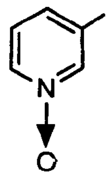 | H | $CH_3$ | $-CH_2CH_2Cl$ | |
| 1.200 | 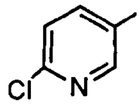 | H | $CH_3$ | $-CH_2CH(Cl)CH_2CH_2CH_2Cl$ | |
| 1.201 | 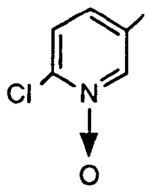 | H | $CH_3$ | $-CH_2CH(Cl)CH_2CH_2CH_2Cl$ | |
| 1.202 | 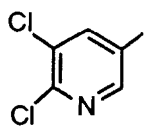 | H | $CH_3$ | $-CH_2CH(Cl)CH_2CH_2CH_2Cl$ | |
| 1.203 | 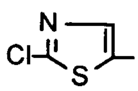 | H | $CH_3$ | $-CH_2CH(Cl)CH_2CH_2CH_2Cl$ | |
| 1.204 | 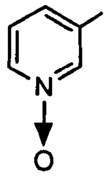 | H | $CH_3$ | $-CH_2CH(Cl)CH_2CH_2CH_2Cl$ | |
| 1.205 | 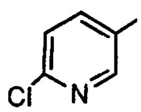 | H | $CH_3$ | $-CH_2CH_2OH$ | amorphe Masse |
| 1.206 | 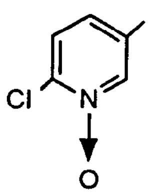 | H | $CH_3$ | $-CH_2CH_2OH$ | |

| Verb. Nr. | A | $R_1$ | $R_2$ | $R_3$ | phys. Daten |
|---|---|---|---|---|---|
| 1.207 | 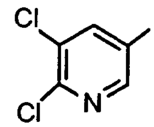 | H | $CH_3$ | $-CH_2CH_2OH$ | |
| 1.208 | 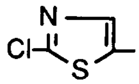 | H | $CH_3$ | $-CH_2CH_2OH$ | |
| 1.209 | 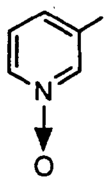 | H | $CH_3$ | $-CH_2CH_2OH$ | |
| 1.210 | 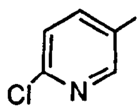 | H | $CH_3$ | $-CH_2CH_2CH_2OH$ | amorphe Masse |
| 1.211 | 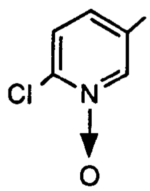 | H | $CH_3$ | $-CH_2CH_2CH_2OH$ | |
| 1.212 | 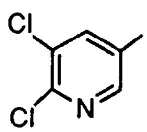 | H | $CH_3$ | $-CH_2CH_2CH_2OH$ | |
| 1.213 | 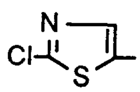 | H | $CH_3$ | $-CH_2CH_2CH_2OH$ | |
| 1.214 | 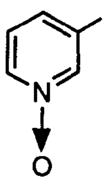 | H | $CH_3$ | $-CH_2CH_2CH_2OH$ | |
| 1.215 | 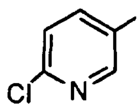 | H | $CH_3$ | $-CH_2(CH_2)_2CH_2OH$ | Smp. 108-110°C |

| Verb. Nr. | A | $R_1$ | $R_2$ | $R_3$ | phys. Daten |
|---|---|---|---|---|---|
| 1.216 | 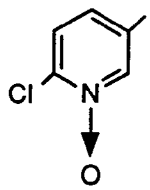 | H | $CH_3$ | $-CH_2(CH_2)_2CH_2OH$ | |
| 1.217 | 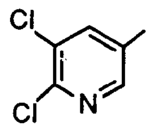 | H | $CH_3$ | $-CH_2(CH_2)_2CH_2OH$ | |
| 1.218 | 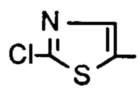 | H | $CH_3$ | $-CH_2(CH_2)_2CH_2OH$ | |
| 1.219 | 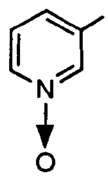 | H | $CH_3$ | $-CH_2(CH_2)_2CH_2OH$ | |
| 1.220 | 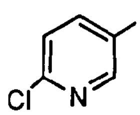 | H | $CH_3$ | $-CH_2(CH_2)_3CH_2OH$ | |
| 1.221 | 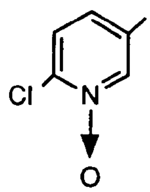 | H | $CH_3$ | $-CH_2(CH_2)_3CH_2OH$ | |
| 1.222 | 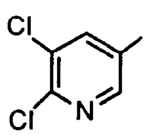 | H | $CH_3$ | $-CH_2(CH_2)_3CH_2OH$ | |
| 1.223 | 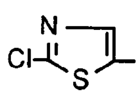 | H | $CH_3$ | $-CH_2(CH_2)_3CH_2OH$ | |
| 1.224 | 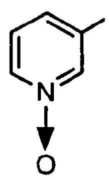 | H | $CH_3$ | $-CH_2(CH_2)_3CH_2OH$ | |

| Verb. Nr. | A | $R_1$ | $R_2$ | $R_3$ | phys. Daten |
|---|---|---|---|---|---|
| 1.225 | 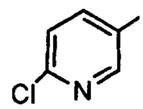 | H | $CH_3$ | $-CH(CH_3)CH_2OH$ | amorphe Masse |
| 1.226 | 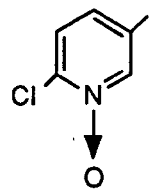 | H | $CH_3$ | $-CH(CH_3)CH_2OH$ | |
| 1.227 | 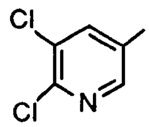 | H | $CH_3$ | $-CH(CH_3)CH_2OH$ | |
| 1.228 | 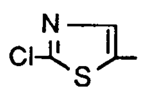 | H | $CH_3$ | $-CH(CH_3)CH_2OH$ | |
| 1.229 | 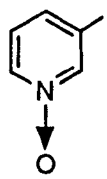 | H | $CH_3$ | $-CH(CH_3)CH_2OH$ | |
| 1.230 | 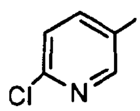 | H | $CH_3$ | $-CH(C_2H_5)CH_2OH$ | |
| 1.231 | 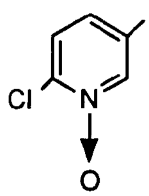 | H | $CH_3$ | $-CH(C_2H_5)CH_2OH$ | |
| 1.232 | 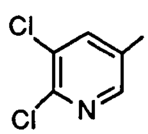 | H | $CH_3$ | $-CH(C_2H_5)CH_2OH$ | |
| 1.233 | 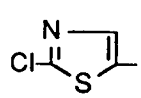 | H | $CH_3$ | $-CH(C_2H_5)CH_2OH$ | |

| Verb. Nr. | A | $R_1$ | $R_2$ | $R_3$ | phys. Daten |
|---|---|---|---|---|---|
| 1.234 | 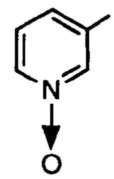 | H | $CH_3$ | $-CH(C_2H_5)CH_2OH$ | |
| 1.235 | 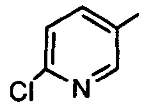 | H | $CH_3$ | $-CH_2CH(CH_3)OH$ | |
| 1.236 | 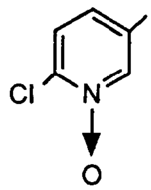 | H | $CH_3$ | $-CH_2CH(CH_3)OH$ | |
| 1.237 | 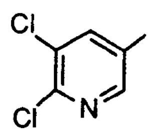 | H | $CH_3$ | $-CH_2CH(CH_3)OH$ | |
| 1.238 | 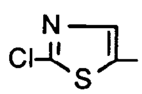 | H | $CH_3$ | $-CH_2CH(CH_3)OH$ | |
| 1.239 | 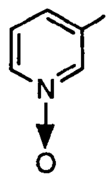 | H | $CH_3$ | $-CH_2CH(CH_3)OH$ | |
| 1.240 | 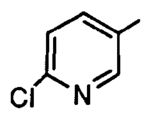 | H | $CH_3$ | $-CH_2CH(OH)CH_2OH$ | |
| 1.241 | 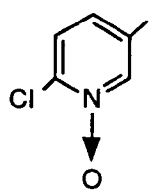 | H | $CH_3$ | $-CH_2CH(OH)CH_2OH$ | |
| 1.242 | 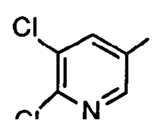 | H | $CH_3$ | $-CH_2CH(OH)CH_2OH$ | |

| Verb. Nr. | A | $R_1$ | $R_2$ | $R_3$ | phys. Daten |
|---|---|---|---|---|---|
| 1.243 | 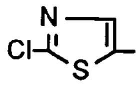 | H | $CH_3$ | $-CH_2CH(OH)CH_2OH$ | |
| 1.244 | 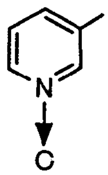 | H | $CH_3$ | $-CH_2CH(OH)CH_2OH$ | |
| 1.245 | 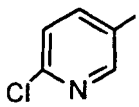 | H | $CH_3$ | $-CH(CH_2OH)_2$ | |
| 1.246 | 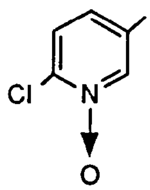 | H | $CH_3$ | $-CH(CH_2OH)_2$ | |
| 1.247 | 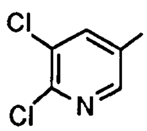 | H | $CH_3$ | $-CH(CH_2OH)_2$ | |
| 1.248 | 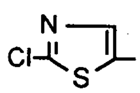 | H | $CH_3$ | $-CH(CH_2OH)_2$ | |
| 1.249 | 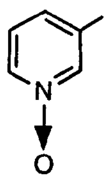 | H | $CH_3$ | $-CH(CH_2OH)_2$ | |
| 1.250 | 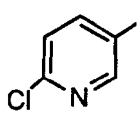 | H | $CH_3$ | $-CH_2CH_2OCH_3$ | |
| 1.251 | 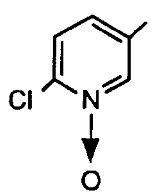 | H | $CH_3$ | $-CH_2CH_2OCH_3$ | |

| Verb. Nr. | A | $R_1$ | $R_2$ | $R_3$ | phys. Daten |
|---|---|---|---|---|---|
| 1.252 | 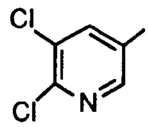 | H | $CH_3$ | $-CH_2CH_2OCH_3$ | |
| 1.253 | 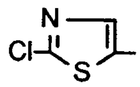 | H | $CH_3$ | $-CH_2CH_2OCH_3$ | |
| 1.254 | 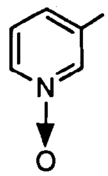 | H | $CH_3$ | $-CH_2CH_2OCH_3$ | |
| 1.255 | 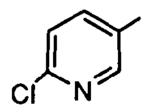 | H | $CH_3$ | $-CH_2CH_2CH_2OC_2H_5$ | |
| 1.256 | 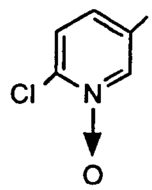 | H | $CH_3$ | $-CH_2CH_2CH_2OC_2H_5$ | |
| 1.257 | 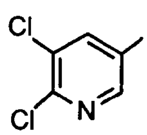 | H | $CH_3$ | $-CH_2CH_2CH_2OC_2H_5$ | |
| 1.258 | 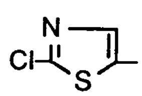 | H | $CH_3$ | $-CH_2CH_2CH_2OC_2H_5$ | |
| 1.259 | 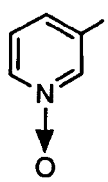 | H | $CH_3$ | $-CH_2CH_2CH_2OC_2H_5$ | |
| 1.260 | 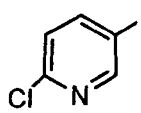 | H | $CH_3$ | $-CH(CH_3)CH_2OCH_3$ | |

| Verb. Nr. | A | $R_1$ | $R_2$ | $R_3$ | phys. Daten |
|---|---|---|---|---|---|
| 1.261 | 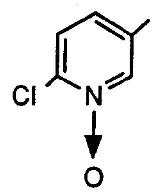 | H | $CH_3$ | $-CH(CH_3)CH_2OCH_3$ | |
| 1.262 | 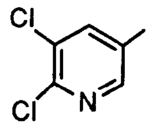 | H | $CH_3$ | $-CH(CH_3)CH_2OCH_3$ | |
| 1.263 | 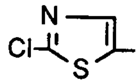 | H | $CH_3$ | $-CH(CH_3)CH_2OCH_3$ | |
| 1.264 | 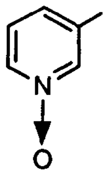 | H | $CH_3$ | $-CH(CH_3)CH_2OCH_3$ | |
| 1.265 | 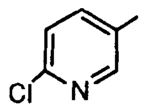 | H | $CH_3$ | $-CH_2CH(OCH_3)_2$ | |
| 1.266 | 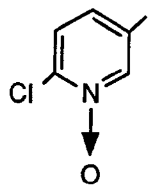 | H | $CH_3$ | $-CH_2CH(OCH_3)_2$ | |
| 1.267 | 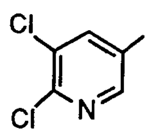 | H | $CH_3$ | $-CH_2CH(OCH_3)_2$ | |
| 1.268 | 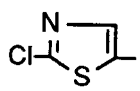 | H | $CH_3$ | $-CH_2CH(OCH_3)_2$ | |
| 1.269 | 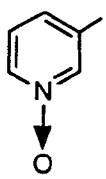 | H | $CH_3$ | $-CH_2CH(OCH_3)_2$ | |

| Verb. Nr. | A | $R_1$ | $R_2$ | $R_3$ | phys. Daten |
|---|---|---|---|---|---|
| 1.270 | 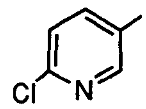 | H | $CH_3$ | $-CH_2CH(OC_2H_5)_2$ | |
| 1.271 | 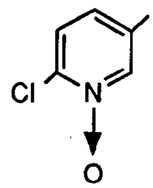 | H | $CH_3$ | $-CH_2CH(OC_2H_5)_2$ | |
| 1.272 | 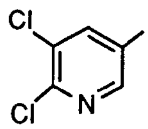 | H | $CH_3$ | $-CH_2CH(OC_2H_5)_2$ | |
| 1.273 | 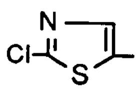 | H | $CH_3$ | $-CH_2CH(OC_2H_5)_2$ | |
| 1.274 | 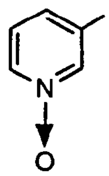 | H | $CH_3$ | $-CH_2CH(OC_2H_5)_2$ | |
| 1.275 | 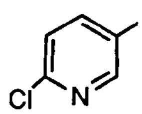 | H | $CH_3$ | $-CH_2CH_2N(CH_3)_2$ | |
| 1.276 | 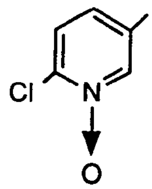 | H | $CH_3$ | $-CH_2CH_2N(CH_3)_2$ | |
| 1.277 | 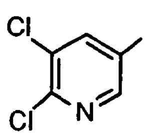 | H | $CH_3$ | $-CH_2CH_2N(CH_3)_2$ | |
| 1.278 | 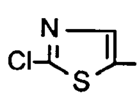 | H | $CH_3$ | $-CH_2CH_2N(CH_3)_2$ | |

| Verb. Nr. | A | $R_1$ | $R_2$ | $R_3$ | phys. Daten |
|---|---|---|---|---|---|
| 1.279 | 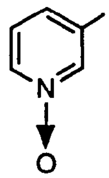 | H | $CH_3$ | $-CH_2CH_2N(CH_3)_2$ | |
| 1.280 | 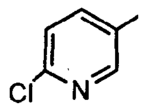 | H | $CH_3$ | $-CH_2CH_2N(C_2H_5)_2$ | |
| 1.281 | 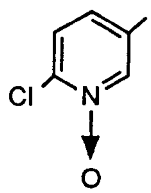 | H | $CH_3$ | $-CH_2CH_2N(C_2H_5)_2$ | |
| 1.282 | 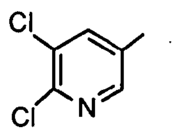 | H | $CH_3$ | $-CH_2CH_2N(C_2H_5)_2$ | |
| 1.283 | 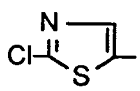 | H | $CH_3$ | $-CH_2CH_2N(C_2H_5)_2$ | |
| 1.284 | 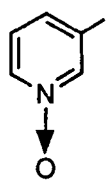 | H | $CH_3$ | $-CH_2CH_2N(C_2H_5)_2$ | |
| 1.285 | 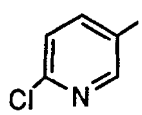 | H | $CH_3$ | $-CH_2CH_2CH_2N(CH_3)_2$ | |
| 1.286 | 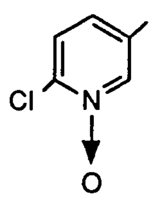 | H | $CH_3$ | $-CH_2CH_2CH_2N(CH_3)_2$ | |
| 1.287 | 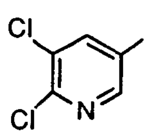 | H | $CH_3$ | $-CH_2CH_2CH_2N(CH_3)_2$ | |

| Verb. Nr. | A | $R_1$ | $R_2$ | $R_3$ | phys. Daten |
|---|---|---|---|---|---|
| 1.288 | 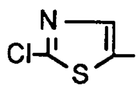 | H | $CH_3$ | $-CH_2CH_2CH_2N(CH_3)_2$ | |
| 1.289 | 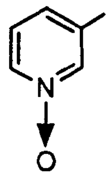 | H | $CH_3$ | $-CH_2CH_2CH_2N(CH_3)_2$ | |
| 1.290 | 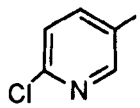 | H | $CH_3$ | $-CH_2CH_2CH_2N(C_2H_5)_2$ | |
| 1.291 | 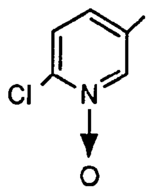 | H | $CH_3$ | $-CH_2CH_2CH_2N(C_2H_5)_2$ | |
| 1.292 | 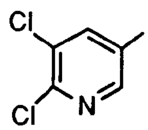 | H | $CH_3$ | $-CH_2CH_2CH_2N(C_2H_5)_2$ | |
| 1.293 | 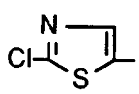 | H | $CH_3$ | $-CH_2CH_2CH_2N(C_2H_5)_2$ | |
| 1.294 | 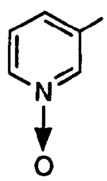 | H | $CH_3$ | $-CH_2CH_2CH_2N(C_2H_5)_2$ | |
| 1.295 | 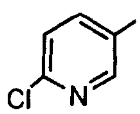 | H | $CH_3$ | $-CH_2COOC_2H_5$ | amorphe Masse |
| 1.296 | 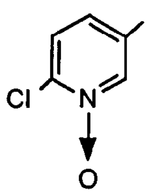 | H | $CH_3$ | $-CH_2COOC_2H_5$ | |

| Verb. Nr. | A | $R_1$ | $R_2$ | $R_3$ | phys. Daten |
|---|---|---|---|---|---|
| 1.297 | 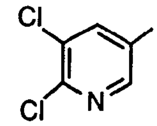 | H | $CH_3$ | $-CH_2COOC_2H_5$ | |
| 1.298 | 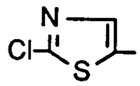 | H | $CH_3$ | $-CH_2COOC_2H_5$ | |
| 1.299 | 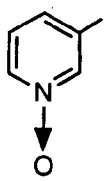 | H | $CH_3$ | $-CH_2COOC_2H_5$ | |
| 1.300 | 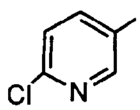 | H | $CH_3$ | $-CH_2CH_2COOC_2H_5$ | Smp. 78-80°C |
| 1.301 | 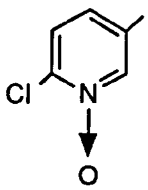 | H | $CH_3$ | $-CH_2CH_2COOC_2H_5$ | |
| 1.302 | 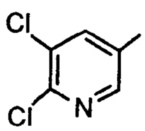 | H | $CH_3$ | $-CH_2CH_2COOC_2H_5$ | |
| 1.303 | 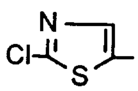 | H | $CH_3$ | $-CH_2CH_2COOC_2H_5$ | |
| 1.304 | 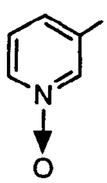 | H | $CH_3$ | $-CH_2CH_2COOC_2H_5$ | |
| 1.305 | 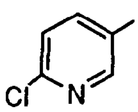 | H | $CH_3$ | $-CH(CH_3)CH_2COOC_2H_5$ | |

| Verb. Nr. | A | $R_1$ | $R_2$ | $R_3$ | phys. Daten |
|---|---|---|---|---|---|
| 1.306 | 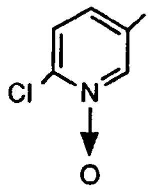 | H | $CH_3$ | $-CH(CH_3)CH_2COOC_2H_5$ | |
| 1.307 | 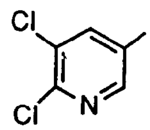 | H | $CH_3$ | $-CH(CH_3)CH_2COOC_2H_5$ | |
| 1.308 | 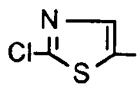 | H | $CH_3$ | $-CH(CH_3)CH_2COOC_2H_5$ | |
| 1.309 | 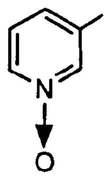 | H | $CH_3$ | $-CH(CH_3)CH_2COOC_2H_5$ | |
| 1.310 | 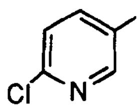 | H | $CH_3$ | $-CH(CH_2OH)COOCH_3$ | |
| 1.311 | 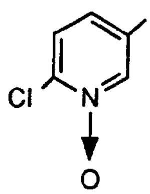 | H | $CH_3$ | $-CH(CH_2OH)COOCH_3$ | |
| 1.312 | 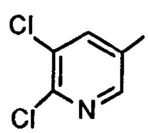 | H | $CH_3$ | $-CH(CH_2OH)COOCH_3$ | |
| 1.313 | 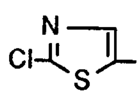 | H | $CH_3$ | $-CH(CH_2OH)COOCH_3$ | |
| 1.314 | 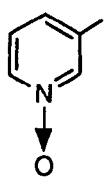 | H | $CH_3$ | $-CH(CH_2OH)COOCH_3$ | |

| Verb. Nr. | A | $R_1$ | $R_2$ | $R_3$ | phys. Daten |
|---|---|---|---|---|---|
| 1.315 | 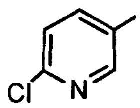 | H | $CH_3$ | 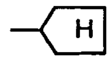 | |
| 1.316 | 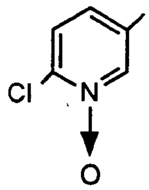 | H | $CH_3$ | 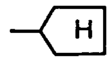 | |
| 1.317 | 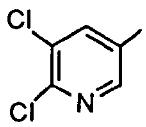 | H | $CH_3$ | 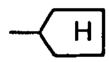 | |
| 1.318 | 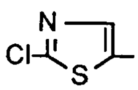 | H | $CH_3$ | 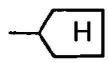 | |
| 1.319 | 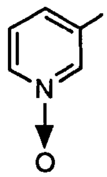 | H | $CH_3$ | 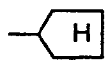 | |
| 1.320 | 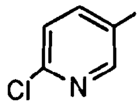 | H | $CH_3$ | 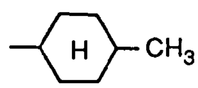 | Smp. 137-139°C<br>(cis Isomeres)<br>Smp. 170-172°C<br>(trans Isomeres) |
| 1.321 | 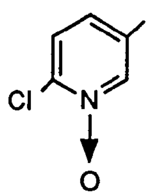 | H | $CH_3$ | 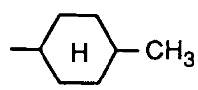 | |
| 1.322 | 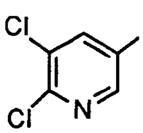 | H | $CH_3$ | 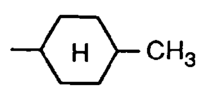 | |
| 1.323 | 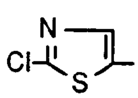 | H | $CH_3$ | 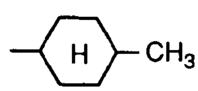 | |

| Verb. Nr. | A | $R_1$ | $R_2$ | $R_3$ | phys. Daten |
|---|---|---|---|---|---|
| 1.324 | 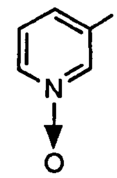 | H | $CH_3$ | 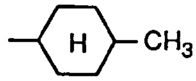 | |
| 1.325 | 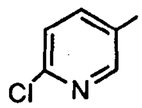 | H | $CH_3$ | 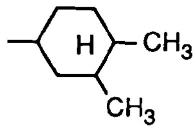 | |
| 1.326 | 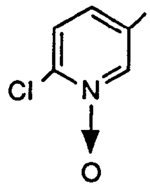 | H | $CH_3$ | 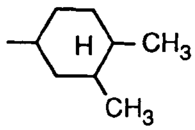 | |
| 1.327 | 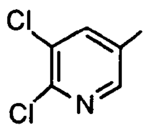 | H | $CH_3$ | 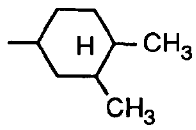 | |
| 1.328 | 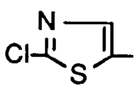 | H | $CH_3$ | 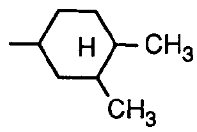 | |
| 1.329 | 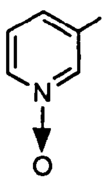 | H | $CH_3$ | 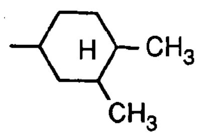 | |
| 1.330 | 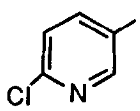 | H | $CH_3$ | 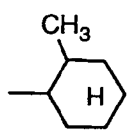 | |
| 1.331 | 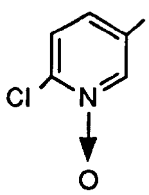 | H | $CH_3$ | 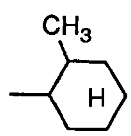 | |

| Verb. Nr. | A | $R_1$ | $R_2$ | $R_3$ | phys. Daten |
|---|---|---|---|---|---|
| 1.332 | 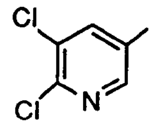 | H | $CH_3$ | 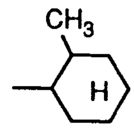 | |
| 1.333 | 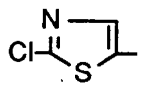 | H | $CH_3$ | 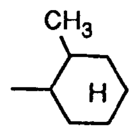 | |
| 1.334 | 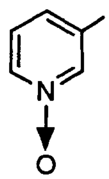 | H | $CH_3$ | 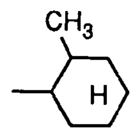 | |
| 1.335 | 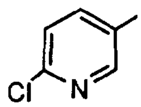 | H | $CH_3$ | $-CH_2CH=CH_2$ | Smp. 75-77°C |
| 1.336 | 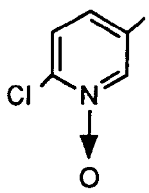 | H | $CH_3$ | $-CH_2CH=CH_2$ | |
| 1.337 | 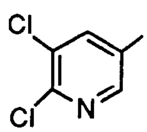 | H | $CH_3$ | $-CH_2CH=CH_2$ | |
| 1.338 | 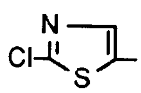 | H | $CH_3$ | $-CH_2CH=CH_2$ | |
| 1.339 | 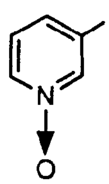 | H | $CH_3$ | $-CH_2CH=CH_2$ | |
| 1.340 | 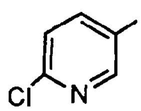 | H | $CH_3$ | 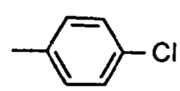 | |

| Verb. Nr. | A | $R_1$ | $R_2$ | $R_3$ | phys. Daten |
|---|---|---|---|---|---|
| 1.341 | 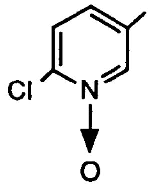 | H | $CH_3$ | 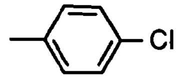 | |
| 1.342 | 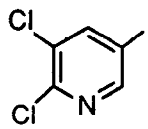 | H | $CH_3$ | 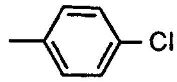 | |
| 1.343 | 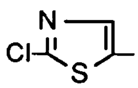 | H | $CH_3$ | 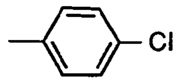 | |
| 1.344 | 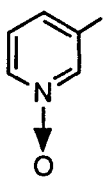 | H | $CH_3$ | 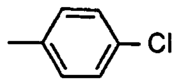 | |
| 1.345 | 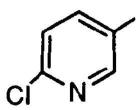 | H | $CH_3$ | 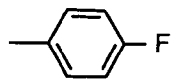 | amorphe Masse |
| 1.346 | 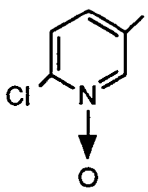 | H | $CH_3$ | 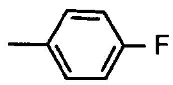 | |
| 1.347 | 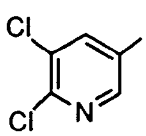 | H | $CH_3$ | 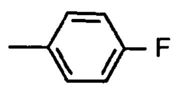 | |
| 1.348 | 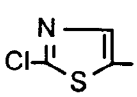 | H | $CH_3$ | 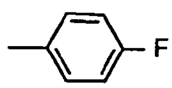 | |

| Verb. Nr. | A | $R_1$ | $R_2$ | $R_3$ | phys. Daten |
|---|---|---|---|---|---|
| 1.349 | 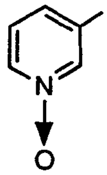 | H | $CH_3$ | 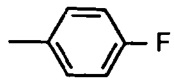 | |
| 1.350 | 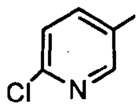 | H | $CH_3$ | 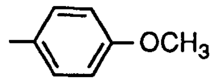 | Smp. 204°C |
| 1.351 | 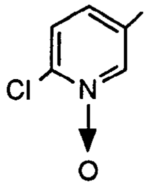 | H | $CH_3$ | 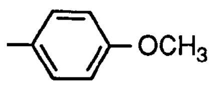 | |
| 1.352 | 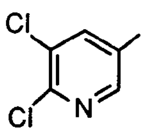 | H | $CH_3$ | 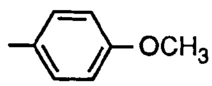 | |
| 1.353 | 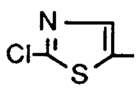 | H | $CH_3$ | 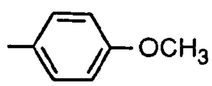 | |
| 1.354 | 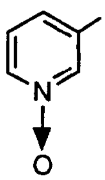 | H | $CH_3$ | 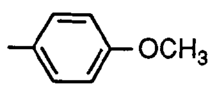 | |
| 1.355 | 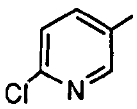 | H | $CH_3$ | 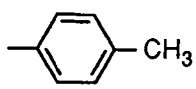 | amorphe Masse |
| 1.356 | 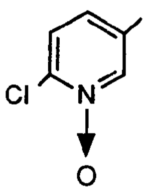 | H | $CH_3$ | 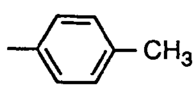 | |
| 1.357 | 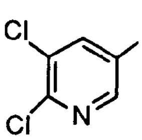 | H | $CH_3$ | 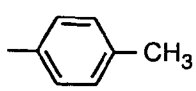 | |

| Verb. Nr. | A | $R_1$ | $R_2$ | $R_3$ | phys. Daten |
|---|---|---|---|---|---|
| 1.358 | 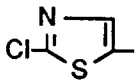 | H | $CH_3$ | 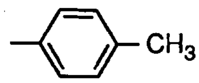 | |
| 1.359 | 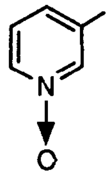 | H | $CH_3$ | 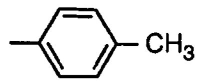 | |
| 1.360 | 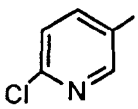 | H | $CH_3$ | 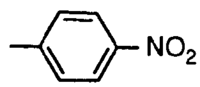 | Smp. 219°C |
| 1.361 | 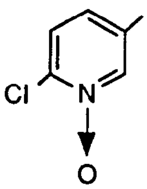 | H | $CH_3$ | 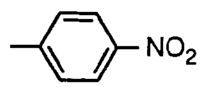 | |
| 1.362 | 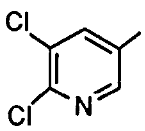 | H | $CH_3$ | 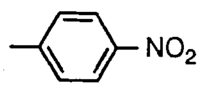 | |
| 1.363 | 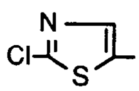 | H | $CH_3$ | 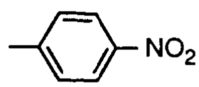 | |
| 1.364 | 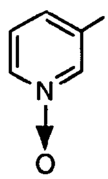 | H | $CH_3$ | 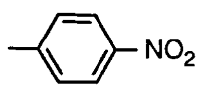 | |
| 1.365 | 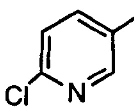 | H | $CH_3$ | 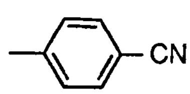 | amorphe Masse |
| 1.366 | 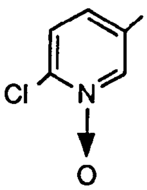 | H | $CH_3$ | 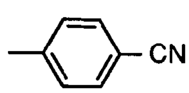 | |

| Verb. Nr. | A | $R_1$ | $R_2$ | $R_3$ | phys. Daten |
|---|---|---|---|---|---|
| 1.367 | 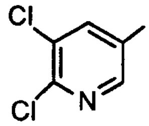 | H | $CH_3$ | 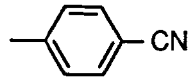 | |
| 1.368 | 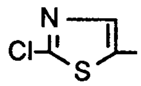 | H | $CH_3$ | 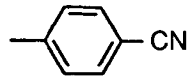 | |
| 1.369 | 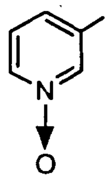 | H | $CH_3$ | 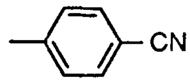 | |
| 1.370 | 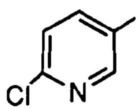 | H | $CH_3$ | 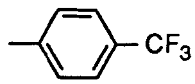 | amorphe Masse |
| 1.371 | 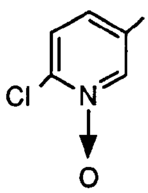 | H | $CH_3$ | 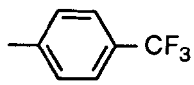 | |
| 1.372 | 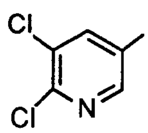 | H | $CH_3$ | 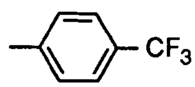 | |
| 1.373 | 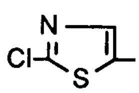 | H | $CH_3$ | 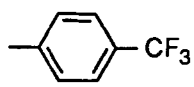 | |
| 1.374 | 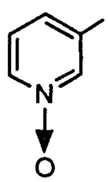 | H | $CH_3$ | 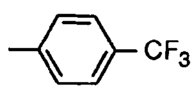 | |
| 1.375 | 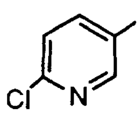 | H | $CH_3$ | 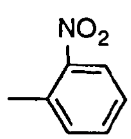 | |

| Verb. Nr. | A | $R_1$ | $R_2$ | $R_3$ | phys. Daten |
|---|---|---|---|---|---|
| 1.376 | 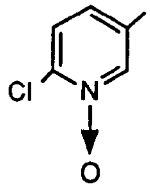 | H | $CH_3$ | 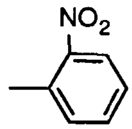 | |
| 1.377 | 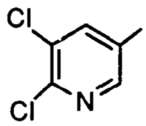 | H | $CH_3$ | 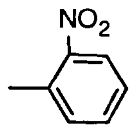 | |
| 1.378 | 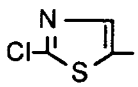 | H | $CH_3$ | 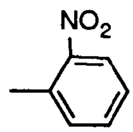 | |
| 1.379 | 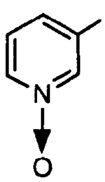 | H | $CH_3$ | 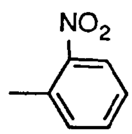 | |
| 1.380 | 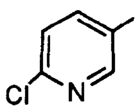 | H | $CH_3$ | 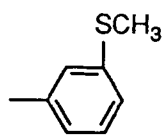 | |
| 1.381 | 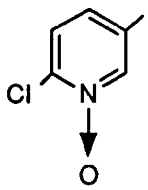 | H | $CH_3$ | 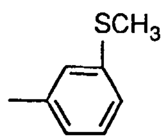 | |
| 1.382 | 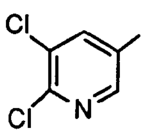 | H | $CH_3$ | 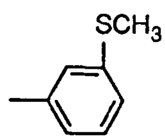 | |
| 1.383 | 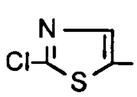 | H | $CH_3$ | 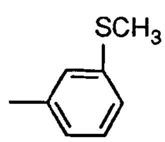 | |

| Verb. Nr. | A | $R_1$ | $R_2$ | $R_3$ | phys. Daten |
|---|---|---|---|---|---|
| 1.384 | 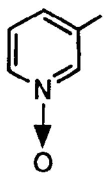 | H | $CH_3$ | 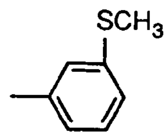 | |
| 1.385 | 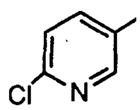 | H | $CH_3$ | 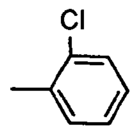 | |
| 1.386 | 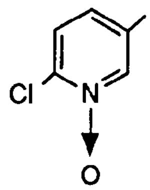 | H | $CH_3$ | 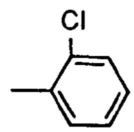 | |
| 1.387 | 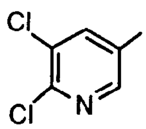 | H | $CH_3$ | 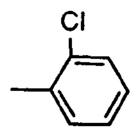 | |
| 1.388 | 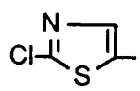 | H | $CH_3$ | 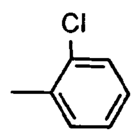 | |
| 1.389 | 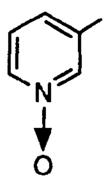 | H | $CH_3$ | 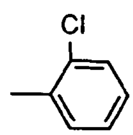 | |
| 1.390 | 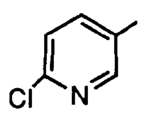 | H | $CH_3$ | 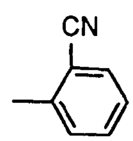 | |
| 1.391 | 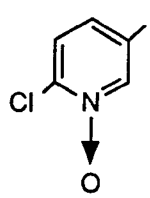 | H | $CH_3$ | 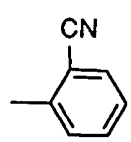 | |

| Verb. Nr. | A | $R_1$ | $R_2$ | $R_3$ | phys. Daten |
|---|---|---|---|---|---|
| 1.392 | 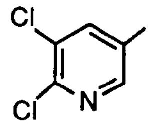 | H | $CH_3$ | 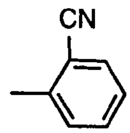 | |
| 1.393 | 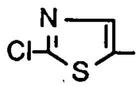 | H | $CH_3$ | 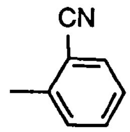 | |
| 1.394 | 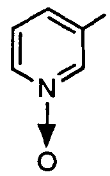 | H | $CH_3$ | 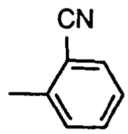 | |
| 1.395 | 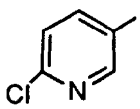 | H | $CH_3$ | 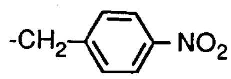 | amorphe Masse |
| 1.396 | 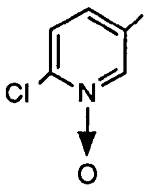 | H | $CH_3$ | 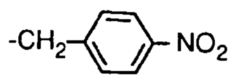 | |
| 1.397 | 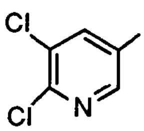 | H | $CH_3$ | 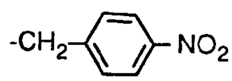 | |
| 1.398 | 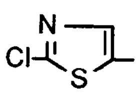 | H | $CH_3$ | 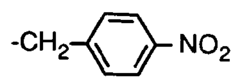 | |
| 1.399 | 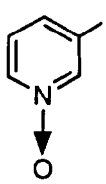 | H | $CH_3$ | 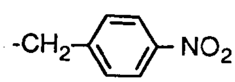 | |
| 1.400 | 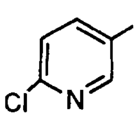 | H | $CH_3$ | 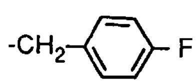 | Smp. 162-164°C |

| Verb. Nr. | A | $R_1$ | $R_2$ | $R_3$ | phys. Daten |
|---|---|---|---|---|---|
| 1.401 | 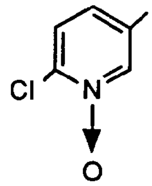 | H | $CH_3$ | 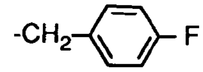 | |
| 1.402 | 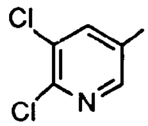 | H | $CH_3$ | 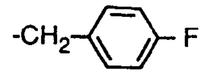 | |
| 1.403 | 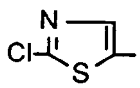 | H | $CH_3$ | 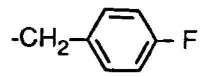 | |
| 1.404 | 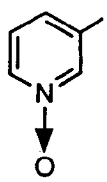 | H | $CH_3$ | 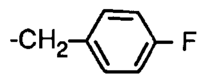 | |
| 1.405 | 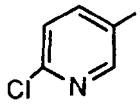 | H | $CH_3$ | 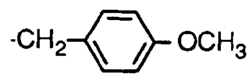 | Smp. 125-127°C |
| 1.406 | 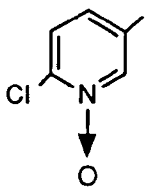 | H | $CH_3$ | 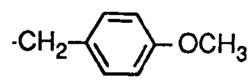 | |
| 1.407 | 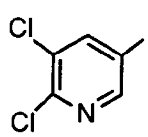 | H | $CH_3$ | 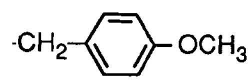 | |
| 1.408 | 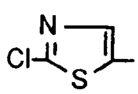 | H | $CH_3$ | 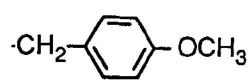 | |

| Verb. Nr. | A | $R_1$ | $R_2$ | $R_3$ | phys. Daten |
|---|---|---|---|---|---|
| 1.409 | 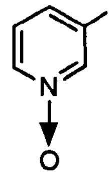 | H | $CH_3$ | 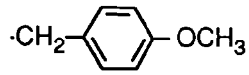 | |
| 1.410 | 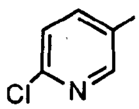 | H | $CH_3$ | 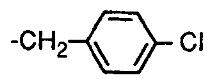 | Smp. 147-149°C |
| 1.411 | 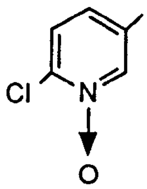 | H | $CH_3$ | 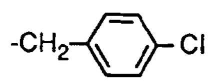 | |
| 1.412 | 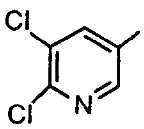 | H | $CH_3$ | 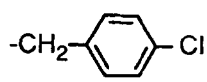 | |
| 1.413 | 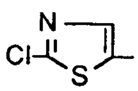 | H | $CH_3$ | 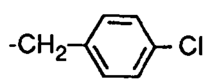 | |
| 1.414 | 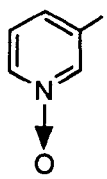 | H | $CH_3$ | 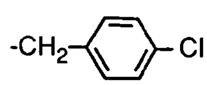 | |
| 1.415 | 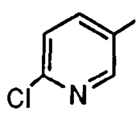 | H | $CH_3$ | 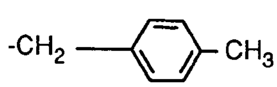 | Smp. 155-157°C |
| 1.416 | 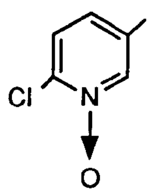 | H | $CH_3$ | 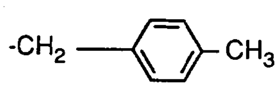 | |

| Verb. Nr. | A | $R_1$ | $R_2$ | $R_3$ | phys. Daten |
|---|---|---|---|---|---|
| 1.417 | 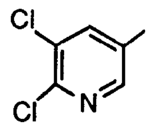 | H | $CH_3$ | 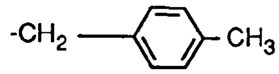 | |
| 1.418 | 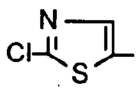 | H | $CH_3$ | 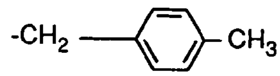 | |
| 1.419 | 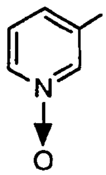 | H | $CH_3$ | 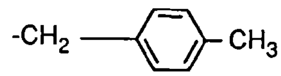 | |
| 1.420 | 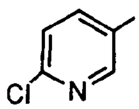 | H | $CH_3$ | 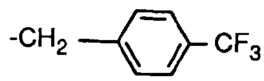 | Smp. 167-169°C |
| 1.421 | 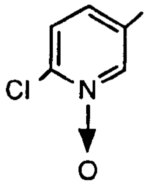 | H | $CH_3$ | 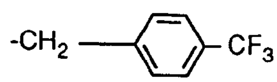 | |
| 1.422 | 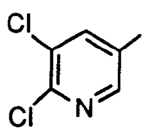 | H | $CH_3$ | 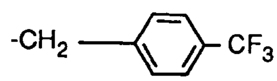 | |
| 1.423 | 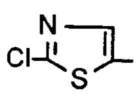 | H | $CH_3$ | 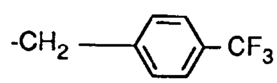 | |
| 1.424 | 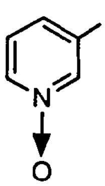 | H | $CH_3$ | 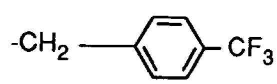 | |
| 1.425 | 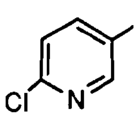 | H | $CH_3$ | 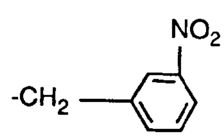 | |

| Verb. Nr. | A | $R_1$ | $R_2$ | $R_3$ | phys. Daten |
|---|---|---|---|---|---|
| 1.426 | 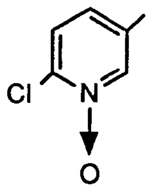 | H | $CH_3$ | 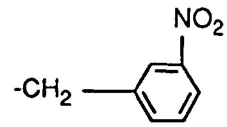 | |
| 1.427 | 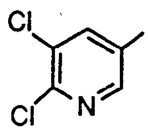 | H | $CH_3$ | 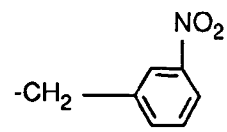 | |
| 1.428 | 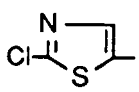 | H | $CH_3$ | 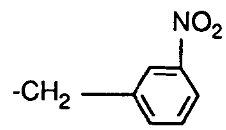 | |
| 1.429 | 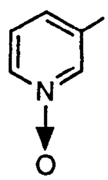 | H | $CH_3$ | 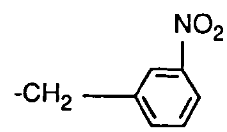 | |
| 1.430 | 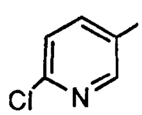 | H | $CH_3$ | 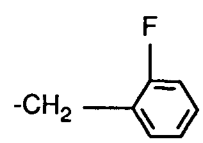 | |
| 1.431 | 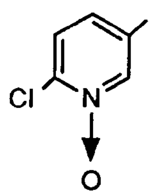 | H | $CH_3$ | 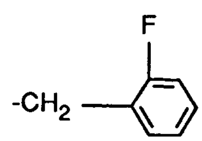 | |
| 1.432 | 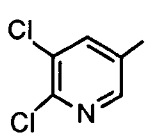 | H | $CH_3$ | 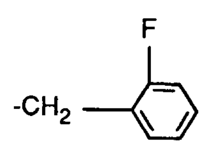 | |

| Verb. Nr. | A | $R_1$ | $R_2$ | $R_3$ | phys. Daten |
|---|---|---|---|---|---|
| 1.433 | 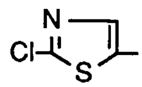 | H | $CH_3$ | -$CH_2$ 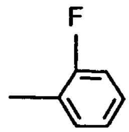 | |
| 1.434 | 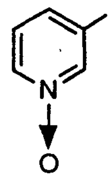 | H | $CH_3$ | -$CH_2$ 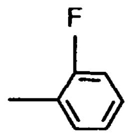 | |
| 1.435 | 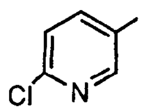 | H | $CH_3$ | -$CH_2$ 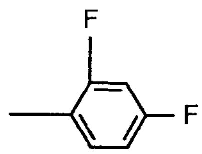 | |
| 1.436 | 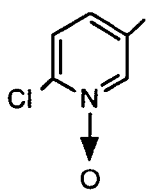 | H | $CH_3$ | -$CH_2$ 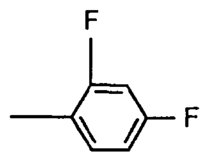 | |
| 1.437 | 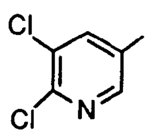 | H | $CH_3$ | -$CH_2$ 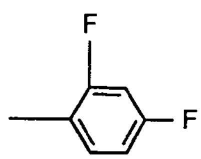 | |
| 1.438 | 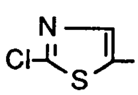 | H | $CH_3$ | -$CH_2$ 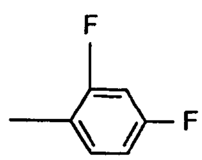 | |
| 1.439 | 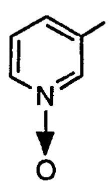 | H | $CH_3$ | -$CH_2$ 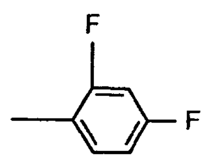 | |

| Verb. Nr. | A | $R_1$ | $R_2$ | $R_3$ | phys. Daten |
|---|---|---|---|---|---|
| 1.440 | 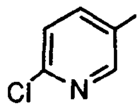 | H | $CH_3$ | $-CH_2$ 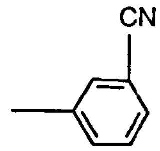 | |
| 1.441 | 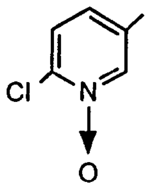 | H | $CH_3$ | $-CH_2$ 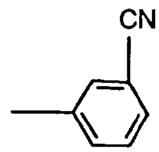 | |
| 1.442 | 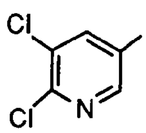 | H | $CH_3$ | $-CH_2$ 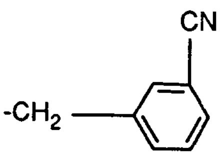 | |
| 1.443 | 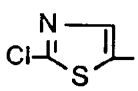 | H | $CH_3$ | $-CH_2$ 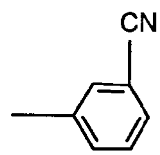 | |
| 1.444 | 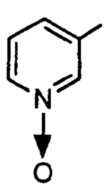 | H | $CH_3$ | $-CH_2$ 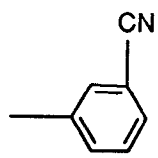 | |
| 1.445 | 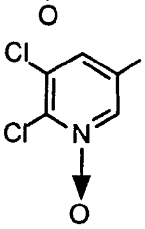 | H | $CH_3$ | $-C_3H_7(n)$ | Smp. 157-158°C |

Biespiel 3:

Formulierungen (%=Gewitchtsprozent)

| Beispiel F1: Emulsions-Konzentrate | a) | b) | c) |
|---|---|---|---|
| Wirkstoff gemäss Beispiel 2 | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzenration hergestellt werden.

| Beispiel F2: Lösungen | a) | b) | c) | d) |
|---|---|---|---|---|
| Wirkstoff gemäss Beispiel 2 | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| Beispiel F3: Granulate | a) | b) | c) | d) |
|---|---|---|---|---|
| Wirkstoff gemäss Beispiel 2 | 5 % | 10 % | 8 % | 21 % |
| Kaolin | 94 % | - | 79 % | 54 % |
| Hochdisperse Kieselsäure | 1 % | - | 13 % | 7 % |
| Attapulgit | - | 90 % | - | 18 % |

Der Wirkstoff wird in Methylenchlorid gelöst, auf den Träger aufgesprüht und das Lösungsmittel anschliessend im Vakuum abgedampft.

| Beispiel F4: Stäubemittel | a) | b) |
| --- | --- | --- |
| Wirkstoff gemäss Beispiel 2 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| Beispiel F5: Spritzpulver | a) | b) | c) |
| --- | --- | --- | --- |
| Wirkstoff gemäss Beispiel 2 | 25 % | 50 % | 75 % |
| Na-Ligninsulfonat | 5 % | 5 % | - |
| Na-Laurylsulfat | 3 % | - | 5 % |
| Na-Diisobutylnaphthalin-sulfonat | - | 6 % | 10 % |
| Octylphenolpolyäthylenglykol-äther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen. Man erhält Spritzpulver, die sich mit Wasser zu Suspensionen jeder gewünschten Konzentration verdünnen lassen.

Beispiel F6: Emulsions-Konzentrat

| | |
| --- | --- |
| Wirkstoff gemäss Beispiel 2 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

Beispiel F7: Stäubemittel

| | a) | b) |
|---|---|---|
| Wirkstoff gemäss Beispiel 2 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

Beispiel F8: Extruder-Granulat

| | |
|---|---|
| Wirkstoff gemäss Beispiel 2 | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

Beispiel F9: Umhüllungs-Granulat

| | |
|---|---|
| Wirkstoff gemäss Beispiel 2 | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

Beispiel F10: Suspensions-Konzentrat

| | |
|---|---|
| Wirkstoff gemäss Beispiel 2 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| Silikonöl in Form einer 75 %igen wässrigen Emulsion | 1 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 4: Wirkung gegen Nilaparvata lugens

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der

Population (% Wirkung) bestimmt.

Die Verbindungen von Beispiel 2 zeigen eine gute Wirkung gegen Nilaparvata lugens in diesem Test. Insbesondere die Verbindungen 1.007, 1.008, 1.009, 1.011, 1.012, 1.013, 1.014, 1.056, 1.057, 1.058, 1.104, 1.128, 1.130, 1.156, 1.205, 1.215, 1.300, 1.320, 1.335 und 1.410 zeigen eine Wirkung von 80-90%.

Beispiel 5: Wirkung gegen Nephotettix cincticeps

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

Die Verbindungen gemäss Beispiel 2 zeigen eine gute Wirkung gegen Nephotettix cincticeps in diesem Test. Insbesondere die Verbindungen 1.007, 1.008, 1.009, 1.011, 1.012, 1.013, 1.014, 1.056, 1.057, 1.058, 1.060, 1.104, 1.128, 1.156, 1.205, 1.215, 1.300, 1.320, 1.335 und 1.410 zeigen eine Wirkung über 80 %.

Beispiel 6: Wirkung gegen Myzus persicae

Erbsenkeimlinge werden Myzus persicae infiziert und anschliessend mit einer Spritzbrühe, die 400 ppm des Wirkstoffes enthält, besprüht und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt.

Die Verbindungen gemäss Beispiel 2 zeigen eine gute Wirkung gegen Myzus persicae in diesem Test. Insbesondere die Verbindungen 1.001, 1.002, 1.006, 1.007, 1.008, 1.009, 1.011, 1.012 1. 104 und 1. 156 zeigen eine Wirkung über 80 %.

Beispiel 7: Wirkung gegen Aphis craccivora

Erbsenkeimlinge werden mit Aphis craccivora infiziert und anschliessend mit einer Spritzbrühe, die 400 ppm des Wirkstoffes enthält, besprüht und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt.

Die Verbindungen gemäss Beispiel 2 zeigen eine gute Wirkung gegen Aphis craccivora in diesem Test. Insbesondere die Verbindungen 1.001, 1.002, 1.006, 1.007, 1.008, 1.009, 1.011, 1.012, 1.104, 1.155, 1.156, 1.205 und 1.300 zeigen eine Wirkung über 80 %.

Beispiel 8: Systemische Wirkung gegen Nilaparvata lugens

Töpfe mit Reispflanzen werden in eine wässrige Emulsions-Lösung, die 400 ppm des Wirkstoffes enthält, gestellt. Anschliessend werden die Reispflanzen mit Larven des 2. und 3. Stadiums besiedelt. 6 Tage später erfolgt die Auswertung. Aus dem Vergleich der Anzahl Zikaden auf den behandelten zu denjenigen auf den unbehandelten Pflanzen wird die prozentuale Reduktion der Population (% Wirkung) bestimmt.

Die Verbindungen gemäss Beispiel 2 zeigen eine gute Wirkung gegen Nilaparvata lugens in diesem Test. Insbesondere die Verbindungen 1.001, 1.002, 1.003, 1.004, 1.006, 1.007, 1.008, 1.009, 1.011, 1.012, 1.013, 1.014, 1.057, 1.058, 1.060, 1.104, 1.128, 1.130, 1.142, 1. 156, 1.205, 1.215, 1.300, 1.320, 1.335, 1.410 und 1.445 zeigen eine Wirkung über 80 %.

Beispiel 9: Systemische Wirkung gegen Nephotettix cincticeps

Töpfe mit Reispflanzen werden in eine wässrige Emulsions-Lösung, die 400 ppm des Wirkstoffes enthält, gestellt. Anschliessend werden die Reispflanzen mit Larven des 2. und 3. Stadiums besiedelt. 6 Tage später erfolgt die Auswertung. Aus dem Vergleich der Anzahl Zikaden auf den behandelten zu denjenigen auf den unbehandelten Pflanzen wird die prozentuale Redkution der Population (% Wirkung) bestimmt.

Die Verbindungen gemäss Beispiel 2 zeigen eine gute Wirkung gegen Nephotettix cincticeps in diesem Test. Insbesondere die Verbindungen 1.001, 1.002, 1.003, 1.004, 1.005, 1.006, 1.008, 1.009, 1.011, 1.012, 1.013, 1.014, 1.057, 1.058, 1.060, 1.104, 1.156 und 1.335 zeigen eine Wirkung über 80 %.

Beispiel 10: Systemische Wirkung gegen Myzus persicae

Erbsenkeimlinge werden mit Myzus persicae infiziert, anschliessend mit den Wurzeln in eine Spritzbrühe, die 400 ppm des Wirkstoffes enthält, gestellt und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt.

Die Verbindungen gemäss Beispiel 2 zeigen eine gute Wirkung gegen Myzus persicae in diesem Test. Insbesondere die Verbindungen 1.001, 1.002, 1.007, 1.008, 1.009, 1.011, 1.012, 1.025, 1.104 und 1.156 zeigen eine Wirkung über 80 %.

Beispiel 11: Wirkung gegen Bemisia tabaci

Buschbohnen-Pflanzen werden in Gazekäfige gestellt und mit Adulten Bemisia tabaci (Weisse Fliege) besiedelt. Nach erfolgter Eiablage werden alle Adulten entfernt und 10 Tage später die Pflanzen mit den darauf befindenden Nymphen mit einer wässrigen Emulsions-Spritzbrühe der zu prüfenden Wirkstoffe (Konzentration 400 ppm) behandelt. Die Auswertung erfolgt 14 Tage nach der Wirkstoff-Applikation auf %-Schlupf im Vergleich zu den unbehandelten Kontrollansätzen.

Verbindungen gemäss Bespiel 2 zeigen in diesem Test gute Wirkung gegen Bemisia tabaci. Insbesondere die Verbindungen 1.001, 1.002, 1.006, 1.007, 1.008, 1.009, 1.011, 1.012, 1.013, 1.014, 1.016, 1.104, 1.156 und 1.335 zeigen eine Wirkung über 80 %.

Beispiel 12: Wirkung gegen Ctenocephalus felis

Es werden 20 bis 25 Eier des Katzenflohs (Ctenocephalus felis) in je eine waagrecht stehende 50 ml Zellkulturenflasche gegeben, in der zuvor 15 g eines Flohlarven-Nährmediums, das 100 ppm des zu prüfenden Wirkstoffes enthält, vorgelegt wurden. Die Flaschen werden verschlossen und in einem Brutschrank bei 26-27°C und 60-70 % Luftfeuchtigkeit deponiert. Nach einer Bebrütungszeit von 21 Tagen wird auf die Entwicklung von adulten Flöhen, nicht-geschlüpften Puppen und Larven bonitiert.

Die Verbindungen gemäss Beispiel 2 zeigen eine gute Wirkung in diesem Test. Insbesondere die Verbindungen 1.009 und 1.025 zeigen eine Wirkung von über 80 %.

Beispiel 13: Wirkung gegen Blattella germanica

In eine Petri-Schale von 10 cm Durchmesser wird so viel einer 0,1%igen acetonischen Lösung des Wirkstoffes gegeben, dass die Menge einer Aufwandmenge von 1 $g/m^2$ entspricht. Wenn das Lösungsmittel verdunstet ist, werden 10 Blattella germanica Nymphen (letztes Nymphenstadium) in die so vorbereitete Schale gegeben und 2 Stunden lang der Wirkung der Testsubstanz ausgesetzt. Dann werden die Nymphen mit $CO_2$ narkotisiert, in eine frische Petri-Schale gebracht und im Dunkeln bei 25°C und ca. 70 % Luftfeuchtigkeit gehalten. Nach 48 Stunden wird die insektizide Wirkung durch Ermittlung der Abtötungsrate bestimmt.

Verbindungen gemäss Beispiel 2 zeigen eine gute Wirkung im obigen Test. Insbesondere Die Verbindung 1. 104 zeigt eine über 60 %-ige Wirkung.

Beispiel 14: Wirkung gegen Boophilus microplus

Vollgesogene adulte Zecken Weibchen werden auf eine PVC Platte aufgeklebt und mit einem Wattebausch überdeckt. Zur Behandlung werden die Testtiere mit 10 ml einer wässrigen Testlösung, die 125 ppm des zu prüfenden Wirkstoffes enthält übergossen. Anschliessend wird der Wattebausch entfernt und die Zecken werden für 4 Wochen zur Eiablage inkubiert. Die Wirkung gegen Boophilus microplus zeigt sich entweder beim Weibchen als Mortalität oder Sterilität, oder bei den Eiern als ovizide Wirkung. Verbindungen gemäss Beispiel 2 zeigen eine gute Wirkung gegen Boophilus microplus. Insbesondere die Verbindungen 1.008, 1.009 und 1.025 zeigen in diesem Test eine Wirkung von über 60 %.

## Patentansprüche

1. Verbindung der Formel I

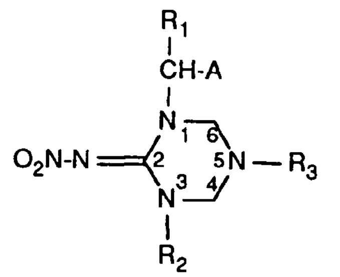

(I),

worin

$R_1$ Wasserstoff oder $C_1$-$C_4$-Alkyl,

$R_2$ Wasserstoff, $C_1$-$C_6$-Alkyl, $C_3$-$C_6$-Cycloalkyl oder einen Rest -$CH_2B$;

$R_3$ Wasserstoff, $C_1$-$C_{10}$-Alkyl, $C_3$-$C_6$-Cycloalkyl, mit 1-12 Resten aus der Gruppe Halogen, Hydroxy, $C_1$-$C_4$-Alkoxy, $C_1$-$C_4$-Haloalkoxy mit 1 bis 9 Halogenatomen, Di-($C_1$-$C_4$-Alkyl)-amino und $C_1$-$C_5$-Alkoxycarbonyl substituiertes $C_1$-$C_{10}$-Alkyl, mit 1-4 $C_1$-$C_4$-Alkylresten oder Halogenatomen substituiertes $C_3$-$C_6$-Cycloalkyl, $C_2$-$C_8$-Alkenyl oder -Alkinyl, mit 1-6 Halogenatomen substituiertes $C_2$-$C_8$-Alkenyl oder -Alkinyl, Phenyl, Benzyl, oder mit 1-3 Ringsubstituenten aus der Gruppe Halogen, $C_1$-$C_4$-Alkyl, $C_1$-$C_4$-Haloalkyl mit 1 bis 9 Halogenatomen, $C_1$-$C_4$-Alkoxy, $C_1$-$C_4$-Haloalkoxy mit 1 bis 9 Halogenatomen, $C_1$-$C_4$-Alkylthio, Nitro und Cyano substituiertes Phenyl oder Benzyl;

A einen unsubstituierten oder ein- bis vierfach substituierten aromatischen oder nichtaromatischen, monocyclischen oder bicyclischen heterocyclischen Rest, der ein bis zwei Substituenten aus der Gruppe $C_1$-$C_3$-Halogenalkyl mit 1 bis 7 Halogenatomen, Cyclopropyl, Halogencyclopropyl mit 1 bis 3 Halogenatomen, $C_2$-$C_3$-Alkenyl, $C_2$-$C_3$-Alkinyl, $C_2$-$C_3$-Halogenalkenyl und $C_2$-$C_3$-Halogenalkinyl mit 1 bis 4 Halogenatomen, $C_1$-$C_3$-Halogenalkoxy mit 1 bis 7 Halogenatomen, $C_1$-$C_3$-Alkylthio, $C_1$-$C_3$-Halogenalkylthio mit 1 bis 7 Halogenatomen, Allyloxy, Propargyloxy, Allylthio, Propargylthio, Halogenallyloxy, Halogenallylthio, Cyan und Nitro und ein bis vier Substituenten aus der Gruppe $C_1$-$C_3$-Alkyl, $C_1$-$C_3$-Alkoxy und Halogen enthalten kann; und

B Phenyl, Cyanophenyl, Nitrophenyl, Halogenphenyl mit 1 bis 3 Halogenatomen, durch $C_1$-$C_3$-Alkyl, $C_1$-$C_3$-Halogenalkyl mit 1 bis 7 Halogenatomen, $C_1$-$C_3$-Alkoxy oder $C_1$-$C_3$-Halogenalkoxy mit 1 bis 7 Halogenatomen substituiertes Phenyl, 3-Pyridyl, 5-Thiazolyl, durch ein bis zwei Substituenten aus der Gruppe $C_1$-$C_3$-Alkyl, $C_1$-$C_3$-Halogenalkyl mit 1 bis 7 Halogenatomen, Cyclopropyl, Halogencyclopropyl, $C_2$-$C_3$-Alkenyl, $C_2$-$C_3$-Alkinyl, $C_1$-$C_3$-Alkoxy, $C_2$-$C_3$-Halogenalkenyl und $C_2$-$C_3$-Halogenalkinyl mit 1 bis 4 Halogenatomen, $C_1$-$C_3$-Halogenalkoxy mit 1 bis 7 Halogenatomen, $C_1$-$C_3$-Alkylthio, $C_1$-$C_3$-Halogenalkylthio mit 1 bis 7 Halogenatomen, Allyloxy, Propargyloxy, Allylthio, Propargylthio, Halogenallyloxy, Halogenallylthio, Halogen, Cyan und Nitro substituiertes 5-Thiazolyl; oder durch ein bis zwei Reste aus der Gruppe $C_1$-$C_3$-Halogenalkyl mit 1 bis 7 Halogenatomen, Cyclopropyl, Halogencyclopropyl, $C_2$-$C_3$-Alkenyl, $C_2$-$C_3$-Alkinyl, $C_2$-$C_3$-Halogenalkenyl und $C_2$-$C_3$-Halogenalkinyl mit 1 bis 4 Halogenatomen, $C_1$-$C_3$-Halogenalkoxy mit 1 bis 7 Halogenatomen, $C_1$-$C_3$-Alkylthio, $C_1$-$C_3$-Halogenalkylthio mit 1 bis 7 Halogenatomen, Allyloxy, Propargyloxy, Allylthio, Propargylthio, Halogenallyloxy, Halogenallylthio,, Cyan und Nitro oder durch ein bis vier Reste aus der Gruppe $C_1$-$C_3$-Alkyl, $C_1$-$C_3$-Alkoxy und Halogen substituiertes 3-Pyridyl; bedeuten, sowie deren Salze mit anorganischen Säuren.

**2.** Verbindungen der Formel I gemäss Anspruch 1, worin

$R_3$ $C_5$-$C_{10}$-Alkyl, $C_3$-$C_6$-Cycloalkyl, mit 1-12 Resten aus der Gruppe Halogen, Hydroxy, $C_1$-$C_4$-Alkoxy, $C_1$-$C_4$-Haloalkoxy mit 1 bis 9 Halogenatomen, Di-($C_1$-$C_4$-Alkyl)-amino und $C_1$-$C_5$-Alkoxycarbonyl substituiertes $C_1$-$C_{10}$-Alkyl, mit 1-4 $C_1$-$C_4$-Alkyl-resten oder Halogenatomen substituiertes $C_3$-$C_6$-Cycloalkyl, $C_2$-$C_8$-Alkenyl oder -Alkinyl, mit 1-6 Halogenatomen substituiertes $C_2$-$C_8$-Alkenyl oder -Alkinyl, Phenyl, Benzyl, oder mit 1-3 Ringsubstituenten aus der Gruppe Halogen, $C_1$-$C_4$-Alkyl, $C_1$-$C_4$-Haloalkyl mit 1 bis 9 Halogenatomen, $C_1$-$C_4$-Alkoxy, $C_1$-$C_4$-Haloalkoxy mit 1 bis 9 Halogenatomen, $C_1$-$C_4$-Alkylthio, Nitro und Cyano substituiertes Phenyl oder Benzyl bedeutet und $R_1$, $R_2$ und A die in Anspruch 1 angegebenen Bedeutungen haben, sowie deren Salze mit anorganischen Säuren.

**3.** Verbindung gemäss Anspruch 1 oder 2, dadurch gekennzeichnet, dass der heterocyclische Rest A ungesättigt ist, über ein Kohlenstoffatom an den Rest des Moleküls der Verbindung der Formel 1 gebunden ist und mindestens ein Stickstoffatom enthält.

4. Verbindung gemäss Anspruch 3, dadurch gekennzeichnet, dass der heterocyclische Rest A ungesättigt ist und über ein Kohlenstoffatom an den Rest des Moleküls der Verbindung der Formel I gebunden ist und ein bis drei Heteroatome aus der Reihe Sauerstoff, Schwefel und Stickstoff enthält, wobei jeweils höchstens ein Sauerstoff- oder Schwefelatom enthalten ist.

5. Verbindungen gemäss Anspruch 4, dadurch gekennzeichnet, dass der heterocyclische Rest A ein bis drei Heteroatome aus der Reihe Sauerstoff, Schwefel und Stickstoff enthält, wovon stets ein Heteroatom Stickstoff ist, und höchstens ein Sauerstoffatom oder Schwefelatom enthalten ist.

6. Verbindungen gemäss Anspruch 1 oder 2, dadurch gekennzeichnet, dass der heterocyclische Rest A einen über ein Kohlenstoffatom an den Rest des Moleküls der Verbindung der Formel 1 gebundenen heterocyclischen Grundkörper aus der Gruppe

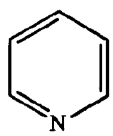 , 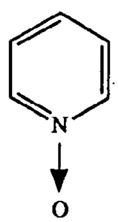 , 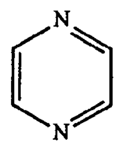 , 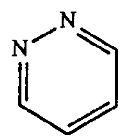 , 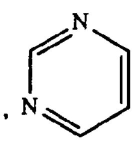 , 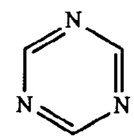 , 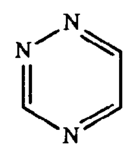 ,

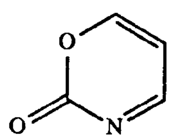 , 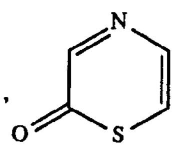 , 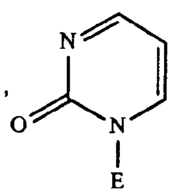 , 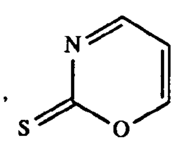 , 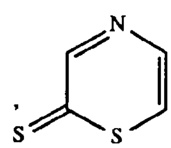 , 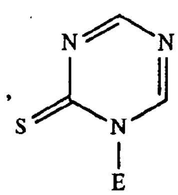 ,

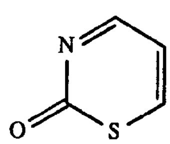 , 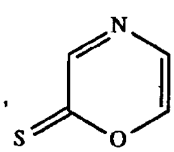 , 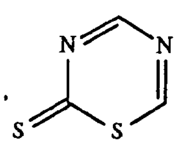 , 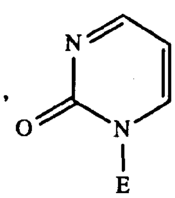 , 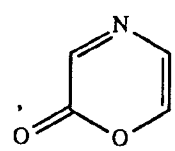 ,

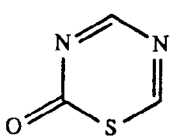 , 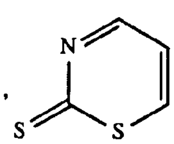 , 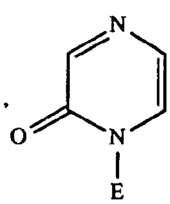 , 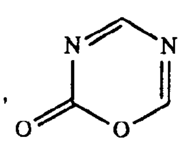 , 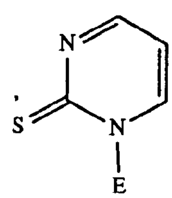 , 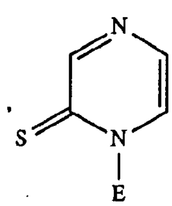 ,

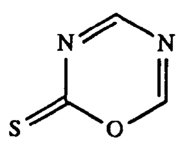 , 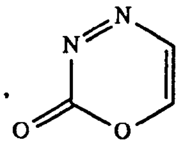 , 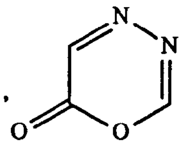 , 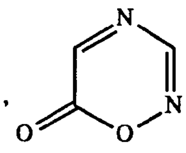 , 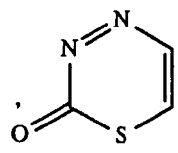 ,

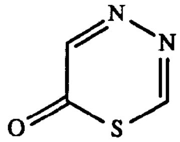 , 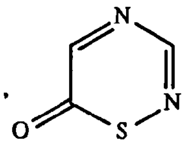 , 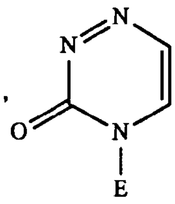 , 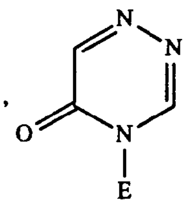 , 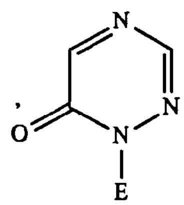 ,

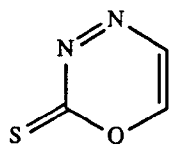 , 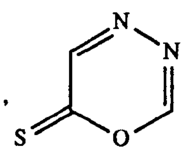 , 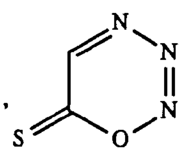 , 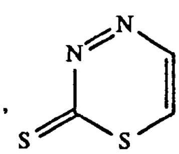 , 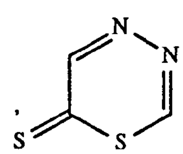 ,

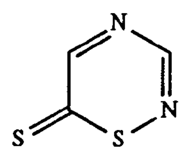 , 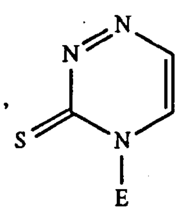 , 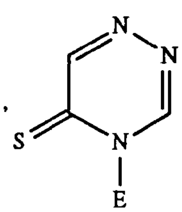 , 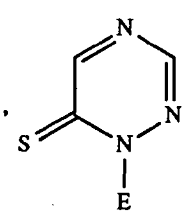 , 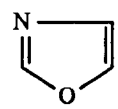 ,

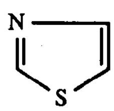 , 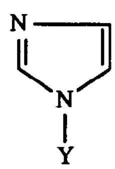 , 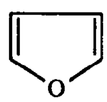 , 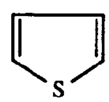 ,

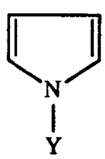 , 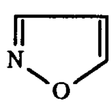 , 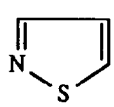 , 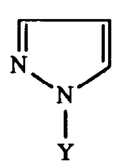 ,

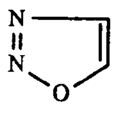 , 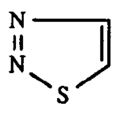 , 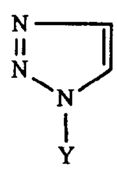 , 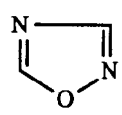 , 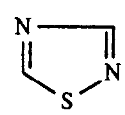 .

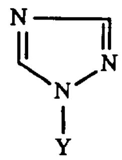 , 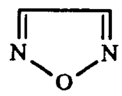 , 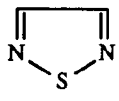 , 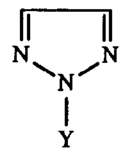 , 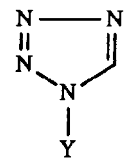 ,

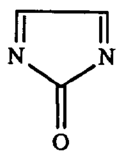 , 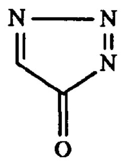 , 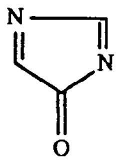 , 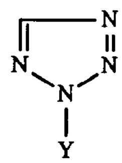 , 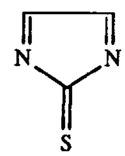 ,

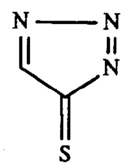 , 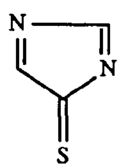 , 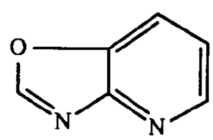 , 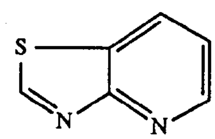 , 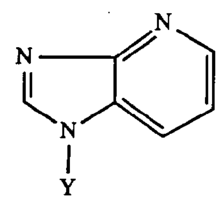 ,

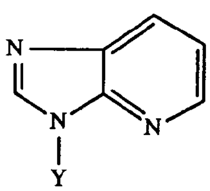 , 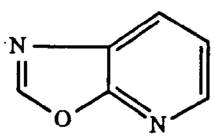 , 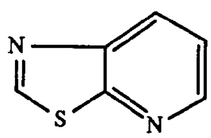 , 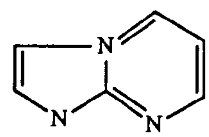 ,

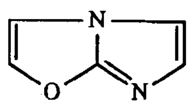 , 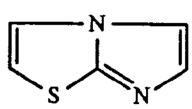 , 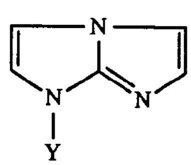 ,

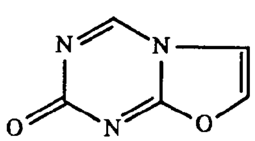 , 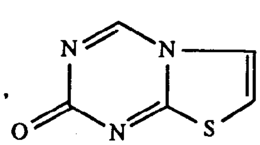 , 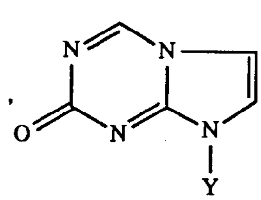 , 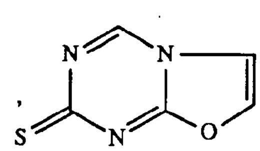 ,

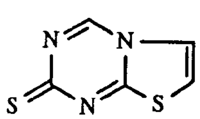 und 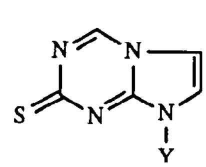

darstellt, welcher unsubstituiert ist oder je nach den Substitutionsmöglichkeiten des Ringsystems bis zu vier der im Anspruch 1 definierten Substituenten tragen kann und worin E für $C_1$-$C_3$-Alkyl und Y für Wasserstoff, $C_1$-$C_3$-Alkyl oder Cyclopropyl stehen.

7. Verbindungen gemäss Anspruch 6, dadurch gekennzeichnet, dass der heterocyclische Rest A unsubstituiert ist oder ein bis drei Substituenten aus der Gruppe Halogen, $C_1$-$C_3$-Alkyl, $C_1$-$C_3$-Halogenalkyl sowie $C_1$-$C_3$-Halogenalkoxy mit 1 bis 7 Halogenatomen und $C_1$-$C_3$-Alkoxy trägt.

8. Verbindungen gemäss Anspruch 7, dadurch gekennzeichnet, dass der Rest A Pyridyl oder Thiazolyl

bedeutet.

**9.** Verbindungen gemäss Anspruch 1 oder 2, dadurch gekennzeichnet, dass der Rest B für einen Phenyl-, Pyridyl- oder Thiazolylrest steht, welcher unsubstituiert oder durch ein bis zwei Reste aus der Gruppe Halogen, $C_1$-$C_3$-Alkyl, $C_1$-$C_3$-Halogenalkyl und $C_1$-$C_3$-Halogenalkoxy mit 1 bis 7 Halogenatomen und $C_1$-$C_3$-Alkoxy substituiert ist.

**10.** Verbindungen gemäss Anspruch 7, dadurch gekennzeichnet, dass der Rest A für 3-Pyridyl, 2-Halogenpyrid-5-yl, 2,3-Dihalogenpyrid-5-yl oder 2-Halogenthiazol-4-yl, 1-Oxopyrid-3-yl, 1-Oxo-2-halogenpyrid-5-yl oder 1-Oxo-2,3-dihalogenpyrid-5-yl steht.

**11.** Verbindungen gemäss Anspruch 1 oder 2, dadurch gekennzeichnet, dass $R_1$ Wasserstoff, $R_2$ Methyl, Äthyl oder Cyclopropyl; und A Pyridyl, 1-Oxopyridyl, Thiazolyl oder jeweils durch ein bis drei Substituenten aus der Gruppe Halogen, $C_1$-$C_3$-Alkyl, $C_1$-$C_3$-Halogenalkyl und $C_1$-$C_3$-Halogenalkoxy mit 1 bis 7 Halogenatomen und $C_1$-$C_3$-Alkoxy substituiertes Piridyl, 1-Oxopyridyl oder Thiazolyl bedeuten.

**12.** Verbindung gemäss Anspruch 1 oder 2, dadurch gekennzeichnet, dass $R_1$ Wasserstoff bedeutet.

**13.** Verbindung gemäss Anspruch 12, dadurch gekennzeichnet, dass $R_1$ Wasserstoff und $R_2$ Methyl bedeuten.

**14.** Verbindung gemäss einem der Ansprüche 1 und 3 bis 13, dadurch gekennzeichnet, dass $R_3$ $C_1$-$C_3$-Alkyl, Cyclopropyl, Cyclohexyl, Phenyl, Benzyl oder den Rest $-CH_2-COO-CH_3$ bedeutet.

**15.** Verbindung gemäss Anspruch 13, dadurch gekennzeichnet, dass $R_3$ mit 1 bis 3 Ringsubstituenten aus der Gruppe Fluor, Chlor, Brom, $C_1$-$C_2$-Alkyl, $C_1$-$C_2$-Halogenalkyl, $C_1$-$C_2$-Alkoxy, $C_1$-$C_2$-Alkylthio, Nitro und Cyano substituiertes Benzyl bedeutet.

**16.** Verbindung gemäss Anspruch 13, dadurch gekennzeichnet, dass $R_3$ mit 1 bis 3 Ringsubstituenten aus der Gruppe Fluor, Chlor, Brom, $C_1$-$C_2$-Alkyl, $C_1$-$C_2$-Halogenalkyl, $C_1$-$C_2$-Alkoxy, $C_1$-$C_2$-Alkylthio, Nitro und Cyano substituiertes Phenyl bedeutet.

**17.** Verbindung gemäss Anspruch 13, dadurch gekennzeichnet, dass $R_3$ mit einer Hydroxy-Gruppe substituiertes $C_1$-$C_6$-Alkyl bedeutet.

**18.** Verbindung gemäss Anspruch 13, dadurch gekennzeichnet, dass $R_3$ mit einer $C_1$-$C_5$-Alkoxycarbonyl-Gruppe substituiertes $C_1$-$C_6$-Alkyl bedeutet.

**19.** Verbindung gemäss Anspruch 13, dadurch gekennzeichnet, dass $R_3$ $-CH_2CH_2F$, $-CH_2CH_2Br$, $-CH_2CH_2CH_2Cl$, $-CH_2CH_2CH_2Br$ oder $-CH_2CHClCH_2CH_2CH_2Cl$ bedeutet.

**20.** Verbindung gemäss Anspruch 13, dadurch gekennzeichnet, dass $R_3$ $-CH_2CH_2O-CH_3$, $-CH_2CH_2CH_2-O-CH_2CH_3$, $-CH(CH_3)CH_2-O-CH_3$, $-CH_2CH(OCH_3)_2$, $-CH_2CH_2-N(CH_2CH_3)_2$, $-CH_2-CH_2CH_2-N(CH_3)_2$ oder $-CH_2CH_2CH_2-N(CH_2CH_3)_2$ bedeutet.

**21.** Verbindung gemäss Anspruch 13, dadurch gekennzeichnet, dass $R_3$ unsubstituiertes oder mit ein oder zwei $C_1$-$C_4$-Alkylresten substituiertes $C_4$-$C_6$-Cycloalkyl bedeutet.

**22.** Verbindung gemäss Anspruch 21, dadurch gekennzeichnet, dass $R_3$ Cyclopentyl oder Cyclohexyl bedeutet.

**23.** Verbindung gemäss Anspruch 21, dadurch gekennzeichnet, dass $R_3$ mit einer oder zwei Methylgruppen substituiertes $C_3$-$C_6$-Cycloalkyl bedeutet.

**24.** Verbindung gemäss Anspruch 1 oder 11, dadurch gekennzeichnet, dass A 2-Chlorthiazol-4-yl, 2,3-Dichlorpyrid-5-yl, 1-Oxopyrid-3-yl oder 1-Oxo-2-chlorpyrid-5-yl; $R_2$ Methyl und $R_3$ Cyclopropyl, $-CH_2CH_2Cl$, $-CH_2CH(OCH_3)_2$ oder $-CH_2CH_2N(CH_3)_2$ bedeuten.

**25.** Verbindung gemäss einem der Ansprüche 1 bis 24, dadurch gekennzeichnet, dass A 2-Chlorthiazol-4-yl

bedeutet.

**26.** Verbindung gemäss einem der Ansprüche 1 bis 24, dadurch gekennzeichnet, dass A 2-Chlorpyrid-5-yl bedeutet.

**27.** Verbindungen gemäss einem der Ansprüche 1 bis 7, dadurch gekennzeichnet, dass A 1-Oxo-2-chlorpyrid-5-yl oder 1-Oxopyrid-5-yl bedeutet.

**28.** Verbindung gemäss Anspruch 1, dadurch gekennzeichnet, dass A 2-Chlopyrid-5-yl, 2,3-Dichlorpyrid-5-yl, 2-Chlorthiazol-4-yl, 1-Oxopyrid-3-yl oder 1-Oxo-2-chlorpyrid-5-yl; $R_1$ Wasserstoff; $R_2$ Methyl; und $R_3$ n-Propyl bedeuten.

**29.** Verbindung gemäss Anspruch 26 der Formel

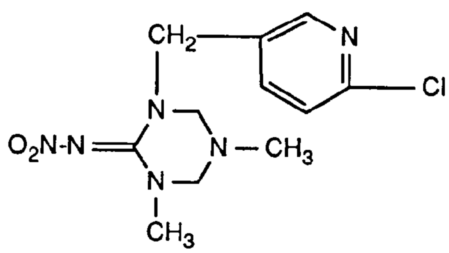

.

**30.** Verbindung gemäss Anspruch 26 der Formel

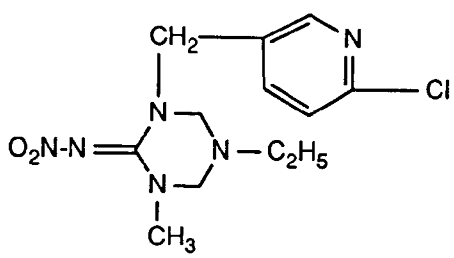

.

**31.** Verbindung gemäss Anspruch 26 der Formel

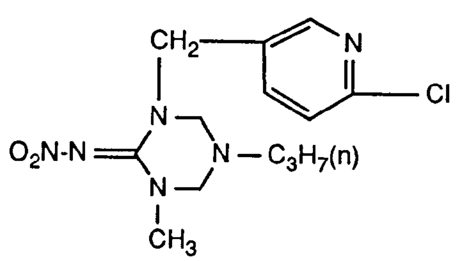

.

**32.** Verbindung gemäss Anspruch 26 der Formel

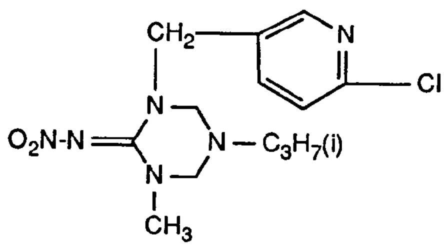

.

**33.** Verbindung gemäss Anspruch 26 der Formel

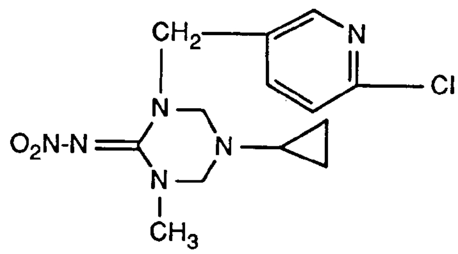

.

**34.** Verbindung gemäss Anspruch 26 der Formel

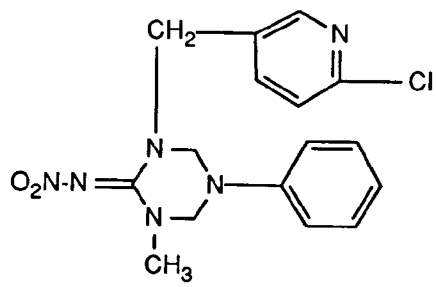

.

**35.** Verbindung gemäss Anspruch 26 der Formel

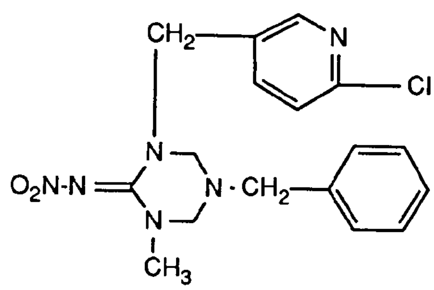

.

**36.** Verbindung gemäss Anspruch 14 der Formel

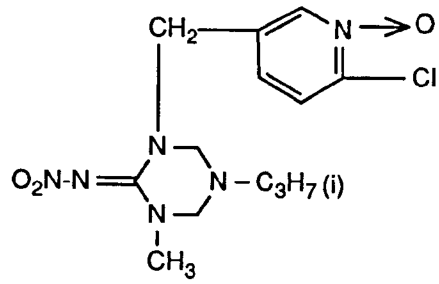

.

37. Verbindung gemäss Anspruch 14 der Formel

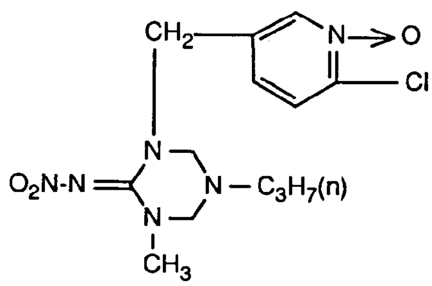

.

38. Verbindung gemäss Anspruch 26 der Formel

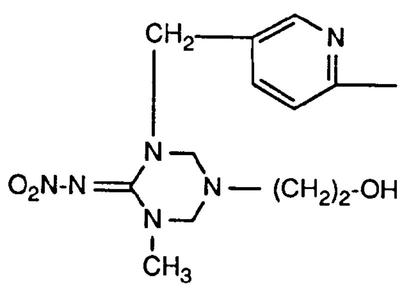

.

39. Verbindung gemäss Anspruch 25 der Formel

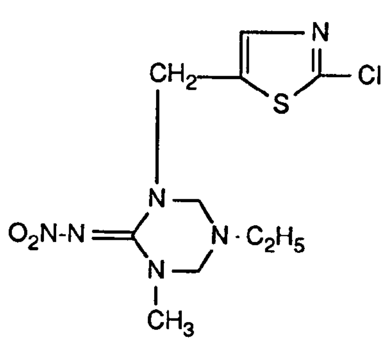

.

40. Verbindung gemäss Anspruch 27 der Formel

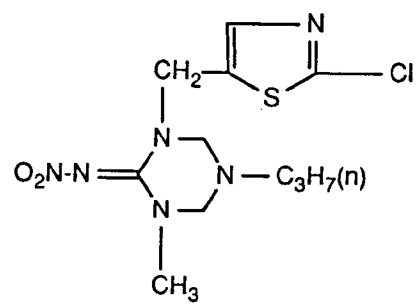

**41.** Verbindung gemäss Anspruch 14 der Formel

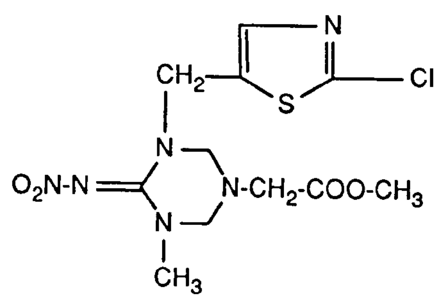

**42.** Verbindung gemäss Anspruch 14 der Formel

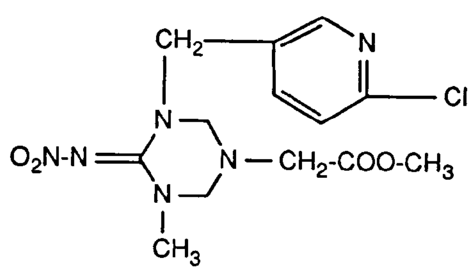

**43.** Verbindung gemäss Anspruch 14 der Formel

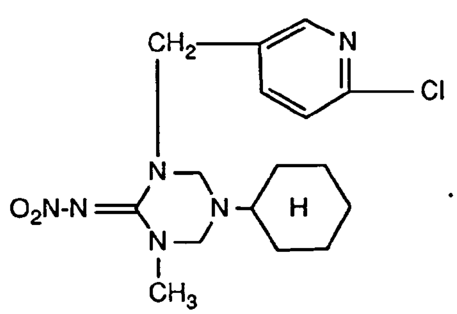

**44.** Verbindung gemäss Anspruch 14 der Formel

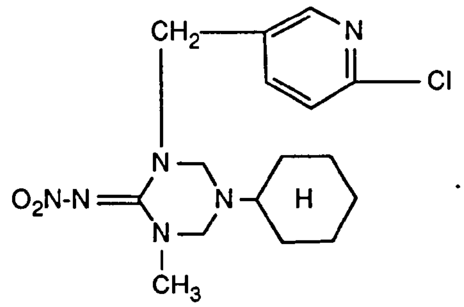

45. Verbindung gemäss Anspruch 26 der Formel

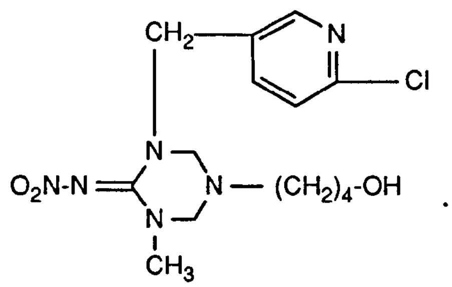

46. Verbindung gemäss Anspruch 26 der Formel

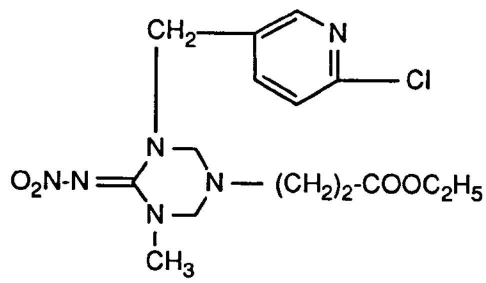

47. Verfahren zur Herstellung einer Verbindung der Formel I gemäss Anspruch 1

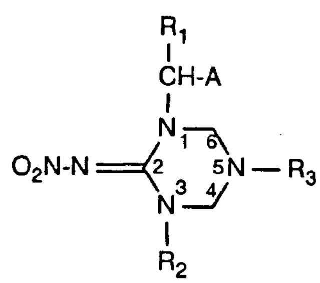

(I),

worin

$R_1$ Wasserstoff oder $C_1$-$C_4$-Alkyl,

$R_2$ Wasserstoff, $C_1$-$C_6$-Alkyl, $C_3$-$C_6$-Cycloalkyl oder einen Rest -$CH_2B$;

$R_3$ Wasserstoff, $C_1$-$C_{10}$-Alkyl, $C_3$-$C_6$-Cycloalkyl, mit 1-12 Resten aus der Gruppe Halogen, Hydroxy, $C_1$-$C_4$-Alkoxy, $C_1$-$C_4$-Haloalkoxy mit 1 bis 9 Halogenatomen, Di-($C_1$-$C_4$-Alkyl)-amino und $C_1$-$C_5$-Alkoxycarbonyl substituiertes $C_1$-$C_{10}$-Alkyl, mit 1-4 $C_1$-$C_4$-Alkylresten oder Halogenatomen substituiertes $C_3$-$C_6$-Cyc-

loalkyl, $C_2$-$C_8$-Alkenyl oder -Alkinyl, mit 1-6 Halogenatomen substituiertes $C_2$-$C_8$-Alkenyl oder -Alkinyl, Phenyl, Benzyl, oder mit 1-3 Ringsubstituenten aus der Gruppe Halogen, $C_1$-$C_4$-Alkyl, $C_1$-$C_4$-Haloalkyl mit 1 bis 9 Halogenatomen, $C_1$-$C_4$-Alkoxy, $C_1$-$C_4$-Haloalkoxy mit 1 bis 9 Halogenatomen, $C_1$-$C_4$-Alkylthio, Nitro und Cyano substituiertes Phenyl oder Benzyl;

A einen unsubstituierten oder ein- bis vierfach substituierten aromatischen oder nichtaromatischen, monocyclischen oder bicyclischen heterocyclischen Rest, der ein bis zwei Substituenten aus der Gruppe $C_1$-$C_3$-Halogenalkyl mit 1 bis 7 Halogenatomen, Cyclopropyl, Halogencyclopropyl mit 1 bis 3 Halogenatomen, $C_2$-$C_3$-Alkenyl, $C_2$-$C_3$-Alkinyl, $C_2$-$C_3$-Halogenalkenyl und $C_2$-$C_3$-Halogenalkinyl mit 1 bis 4 Halogenatomen, $C_1$-$C_3$-Halogenalkoxy mit 1 bis 7 Halogenatomen, $C_1$-$C_3$-Alkylthio, $C_1$-$C_3$-Halogenalkylthio mit 1 bis 7 Halogenatomen, Allyloxy, Propargyloxy, Allylthio, Propargylthio, Halogenallyloxy, Halogenallylthio, Cyan und Nitro und ein bis vier Substituenten aus der Gruppe $C_1$-$C_3$-Alkyl, $C_1$-$C_3$-Alkoxy und Halogen aufweisen kann; und

B Phenyl, Cyanophenyl, Nitrophenyl, Halogenphenyl mit 1 bis 3 Halogenatomen, durch $C_1$-$C_3$-Alkyl, $C_1$-$C_3$-Halogenalkyl mit 1 bis 7 Halogenatomen, $C_1$-$C_3$-Alkoxy oder $C_1$-$C_3$-Halogenalkoxy mit 1 bis 7 Halogenatomen substituiertes Phenyl, 3-Pyridyl, 5-Thiazolyl, durch ein bis zwei Substituenten aus der Gruppe $C_1$-$C_3$-Alkyl, $C_1$-$C_3$-Halogenalkyl mit 1 bis 7 Halogenatomen, Cyclopropyl, Halogencyclopropyl, $C_2$-$C_3$-Alkenyl, $C_2$-$C_3$-Alkinyl, $C_1$-$C_3$-Alkoxy, $C_2$-$C_3$-Halogenalkenyl und $C_2$-$C_3$-Halogenalkinyl mit 1 bis 4 Halogenatomen, $C_1$-$C_3$-Halogenalkoxy mit 1 bis 7 Halogenatomen, $C_1$-$C_3$-Alkylthio, $C_1$-$C_3$-Halogenalkylthio mit 1 bis 7 Halogenatomen, Allyloxy, Propargyloxy, Allylthio, Propargylthio, Halogenallyloxy, Halogenallylthio, Halogen, Cyan und Nitro substituiertes 5-Thiazolyl; oder durch ein bis zwei Reste aus der Gruppe $C_1$-$C_3$-Halogenalkyl mit 1 bis 7 Halogenatomen, Cyclopropyl, Halogencyclopropyl, $C_2$-$C_3$-Alkenyl, $C_2$-$C_3$-Alkinyl, $C_2$-$C_3$-Halogenalkenyl und $C_2$-$C_3$-Halogenalkinyl mit 1 bis 4 Halogenatomen, $C_1$-$C_3$-Halogenalkoxy mit 1 bis 7 Halogenatomen, $C_1$-$C_3$-Alkylthio, $C_1$-$C_3$-Halogenalkylthio mit 1 bis 7 Halogenatomen, Allyloxy, Propargyloxy, Allylthio, Propargylthio, Halogenallyloxy, Halogenallylthio, Cyan und Nitro oder durch ein bis vier Reste aus der Gruppe $C_1$-$C_3$-Alkyl, $C_1$-$C_3$-Alkoxy und Halogen substituiertes 3-Pyridyl; bedeuten, sowie deren Salze mit anorganischen Säuren, dadurch gekennzeichnet, dass man

a) eine Verbindung der Formel II

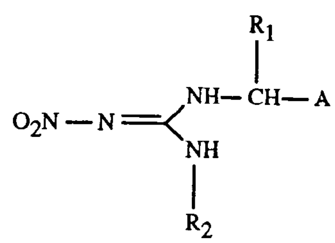

(II)

mit Formaldehyd, bzw. Paraformaldehyd und einer Verbindung der Formel III

$$H_2N\text{-}R_3 \qquad (III)$$

umsetzt; oder

b) eine Verbindung der Formel IV

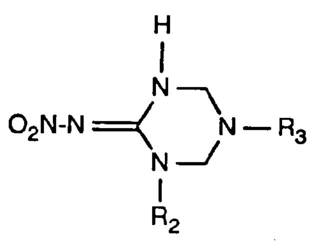

(IV)

mit einer Verbindung der Formel V

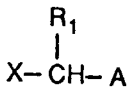

(V)

umsetzt; oder

c) zur Herstellung einer Verbindung der Formel I, worin $R_2$ eine von Wasserstoff abweichende Bedeutung hat, eine erhaltene Verbindung der Formel I, worin $R_2$ Wasserstoff bedeutet, mit einer Verbindung der Formel VI

$Y\text{-}R_2$ (VI)

umsetzt;

und dass man erwünschtenfalls eine erhaltene Verbindung der Formel I in ein Salz derselben überführt; wobei in den Formeln II bis VI $R_1$, $R_2$, $R_3$ und A die in Anspruch 1 angegebenen Bedeutungen haben, X für ein Halogenatom und Y für eine Abgangsgruppe steht.

**48.** Verbindung der Formel IV gemäss Anspruch 47

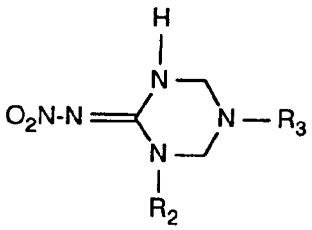

(IV)

worin $R_2$ und $R_3$ die in Anspruch 1 angegebenen Bedeutungen haben, mit Ausnahme von 2-Nitroimino-5-methyl-1,3,5-triazacrylohexan und 2-Nitroimino-1,3,5-triazacyclohexan.

**49.** Verfahren zur Herstellung einer Verbindung der Formel IV gemäss Anspruch 48, dadurch gekennzeichnet, dass man eine Verbindung der Formel VII

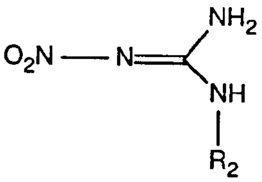

(VII)

mit Formaldehyd, bzw. Paraformaldehyd und einer Verbindung der Formel III

$H_2N\text{-}R_3$ (III)

umsetzt, wobei in der Formeln VII und III $R_2$ und $R_3$ die unter Anspruch 1 angegebenen Bedeutungen haben.

**50.** Schädlingsbekämpfungsmittel, welches als aktive Komponente eine Verbindung gemäss einem der Ansprüche 1 bis 46 zusammen mit geeigneten Trägern und/oder anderen Zuschlagsstoffen enthält.

**51.** Verwendung einer Verbindung gemäss einem der Ansprüche 1 bis 46 zur Bekämpfung von Insekten und Vertretern der Ordnung Akarina an Tieren und Pflanzen.

**52.** Verwendung gemäss Anspruch 51, zur Bekämpfung von pflanzenschädigenden Insekten.

**53.** Verwendung gemäss Anspruch 52 zur Bekämpfung von saugenden Insekten.

**54.** Verfahren zur Bekämpfung von Insekten und Vertretern der Ordnung Akarina, dadurch gekennzeichnet, dass man die Schädlinge bzw. deren verschiedene Entwicklungsstadien oder ihren Aufenthaltsort mit

einer pestizid wirksamen Menge einer Verbindung der Formel I gemäss einem der Ansprüche 1 bis 46 oder mit einem Mittel enthaltend neben Zusatz- und Trägerstoffen eine pestizid wirksame Menge dieser Verbindung, in Kontakt bringt oder behandelt.

**55.** Verfahren gemäss Anspruch 54 zur Bekämpfung von pflanzenschädigenden Insekten.

**56.** Verfahren gemäss Anspruch 55 zur Bekämpfung von saugenden Insekten.

**Patentansprüche für folgenden Vertragsstaat:ES**

**1.** Verfahren zur Herstellung einer Verbindung der Formel I

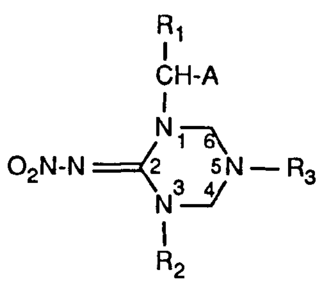

(I)

worin

$R_1$ Wasserstoff oder $C_1$-$C_4$-Alkyl,

$R_2$ Wasserstoff, $C_1$-$C_6$-Alkyl, $C_3$-$C_6$-Cycloalkyl oder einen Rest -$CH_2B$;

$R_3$ Wasserstoff, $C_1$-$C_{10}$-Alkyl, $C_3$-$C_6$-Cycloalkyl, mit 1-12 Resten aus der Gruppe Halogen, Hydroxy, $C_1$-$C_4$-Alkoxy, $C_1$-$C_4$-Haloalkoxy mit 1 bis 9 Halogenatomen, Di-($C_1$-$C_4$-Alkyl)-amino und $C_1$-$C_5$-Alkoxycarbonyl substituiertes $C_1$-$C_{10}$-Alkyl, mit 1-4 $C_1$-$C_4$-Alkylresten oder Halogenatomen substituiertes $C_3$-$C_6$-Cycloalkyl, $C_2$-$C_8$-Alkenyl oder -Alkinyl, mit 1-6 Halogenatomen substituiertes $C_2$-$C_8$-Alkenyl oder -Alkinyl, Phenyl, Benzyl, oder mit 1-3 Ringsubstituenten aus der Gruppe Halogen, $C_1$-$C_4$-Alkyl, $C_1$-$C_4$-Haloalkyl mit 1 bis 9 Halogenatomen, $C_1$-$C_4$-Alkoxy, $C_1$-$C_4$-Haloalkoxy mit 1 bis 9 Halogenatomen, $C_1$-$C_4$-Alkylthio, Nitro und Cyano substituiertes Phenyl oder Benzyl;

A einen unsubstituierten oder ein- bis vierfach substituierten aromatischen oder nichtaromatischen, monocyclischen oder bicyclischen heterocyclischen Rest, der ein bis zwei Substituenten aus der Gruppe $C_1$-$C_3$-Halogenalkyl mit 1 bis 7 Halogenatomen, Cyclopropyl, Halogencyclopropyl mit 1 bis 3 Halogenatomen, $C_2$-$C_3$-Alkenyl, $C_2$-$C_3$-Alkinyl, $C_2$-$C_3$-Halogenalkenyl und $C_2$-$C_3$-Halogenalkinyl mit 1 bis 4 Halogenatomen, $C_1$-$C_3$-Halogenalkoxy mit 1 bis 7 Halogenatomen, $C_1$-$C_3$-Alkylthio, $C_1$-$C_3$-Halogenalkylthio mit 1 bis 7 Halogenatomen, Allyloxy, Propargyloxy, Allylthio, Propargylthio, Halogenallyloxy, Halogenallylthio, Cyan und Nitro und ein bis vier Substituenten aus der Gruppe $C_1$-$C_3$-Alkyl, $C_1$-$C_3$-Alkoxy und Halogen enthalten kann; und

B Phenyl, Cyanophenyl, Nitrophenyl, Halogenphenyl mit 1 bis 3 Halogenatomen, durch $C_1$-$C_3$-Alkyl, $C_1$-$C_3$-Halogenalkyl mit 1 bis 7 Halogenatomen, $C_1$-$C_3$-Alkoxy oder $C_1$-$C_3$-Halogenalkoxy mit 1 bis 7 Halogenatomen substituiertes Phenyl, 3-Pyridyl, 5-Thiazolyl, durch ein bis zwei Substituenten aus der Gruppe $C_1$-$C_3$-Alkyl, $C_1$-$C_3$-Halogenalkyl mit 1 bis 7 Halogenatomen, Cyclopropyl, Halogencyclopropyl, $C_2$-$C_3$-Alkenyl, $C_2$-$C_3$-Alkinyl, $C_1$-$C_3$-Alkoxy, $C_2$-$C_3$-Halogenalkenyl und $C_2$-$C_3$-Halogenalkinyl mit 1 bis 4 Halogenatomen, $C_1$-$C_3$-Halogenalkoxy mit 1 bis 7 Halogenatomen, $C_1$-$C_3$-Alkylthio, $C_1$-$C_3$-Halogenalkylthio mit 1 bis 7 Halogenatomen, Allyloxy, Propargyloxy, Allylthio, Propargylthio, Halogenallyloxy, Halogenallylthio, Halogen, Cyan und Nitro substituiertes 5-Thiazolyl; oder durch ein bis zwei Reste aus der Gruppe $C_1$-$C_3$-Halogenalkyl mit 1 bis 7 Halogenatomen, Cyclopropyl, Halogencyclopropyl, $C_2$-$C_3$-Alkenyl, $C_2$-$C_3$-Alkinyl, $C_2$-$C_3$-Halogenalkenyl und $C_2$-$C_3$-Halogenalkinyl mit 1 bis 4 Halogenatomen, $C_1$-$C_3$-Halogenalkoxy mit 1 bis 7 Halogenatomen, $C_1$-$C_3$-Alkylthio, $C_1$-$C_3$-Halogenalkylthio mit 1 bis 7 Halogenatomen, Allyloxy, Propargyloxy, Allylthio, Propargylthio, Halogenallyloxy, Halogenallylthio, Cyan und Nitro oder durch ein bis vier Reste aus der Gruppe $C_1$-$C_3$-Alkyl, $C_1$-$C_3$-Alkoxy und Halogen substituiertes 3-Pyridyl; bedeuten, sowie deren Salze mit anorganischen Säuren, dadurch gekennzeichnet, dass man

a) eine Verbindung der Formel II

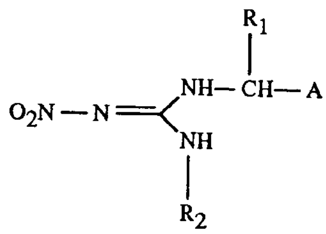

(II)

mit Formaldehyd, bzw. Paraformaldehyd und einer Verbindung der Formel III

$H_2N\text{-}R_3$ (III)

umsetzt; oder

b) eine Verbindung der Formel IV

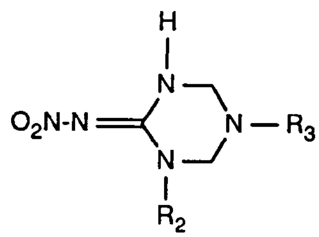

(IV)

mit einer Verbindung der Formel V

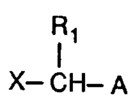

(V)

umsetzt; oder

c) zur Herstellung einer Verbindung der Formel I, worin $R_2$ eine von Wasserstoff abweichende Bedeutung hat, eine erhaltene Verbindung der Formel I, worin $R_2$ Wasserstoff bedeutet, mit einer Verbindung der Formel VI

$Y\text{-}R_2$ (VI)

umsetzt;

und dass man erwünschtenfalls eine erhaltene Verbindung der Formel I in ein Salz derselben überführt; wobei in den Formeln II bis VI $R_1$, $R_2$, $R_3$ und A die in Anspruch 1 angegebenen Bedeutungen haben, X für ein Halogenatom und Y für eine Abgangsgruppe steht.

**2.** Verfahren gemäss Anspruch 1, zur Herstellung einer Verbindung der Formel I worin

$R_3$ $C_5$-$C_{10}$-Alkyl, $C_3$-$C_6$-Cycloalkyl, mit 1-12 Resten aus der Gruppe Halogen, Hydroxy, $C_1$-$C_4$-Alkoxy, $C_1$-$C_4$-Haloalkoxy mit 1 bis 9 Halogenatomen, Di-($C_1$-$C_4$-Alkyl)-amino und $C_1$-$C_5$-Alkoxycarbonyl substituiertes $C_1$-$C_{10}$-Alkyl, mit 1-4 $C_1$-$C_4$-Alkyl-resten oder Halogenatomen substituiertes $C_3$-$C_6$-Cycloalkyl, $C_2$-$C_8$-Alkenyl oder -Alkinyl, mit 1-6 Halogenatomen substituiertes $C_2$-$C_8$-Alkenyl oder -Alkinyl, Phenyl, Benzyl, oder mit 1-3 Ringsubstituenten aus der Gruppe Halogen, $C_1$-$C_4$-Alkyl, $C_1$-$C_4$-Haloalkyl mit 1 bis 9 Halogenatomen, $C_1$-$C_4$-Alkoxy, $C_1$-$C_4$-Haloalkoxy mit 1 bis 9 Halogenatomen, $C_1$-$C_4$-Alkylthio, Nitro und Cyano substituiertes Phenyl oder Benzyl bedeutet und $R_1$, $R_2$ und A die in Anspruch 1 angegebenen Bedeutungen haben, sowie deren Salze mit anorganischen Säuren.

**3.** Verfahren gemäss Anspruch 1 oder 2 zur Herstellung einer Verbindung der Formel I, worin der heterocyclische Rest A ungesättigt ist, über ein Kohlenstoffatom an den Rest des Moleküls der Verbindung der Formel I gebunden ist und mindestens ein Stickstoffatom enthält.

4. Verfahren gemäss Anspruch 3 zur Herstellung einer Verbindung der Formel I, worin der heterocyclische Rest A ungesättigt ist und über ein Kohlenstoffatom an den Rest des Moleküls der Verbindung der Formel 1 gebunden ist und ein bis drei Heteroatome aus der Reihe Sauerstoff, Schwefel und Stickstoff enthält, wobei jeweils höchstens ein Sauerstoffoder Schwefelatom enthalten ist.

5. Verfahren gemäss Anspruch 4 zur Herstellung einer Verbindung der Formel I, worin der heterocyclische Rest A ein bis drei Heteroatome aus der Reihe Sauerstoff, Schwefel und Stickstoff enthält, wovon stets ein Heteroatom Stickstoff ist, und höchstens ein Sauerstoffatom oder Schwefelatom enthalten ist.

6. Verfahren gemäss Anspruch 1 oder 2 zur Herstellung einer Verbindung der Formel I, worin der heterocyclische Rest A einen über ein Kohlenstoffatom an den Rest des Moleküls der Verbindung der Formel 1 gebundenen heterocyclischen Grundkörper aus der Gruppe

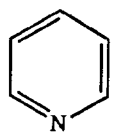 , 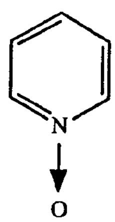 , 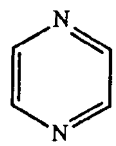 , 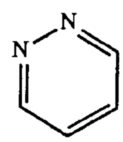 , 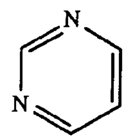 , 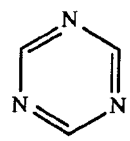 , 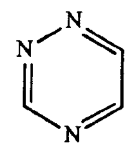 ,

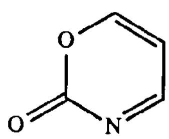 , 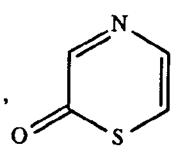 , 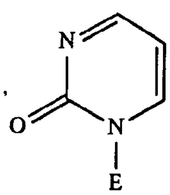 , 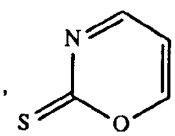 , 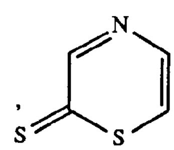 , 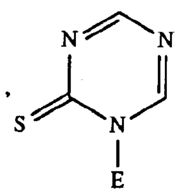 ,

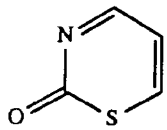 , 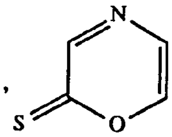 , 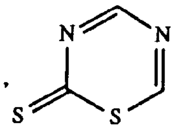 , 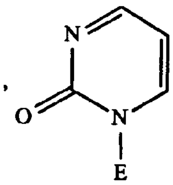 , 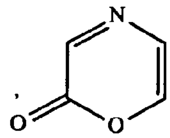 ,

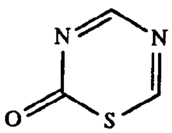 , 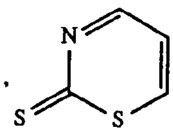 , 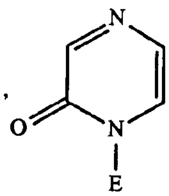 , 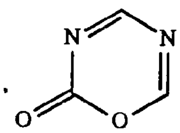 , 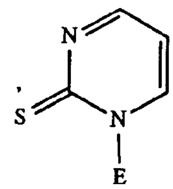 , 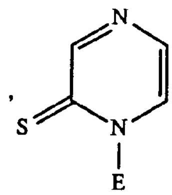 ,

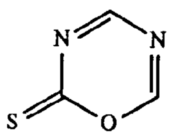 , 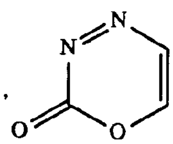 , 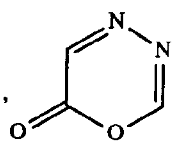 , 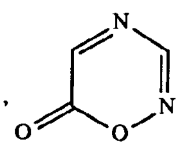 , 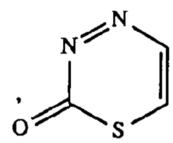 ,

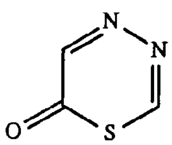 , 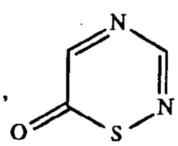 , 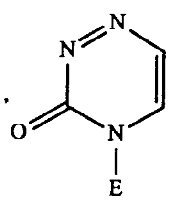 , 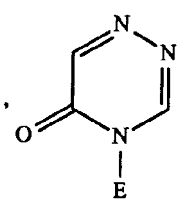 , 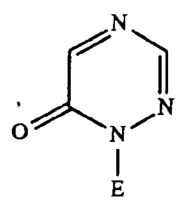 ,

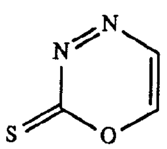 , 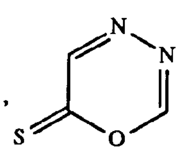 , 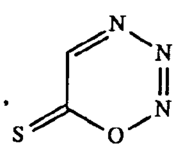 , 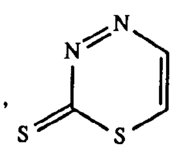 , 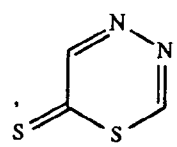 ,

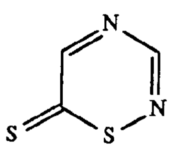 , 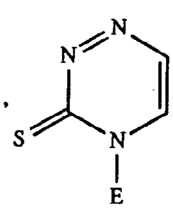 , 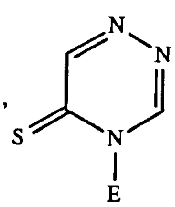 , 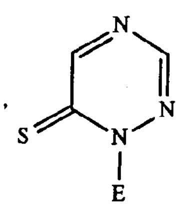 , 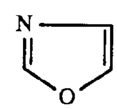 ,

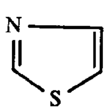 , 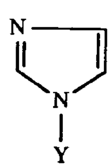 , 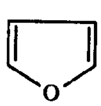 , 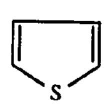 ,

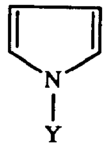 , 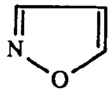 , 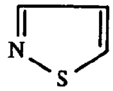 , 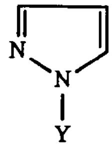 ,

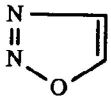 , 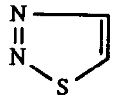 , 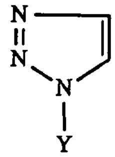 , 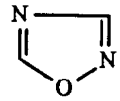 , 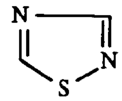 ,

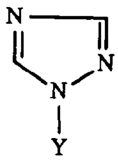 , 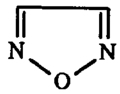 , 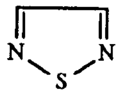 , 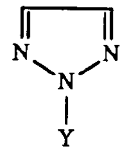 , 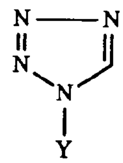 ,

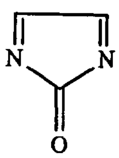 , 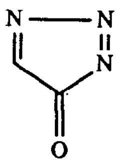 , 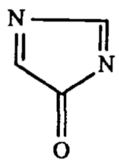 , 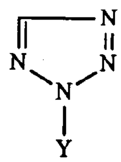 , 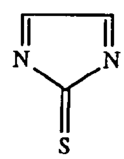 ,

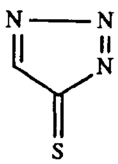 , 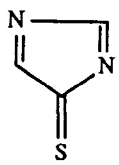 , 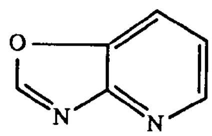 , 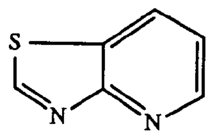 , 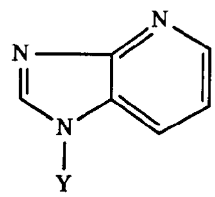 ,

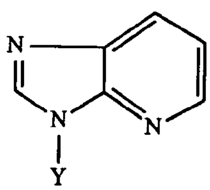 , 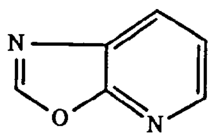 , 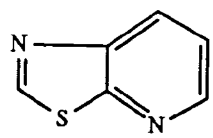 , 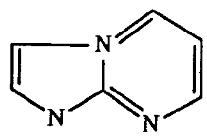 ,

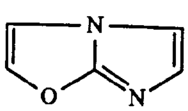 , 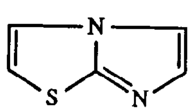 , 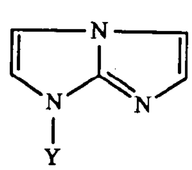 .

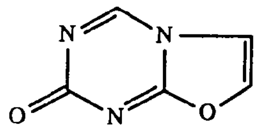, 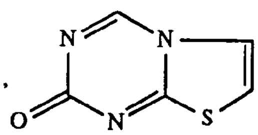, 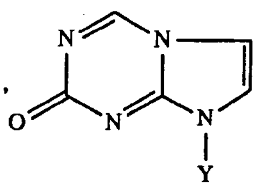, 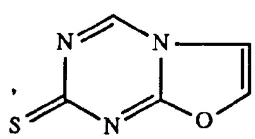,

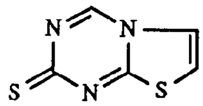 und 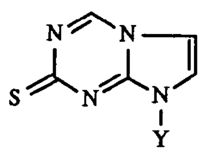

darstellt, welcher unsubstituiert ist oder je nach den Substitutionsmöglichkeiten des Ringsystems bis zu vier der im Anspruch 1 definierten Substituenten tragen kann und worin E für $C_1$-$C_3$-Alkyl und Y für Wasserstoff, $C_1$-$C_3$-Alkyl oder Cyclopropyl stehen.

**7.** Verfahren gemäss Anspruch 6 zur Herstellung einer Verbindung der Formel I , worin der heterocyclische Rest A unsubstituiert ist oder ein bis drei Substituenten aus der Gruppe Halogen, $C_1$-$C_3$-Alkyl, $C_1$-$C_3$-Halogenalkyl sowie $C_1$-$C_3$-Halogenalkoxy mit 1 bis 7 Halogenatomen und $C_1$-$C_3$-Alkoxy trägt.

**8.** Verfahren gemäss Anspruch 7 zur Herstellung einer Verbindung der Formel I, worin der Rest A Pyridyl oder Thiazolyl bedeutet.

**9.** Verfahren gemäss Anspruch 1 oder 2 zur Herstellung einer Verbindung der Formel I, worin der Rest B für einen Phenyl-, Pyridyl- oder Thiazolylrest steht, welcher unsubstituiert oder durch ein bis zwei Reste aus der Gruppe Halogen, $C_1$-$C_3$-Alkyl, $C_1$-$C_3$-Halogenalkyl und $C_1$-$C_3$-Halogenalkoxy mit 1 bis 7 Halogenatomen und $C_1$-$C_3$-Alkoxy substituiert ist.

**10.** Verfahren gemäss Anspruch 7 zur Herstellung einer Verbindung der Formel I, worin der Rest A für 3-Pyridyl, 2-Halogenpyrid-5-yl, 2,3-Dihalogenpyrid-5-yl oder 2-Halogenthiazol-4-yl, 1-Oxopyrid-3-yl, 1-Oxo-2-halogenpyrid-5-yl oder 1-Oxo-2,3-dihalogenpyrid-5-yl steht.

**11.** Verfahren gemäss Anspruch 1 oder 2 zur Herstellung einer Verbindung der Formel I, worin $R_1$ Wasserstoff, $R_2$ Methyl, Äthyl oder Cyclopropyl; und A Pyridyl, 1-Oxopyridyl, Thiazolyl oder jeweils durch ein bis drei Substituenten aus der Gruppe Halogen, $C_1$-$C_3$-Alkyl, $C_1$-$C_3$-Halogenalkyl und $C_1$-$C_3$-Halogenalkoxy mit 1 bis 7 Halogenatomen und $C_1$-$C_3$-Alkoxy substituiertes Pyridyl, 1-Oxopyridyl oder Thiazolyl bedeuten.

**12.** Verfahren gemäss Anspruch 1 oder 2 zur Herstellung einer Verbindung der Formel I, worin $R_1$ Wasserstoff bedeutet.

**13.** Verfahren gemäss Anspruch 12 zur Herstellung einer Verbindung der Formel I, worin $R_1$ Wasserstoff und $R_2$ Methyl bedeuten.

**14.** Verfahren gemäss einem der Ansprüche 1 und 3 bis 13 zur Herstellung einer Verbindung der Formel I, worin $R_3$ $C_1$-$C_3$-Alkyl, Cyclopropyl, Cyclohexyl, Phenyl, Benzyl oder den Rest $-CH_2-COO-CH_3$ bedeutet.

**15.** Verfahren gemäss Anspruch 13 zur Herstellung einer Verbindung der Formel I, worin $R_3$ mit 1 bis 3 Ringsubstituenten aus der Gruppe Fluor, Chlor, Brom, $C_1$-$C_2$-Alkyl, $C_1$-$C_2$-Halogenalkyl, $C_1$-$C_2$-Alkoxy, $C_1$-$C_2$-Alkylthio, Nitro und Cyano substituiertes Benzyl bedeutet.

**16.** Verfahren gemäss Anspruch 13 zur Herstellung einer Verbindung der Formel I, worin $R_3$ mit 1 bis 3 Ringsubstituenten aus der Gruppe Fluor, Chlor, Brom, $C_1$-$C_2$-Alkyl, $C_1$-$C_2$-Halogenalkyl, $C_1$-$C_2$-Alkoxy, $C_1$-$C_2$-Alkylthio, Nitro und Cyano substituiertes Phenyl bedeutet.

**17.** Verfahren gemäss Anspruch 13 zur Herstellung einer Verbindung der Formel I, worin $R_3$ mit einer Hydroxy-Gruppe substituiertes $C_1$-$C_6$-Alkyl bedeutet.

**18.** Verfahren gemäss Anspruch 13 zur Herstellung einer Verbindung der Formel I, worin $R_3$ mit einer $C_1$-$C_5$-Alkoxycarbonyl-Gruppe substituiertes $C_1$-$C_6$-Alkyl bedeutet.

**19.** Verfahren gemäss Anspruch 13 zur Herstellung einer Verbindung der Formel I, worin $R_3$ $-CH_2CH_2F$, $-CH_2CH_2Br$, $-CH_2CH_2CH_2Cl$, $-CH_2CH_2CH_2Br$ oder $-CH_2CHClCH_2CH_2CH_2Cl$ bedeutet.

**20.** Verfahren gemäss Anspruch 13 zur Herstellung einer Verbindung der Formel I, worin $R_3$ $-CH_2CH_2O-CH_3$, $-CH_2CH_2CH_2-O-CH_2CH_3$, $-CH(CH_3)CH_2-O-CH_3$, $-CH_2CH(OCH_3)_2$, $-CH_2CH_2-N(CH_2CH_3)_2$, $-CH_2-CH_2CH_2-N(CH_3)_2$ oder $-CH_2CH_2CH_2-N(CH_2CH_3)_2$ bedeutet.

**21.** Verfahren gemäss Anspruch 13 zur Herstellung einer Verbindung der Formel I, worin $R_3$ unsubstituiertes oder mit ein oder zwei $C_1$-$C_4$-Alkylresten substituiertes $C_4$-$C_6$-Cycloalkyl bedeutet.

**22.** Verfarhen gemäss Anspruch 21 zur Herstellung einer Verbindung der Formel I, worin $R_3$ Cyclopentyl oder Cyclohexyl bedeutet.

**23.** Verfahren gemäss Anspruch 21 zur Herstellung einer Verbindung der Formel I, worin $R_3$ mit einer oder zwei Methylgruppen substituiertes $C_3$-$C_6$-Cycloalkyl bedeutet.

**24.** Verfahren gemäss Anspruch 1 oder 11 zur Herstellung einer Verbindung der Formel I, worin A 2-Chlorthiazol-4-yl, 2,3-Dichlorpyrid-5-yl, 1-Oxopyrid-3-yl oder 1-Oxo-2-chlorpyrid-5-yl; $R_2$ Methyl und $R_3$ Cyclopropyl, $-CH_2CH_2Cl$, $-CH_2CH(OCH_3)_2$ oder $-CH_2CH_2N(CH_3)_2$ bedeuten.

**25.** Verfahren gemäss einem der Ansprüche 1 bis 24 zur Herstellung einer Verbindung der Formel I, worin A 2-Chlorthiazol-4-yl bedeutet.

**26.** Verfahren gemäss einem der Ansprüche 1 bis 24 zur Herstellung einer Verbindung der Formel I, worin A 2-Chlorpyrid-5-yl bedeutet.

**27.** Verfahren gemäss einem der Ansprüche 1 bis 7 zur Herstellung einer Verbindung der Formel I, worin A 1-Oxo-2-chlorpyrid-5-yl oder 1-Oxopyrid-5-yl bedeutet.

**28.** Verfahren gemäss Anspruch 1 zur Herstellung einer Verbindung der Formel I, worin A 2-Chlorpyrid-5-yl, 2,3-Dichlorpyrid-5-yl, 2-Chlorthiazol-4-yl, 1-Oxopyrid-3-yl oder 1-Oxo-2-chlorpyrid-5-yl; $R_1$ Wasserstoff; $R_2$ Methyl; und $R_3$ n-Propyl bedeuten.

**29.** Verfahren gemäss Anspruch 26 zur Herstellung der Verbindung der Formel

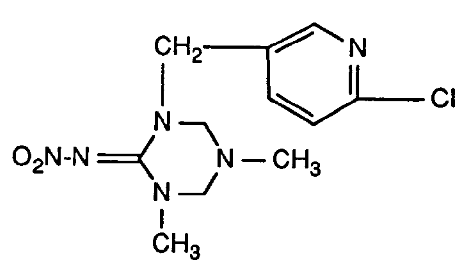

.

**30.** Verfahren gemäss Anspruch 26 zur Herstellung der Verbindung der Formel

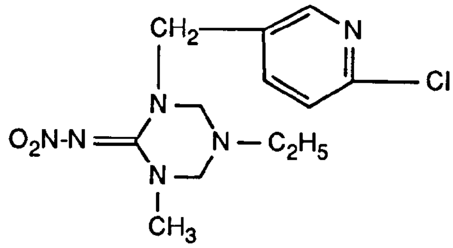

.

**31.** Verfahren gemäss Anspruch 26 zur Herstellung der Verbindung der Formel

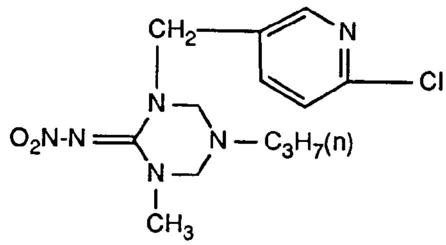

.

**32.** Verfahren gemäss Anspruch 26 zur Herstellung der Verbindung der Formel

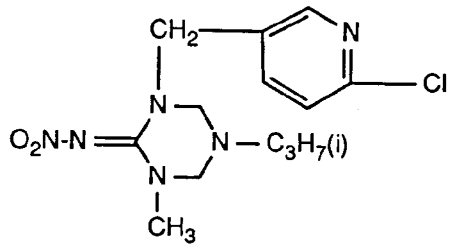

.

**33.** Verfahren gemäss Anspruch 26 zur Herstellung der Verbindung der Formel

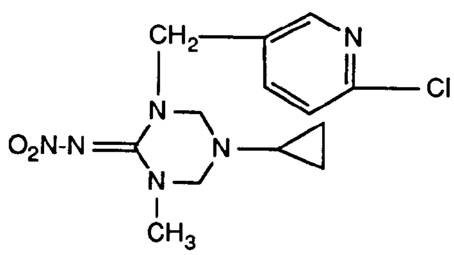

.

**34.** Verfahren gemäss Anspruch 26 zur Herstellung der Verbindung der Formel

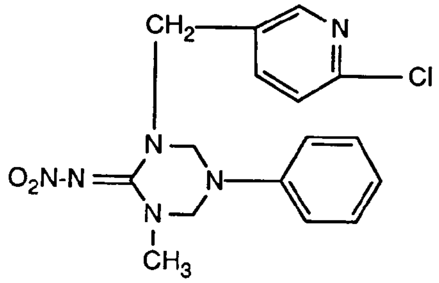

.

35. Verfahren gemäss Anspruch 26 zur Herstellung der Verbindung der Formel

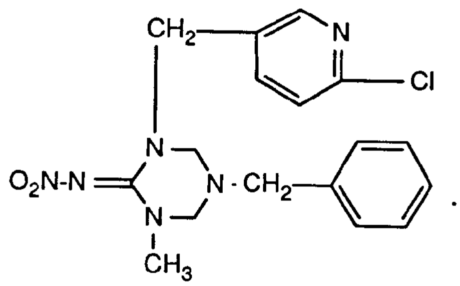

36. Verfahren gemäss Anspruch 14 zur Herstellung der Verbindung der Formel

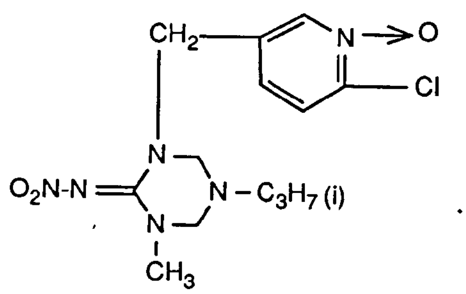

37. Verfahren gemäss Anspruch 14 zur Herstellung der Verbindung der Formel

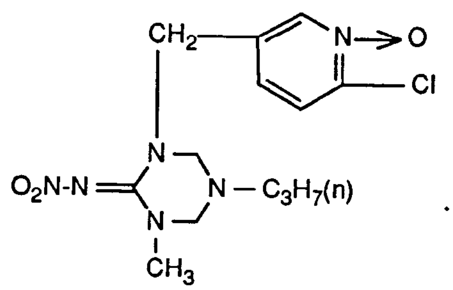

38. Verfahren gemäss Anspruch 26 zur Herstellung der Verbindung der Formel

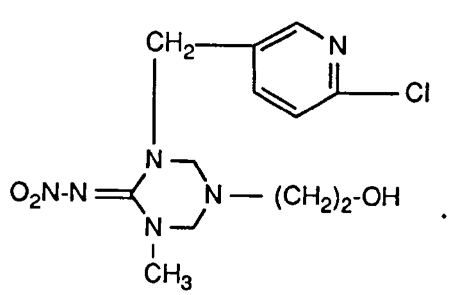

39. Verfahren gemäss Anspruch 25 zur Herstellung der Verbindung der Formel

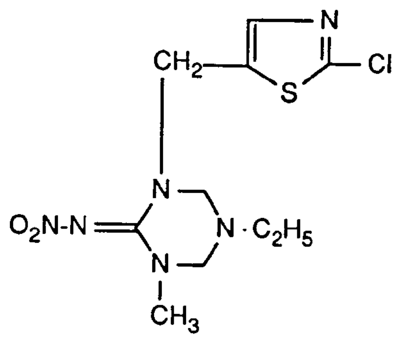

40. Verfahren gemäss Anspruch 27 zur Herstellung der Verbindung der Formel

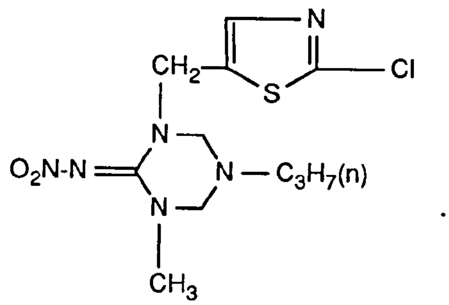

41. Verfahren gemäss Anspruch 14 zur Herstellung der Verbindung der Formel

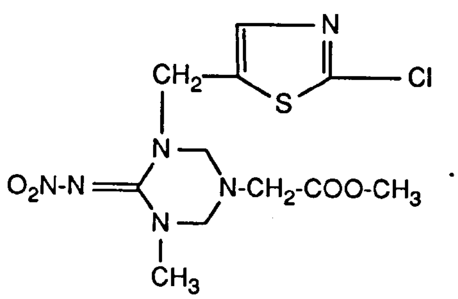

42. Verfahren gemäss Anspruch 14 zur Herstellung der Verbindung der Formel

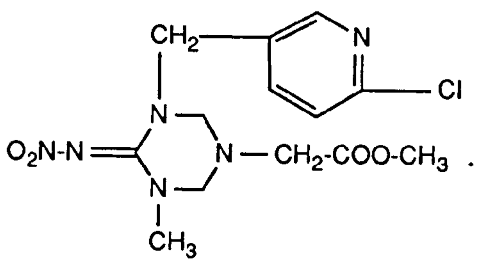

43. Verfahren gemäss Anspruch 14 zur Herstellung der Verbindung der Formel

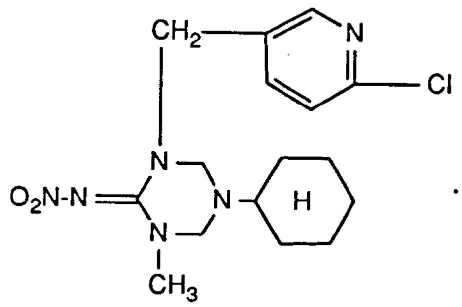

.

44. Verfahren gemäss Anspruch 14 zur Herstellung der Verbindung der Formel

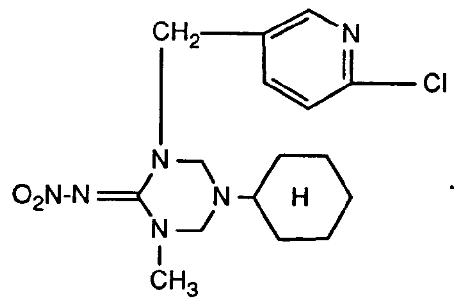

.

45. Verfahren gemäss Anspruch 26 zur Herstellung der Verbindung der Formel

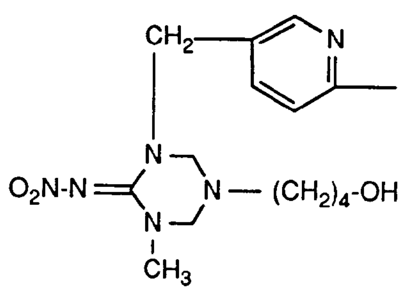

.

46. Verfahren gemäss Anspruch 26 zur Herstellung der Verbindung der Formel

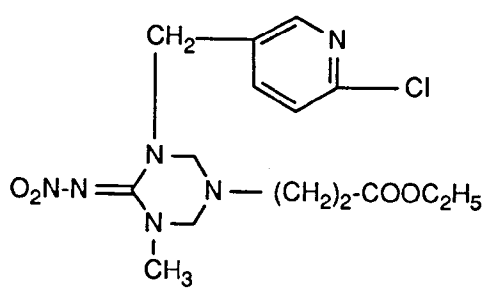

47. Verfahren zur Herstellung einer Verbindung der Formel IV gemäss Anspruch 1

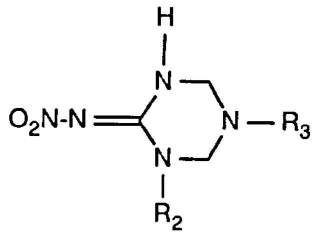

(IV)

worin $R_2$ und $R_3$ die in Anspruch 1 angegebenen Bedeutungen haben, mit Ausnahme von 2-Nitroimino-5-methyl-1,3,5-triazacrylohexan und 2-Nitroimino-1,3,5-triazacyclohexan, dadurch gekennzeichnet, dass man eine Verbindung der Formel VII

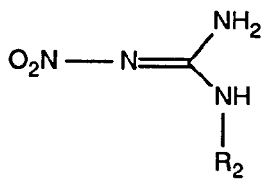

(VII)

mit Formaldehyd, bzw. Paraformaldehyd und einer Verbindung der Formel III

$H_2N-R_3$ (III)

umsetzt, wobei in der Formeln VII und III $R_2$ und $R_3$ die unter Anspruch 1 angegebenen Bedeutungen haben.

**48.** Schädlingsbekämpfungsmittel, welches als aktive Komponente eine Verbindung der Formel I gemäss einem der Ansprüche 1 bis 46 zusammen mit geeigneten Trägern und/oder anderen Zuschlagsstoffen enthält.

**49.** Verwendung einer Verbindung der Formel I gemäss einem der Ansprüche 1 bis 46 zur Bekämpfung von Insekten und Vertretern der Ordnung Akarina an Tieren und Pflanzen.

**50.** Verwendung gemäss Anspruch 49, zur Bekämpfung von pflanzenschädigenden Insekten.

**51.** Verwendung gemäss Anspruch 50 zur Bekämpfung von saugenden Insekten.

**52.** Verfahren zur Bekämpfung von Insekten und Vertretern der Ordnung Akarina, dadurch gekennzeichnet, dass man die Schädlinge bzw. deren verschiedene Entwicklungsstadien oder ihren Aufenthaltsort mit einer pestizid wirksamen Menge einer Verbindung der Formel I gemäss einem der Ansprüche 1 bis 46 oder mit einem Mittel enthaltend neben Zusatz- und Trägerstoffen eine pestizid wirksame Menge dieser Verbindung, in Kontakt bringt oder behandelt.

**53.** Verfarhen gemäss Anspruch 52 zur Bekämpfung von pflanzenschädigenden Insekten.

**54.** Verfahren gemäss Anspruch 53 zur Bekämpfung von saugenden Insekten.

Europäisches Patentamt

# EUROPÄISCHER RECHERCHENBERICHT

Nummer der Anmeldung

EP 91 81 0757

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| P,X | EP-A-0428941 (AGRO-KANESHO CO., LTD.)<br>* Ansprüche; Seiten 7, 8, Tabelle 1 * | 1-13, 24-26, 28-32, 47, 48 50 | C07D401/06<br>C07D417/06<br>A01N51/00<br>//(C07D401/06, 251:00,213:00)<br>(C07D417/06, 277:00,251:00) |
| D,X | EP-A-0386565 (NIHON TOKUSHU NOYAKU SEIZO K.K.)<br>* Ansprüche 1, 4-7 *<br>* Tabelle 1, Verbindungen 10-13, 15, 16, 40 *<br>* Beispiele 2, 3, 6 * | 1-10, 12, 25, 26, 50 | |
| D,X | US-A-4937340 (DER-SHING HUANG ET AL.)<br>* Ansprüche 1, 3 * | 48 | |
| A,D | EP-A-0259738 (NIHON TOKUSHU NOYAKU SEIZO K.K.)<br>* Ansprüche 1, 4-9 * | 1, 47, 50, 52, 54, 55 | |
| A,D | EP-A-0375907 (NIHON TOKUSHU NOYAKU SEIZO K.K.)<br>* Ansprüche * | 1, 47, 50 | RECHERCHIERTE SACHGEBIETE (Int. Cl.5 ) |
| A,D | EP-A-0376279 (TAKEDA CHEMICAL INDUSTRIES, LTD.)<br>* Seiten 22-29; Ansprüche * | 1, 47, 50 | A01N51/00<br>C07D251/00<br>C07D401/00<br>C07D417/00 |

Der vorliegende Recherchenbericht wurde für alle Patentansprüche erstellt

| Recherchenort | Abschlußdatum der Recherche | Prüfer |
|---|---|---|
| BERLIN | 18 DEZEMBER 1991 | HASS C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)